(19) Europäisches Patentamt
European Patent Office
Office européen des brevets

(11) EP 3 290 101 A1

(12) # EUROPÄISCHE PATENTANMELDUNG

(43) Veröffentlichungstag:
**07.03.2018 Patentblatt 2018/10**

(21) Anmeldenummer: **16187197.5**

(22) Anmeldetag: **05.09.2016**

(51) Int Cl.:
***B01D 67/00*** *(2006.01)* ***B01D 57/02*** *(2006.01)*
***B01D 69/10*** *(2006.01)* ***B01D 69/12*** *(2006.01)*
***C07C 51/42*** *(2006.01)* ***A61M 1/16*** *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Nanoscience for life GmbH & Co. KG**
**65193 Wiesbaden (DE)**

(72) Erfinder: **DIETZ, Max**
**65193 Wiesbaden (DE)**

(74) Vertreter: **Arth, Hans-Lothar**
**ABK Patent Attorneys**
**Jasminweg 9**
**14052 Berlin (DE)**

(54) **VERFAHREN ZUR HERSTELLUNG STOFFSELEKTIVER TRENNMEMBRANEN UND DEREN VERWENDUNG**

(57) Die vorliegende Erfindung betrifft ein Verfahren zur Herstellung einer offenporigen Membran mit nanocavitären Polymerstrukturen sowie deren Verwendung zur selektiven Abtrennung von Carbonsäuren.

EP 3 290 101 A1

## Beschreibung

## Abstract

Die vorliegende Erfindung betrifft ein Verfahren zur Herstellung von offenporigen nanocavitären Polymerstrukturen in soliden Trennmedien zur Abtrennung von Fett- und/oder Carbonsäuren aus vornehmlich wässrigen Gemischen.

## Beschreibung

Carbon- bzw. Fettsäuren sind amphiphile Moleküle, die sich kaum in Wasser lösen und daher in Wasser lediglich nur in geringen Mengen in volatiler Form vorliegen. Die häufigste Form, in der Fettsäuren in Wasser vorkommen, besteht daher in Form von Micellen und Emulsionen, die eine Phasentrennung bewirken. Die Amphiphilie bedingt, dass sie sich an Oberflächen abscheiden, die hydrophob oder lipophil sind.

Sofern sie mit Detergenzien gelöst sind, liegen sie in einem wässrigen Medium als micellare Partikel vor. Die meisten organischen Verbindungen adsorbieren Carbonsäuren bis zu einem gewissen Anteil über elektrostatische Bindungskräfte. Daher liegen Carbonsäuren in wässrigen organischen Lösungen zum überwiegenden Teil in einer gebundenen Form bzw. als Micellen mit anderen organischen Verbindungen vor. Aufgrund der Kleinheit derartiger Micellen ist eine Filtration dieser Gebilde praktisch nicht möglich oder es kommt zu einer Belegung der Filteroberflächen, die einen Verschluss der Filterfläche bedingt (Fouling). Daher werden klassische Extraktionsverfahren, wie die Filtration oder Dialyse, für eine Abtrennung von Carbonsäuren praktisch nicht angewandt.

Zur Abtrennung von Carbonsäuren können grundsätzlich klassische Extraktionsverfahren angewandt werden.

Hierzu gehören

- die Fest-Flüssig-Extraktionsverfahren, bei denen eine Adsorption der abzutrennenden Moleküle an Oberflächen erfolgt, z.B. bei der Chromatographie,
- die Flüssig-Flüssig-Extraktion, bei der sich das abzutrennende Molekül in einem flüssigen Extraktionsmittel, z.B. einem Alkohol oder einem unpolaren Lösungsmittel aus einem flüssigen Stoffgemisch anreichert,
- die Flüssig-Gas-Extraktion, bei der das abzutrennende Molekül durch ein komprimiertes Gas aus einem flüssigen Stoffgemisch herausgelöst und abgetrennt wird, z.B. in Form der überkritischen $CO_2$-Extraktion,
- die Destillation, bei der das abzutrennende Molekül aus einem flüssigen Stoffgemisch verdampft und in einer Kühlkolonne wieder kondensiert wird, sowie
- analytische Verfahren, wie die Gaschromatographie, die eine Kombination der o. g. Verfahren darstellt.

Die aufgeführten Extraktionsverfahren werden in der Analytischen Chemie (hier insb. die Chromatographie) sowie in der Pharmazie und Chemischen Industrie (hier insb. die Chromatographie und Flüssig-Flüssig-Extraktion) und der Petrochemie (hier insb. die Destillation) angewandt. Dabei sind die Verfahren, die einen hohen Stoffmengentransport ermöglichen, mit einem erheblichen Energieeintrag und damit Kostenaufwand verbunden. Membranbasierte Abtrennverfahren für in einem wässrigen Medium gelöste Carbonsäuren sind nicht existent.

## Detaillierte Beschreibung

Carbonsäuren lassen sich mit den vorgenannten Verfahren nur diskontinuierlich aus einem wässrigen Medium separieren. Im deprotonierten Zustand weisen Carbonsäuren eine negative Ladung auf und können somit in einem elektrischen Spannungsfeld bewegt werden. Daher ist grundsätzlich eine elektrophoretische Separation von Carbonsäuren möglich. Beim Durchtritt von Carbonsäuren durch ein offenporiges Separationsmedium, wie z.B. einer Filtermembran, werden die Oberflächen durch hieran adhärierende Carbonsäuren belegt, dabei ist es unerheblich, ob die Oberflächeneigenschaften derartiger Membranen hydrophob oder hydrophil sind. Ferner werden Verbindungen, die sich ebenfalls in dem wässrigen Medium befinden und die selbst eine negative Ladung aufweisen und/oder mit deprotonierten Carbonsäuren beladen sind, bei einer elektrophoretischen Abtrennung durch eine Membran mittransportiert. Daher ist eine selektive Abtrennung von Carbonsäuren aus wässrigen organischen Gemischen durch eine offenporige Membran mit Methoden aus dem Stand der Technik nicht möglich.

Membranbasierte Verfahren zur stofflichen Trennung auf molekularer Ebene werden nach dem Stand der Technik mit geschlossenen Membranen, die einen diffusiven Stofftransport der abzutrennenden molekularen Strukturen ermöglichen, durchgeführt und unter dem Begriff Nanofiltration zusammengefasst. Nachteilig ist dabei, dass die pro Membranflächeneinheit transportierbaren Stoffmengen nur gering sind und für die Abtrennung ein hoher Energiebedarf (z.B. für einen pneumatischen Druckaufbau von 20 - 80 bar) erforderlich ist (A comprehensive review of nanofiltration membranes: Treatment, pretreatment, modelling, and atomic force microscopy. N. Hilal, H. Al-Zoubi, N.A. Darwish, A.W. Mohammad, M. Abu Arabi. Desalination 2004,170:281-308). Daher eignen sich Trennverfahren mit geschlossenen

Membranen nicht, wenn große Stoffmengen abgetrennt werden sollen. Zur Separation von Stoffklassen wurde in jüngerer Zeit die Verwendung offenporiger Membranen mit nanoskalierten Kanaldurchmessern vorgeschlagen, womit theoretisch ein wesentlich höherer Stofftransport möglich ist, als mit geschlossenen Membranen (Molecular Sieving Using Nanofilters: Past, Present and Future. Jongyoon Han, Jianping Fu, Reto B. Schoch. Lab Chip. 2008, 8(1): 23-33). In neuerer Zeit wurden hierzu mikro- und nanofluidische Verfahren zur selektiven Trennung von Stoffgemischen vorgestellt. Dabei konnte gezeigt werden, dass hydrophile und hydrophobe Verbindungen, die zusammen in einem wässrigen Medium vorliegen, durch Membranen, die hoch geordnete nanoskalierte Kanäle aufweisen und mit einer hydrophilen oder hydrophoben Oberflächenbeschichtung versehen worden waren, mittels Diffusion selektiv abgetrennt werden können (Solvent-Extraction and Langmuir-Adsorption-Based Transport in Chemically Functionalized Nanopore Membranes. Damian J. Odom, Lane A. Baker, and Charles R. Martin. J. Phys. Chem. B 2005, 109, 20887-20894). Die Selektivität für die abzutrennenden Verbindungen wird dabei durch eine Oberflächenbeschichtung der Kanalwände erreicht. Hierdurch konnte für die Separation von apolaren Verbindungen ein Selektivitätsindex von bis zu fünf für einen diffusiven Stofftransport dokumentiert werden. Es wurde jetzt gefunden, dass die mit einer alleinigen Oberflächenbeschichtung von nanoskalierten Kanälen erreichbaren Eigenschaften nicht geeignet sind, um eine ausreichende Selektivität für die Separation von Carbonsäuren gegenüber anderen organischen Verbindungen, die Carbonsäuren ähnlich sind, in einem diffusiven Separationsprozess zu gewährleisten. Ferner besteht bei derartigen Membranen keine Selektivität für die Separation von unterschiedlichen organischen Verbindungen, wenn an die Trennmembran ein elektrischer Gradient angelegt wird. So zeigte sich, dass sich aus einer Lösung, die Albumin und gelöste Fettsäuren enthielt, Fettsäuren durch eine Membran, die Kanäle von 100nm aufwies und deren Oberflächen mit Alcylsilanen beschichtet waren, durch eine Diffusion in ein Akzeptormedium abtrennen lassen, wobei ein minimaler Transport von Albumin durch die Membran stattfindet. Wurde der gleiche Versuch mit Anlage eines transmembranösen elektrischen Gradienten durchgeführt, so kam es zu einem Durchtritt von Albumin und Fettsäuren durch eine solche Membran, der in gleichem Maße stattfand, eine Selektivität des Stofftransports bestand dann nicht mehr. Somit lässt sich durch eine Oberflächenbeschichtung, wie z. B. durch eine Hydrophobisierung der Filtermembranoberflächen, alleine ein selektiver elektrophoretischer Stofftransport für amphiphile Carbonsäuren nicht herstellen. Für elektrophoretische Separationen von organischen Verbindungen mit Membranen ist bekannt, dass es zu starken Foulingprozessen, insbesondere an anionenselektiven Membranen, kommt (Fouling of electrodialysis membranes by organic substances. Lindstrand, V; Sundstrom, G und Jönsson, Ann-Sofi LU (2000), in Desalination 128(1). p.102-91.) Ferner ist die Entstehung elektro-osmotischer Fluss-Phänomene bei einer Spannungsanlage an Membranen mit mikro- und nanofluidischen Kanälen beschrieben.

Aus dem bisherigen Stand der Technik ist demnach nicht ersichtlich, wie man stoffgruppenspezifische Trenneigenschaften für Carbonsäuren in eine offenporige Trennapparatur implementieren könnte.

Daher ist es Aufgabe der vorliegenden Erfindung, eine offenporige Trennmembran und ein Verfahren zur Abtrennung von Carbonsäuren und/oder Fettsäuren aus einer Lösung oder einer Emulsion durch eine Membran bereitzustellen. Ferner ist es Aufgabe der Erfindung, Verfahren zur Herstellung solcher Trennmembranen und Verfahren zur Trennung von Carbonsäuren und/oder Fettsäuren unter Verwendung solcher Trennmembranen bereitzustellen.

Diese Aufgabe wird erfindungsgemäß durch die technische Lehre der unabhängigen Ansprüche gelöst. Weitere vorteilhafte Ausgestaltungen der Erfindung ergeben sich aus den abhängigen Ansprüchen, der Beschreibung, den Figuren sowie den Beispielen.

Die vorliegende Erfindung betrifft ein Verfahren zur Herstellung von offenporigen Membranen zur diffusiven und/oder elektrophoretischen Abtrennung von Carbonsäuren aus flüssigen Stoffgemischen, umfassend die Schritte:

a) Bereitstellen einer offenporigen Trägermembran,
b) Einbringen einer Mono-und/oder Oligomerlösung in die offenporige Trägermembran aus Schritt a),
c) Reaktionsaktivierung zur Ausbildung eines offenporigen raumfüllenden nanocavitären polymeren Strukturnetzwerkes.

Ausgehend von Lösungen mit reaktionsfähigen Aminosäure-Monomeren kam es nach Einleitung einer nukleophilen Polymerisationsreaktion zur Ausbildung von kompakten, aber offenporigen Massen, die ein großes Aufnahmevermögen für Fettsäuren zeigten und aufgrund der Hydrophobie nicht mit Wasser benetzbar waren. Derartige Polymergebilde sind allerding spröde und fragil, da offenbar keine ausreichende Quervernetzung zwischen den Polymerketten während der Selbstassemblierung stattfindet. Es wurde daher versucht eine derartige Selbstassemblierung in vorgegebenen nanoskalierten Kanälen einzurichten, indem die Kanaloberflächen zunächst mit einer reaktionsinitiierenden Beschichtung, wie z.B. Aminosilanen, vollflächig beschichtet wurden. Eine statische oder dynamische Beschickung der Membrankanäle mit einer Monomer-Lösung brachte unterschiedliche Ergebnisse. Zum Teil kam es durch die Polymerisation zu einem vollständigen Verschluss der Kanäle und zum Teil nur zu einer oberflächlichen Belegung der Kanäle mit einer Polymerschicht. Während Membranen mit vollständig verlegten Kanälen nicht von Carbonsäuren passiert werden konnten, zeigten Membranen, bei denen eine oberflächliche Kanalbeschichtung erfolgt war, die mittels Raster-Elektronenmikroskopie quantifiziert werden konnte, eine Verstärkung des elektroosmotischen Flusses, bei Anlage einer

elektrischen Spannung in einer Elektrodialyse-Zelle.
Ein elektro-osmotischer Fluss kommt zustande durch eine Belegung von Oberflächen, z.B. in einer Membran, mit Ionen, die in einem elektrischen Feld bewegt werden. Die oberflächlich bewegliche Ionenschicht wird im elektrischen Feld transportiert, wodurch die an die Ionen gebundene Wasserhülle mittransportiert wird und somit ein Wasservolumentransport entsteht. Aus der Literatur ist bekannt, dass es bei einer Elektrodialyse mit Membranen, die mikrofluidische Kanäle aufweisen, zur Ausbildung eines elektroosmotischen Flusses (EOF) kommt. Bei einer negativ geladenen Oberfläche der Kanalwände ist die Richtung des EOF entgegengesetzt zur elektro-kinetischen Transportrichtung von Anionen. Bei einer Elektrodialyse von gelösten Fettsäuren mit Membranen, deren Nanokanäle mit hydrophoben Verbindungen beschichtet worden waren, kam es ebenfalls zur Ausbildung eines EOF. Dieses Phänomen kann erklärt werden durch eine Adsorption von Fettsäuren an den Kanalwandoberflächen, die über hydrophobe Wechselkräfte zustande kommt, wodurch an den Kanalwandoberflächen eine negative Ladung durch deprotonierte Carboxylgruppen entsteht. Bei Elektrodialyse-Untersuchungen, die mit derartigen Membranen erfolgten, um Carbonsäuren, die in einem wässrigen Medium gelöst vorlagen, aus diesem in ein anderes wässriges Medium abzutrennen, zeigte sich, dass durch den EOF der elektrokinetische Transport der Carbonsäuren erheblich eingeschränkt wird. Nach Einrichten eines pneumatischen Drucks in der Donorkammer, der dem Druckaufbau, der durch den EOF erreicht wird, entsprach, steigerte sich die Menge an elektro-kinetisch transportierten Carbonsäuren. Die Einrichtung eines Druckaufbaus in einer solchen Vorrichtung, der zu einer Unterbindung eines elektro-osmotischen Flusses führt, ist schwierig, da die Stärke des elektro-osmotischen Flusses von vielen Faktoren, wie der Ionenkonzentration des Akzeptor- und Donormediums sowie von der angelegten Spannung sowie weiteren Faktoren (z. B. der Oberflächenladung der Membran-Kanäle) abhängt.
Da ein EOF die Effektivität einer Separation von Carbonsäuren, die mit einer Elektrodialyse erreicht werden kann, beeinträchtigt, ist es auch die Aufgabe der Erfindung, eine Membran bereitzustellen, bei der kein oder nur ein geringer elektro - osmotischer Fluss entsteht, sodass die Einrichtung eines pneumatischen Gegendrucks nicht erforderlich ist.
Überraschenderweise wurde gefunden, dass bei erfindungsgemäß hergestellten offenporigen Membranen kein elektroosmotischer Fluss bei einer elektrophoretischen Abtrennung von Carbonsäuren entsteht.

Die vorliegende Erfindung betrifft demnach ein Verfahren zur Herstellung von offenporigen Membranen zur diffusiven und/oder elektrophoretischen Abtrennung von Carbonsäuren aus flüssigen Stoffgemischen, umfassend die Schritte:

a) Bereitstellen einer offenporigen Trägermembran,
b) Einbringen einer Mono-und/oder Oligomerlösung in die offenporige Trägermembran aus Schritt a),
c) Reaktionsaktivierung zur Ausbildung eines offenporigen raumfüllenden nanocavitären polymeren Strukturnetzwerkes,

wobei die offenporigen Membranen einen elektro-osmotischen Fluss durch die offenporigen Membranen verhindern.

Bevorzugt ist die Herstellung von selbst-assemblierten nanocavitären Polymerstrukturen zur Reduktion/Unterbindung eines elektro-osmotischen Flusses.

Ausgehend von den Ergebnissen einer oberflächlichen Beschichtung von Nanokanälen mit hydrophoben Polymeren wurde durch eine Veränderung der Reaktionsparameter (u.a. Zeitdauer, Reaktionstemperatur, Konzentration der Monomere) ein höherer vollflächiger polymerer Schichtaufbau in den Nanokanälen erreicht, wodurch es zu einer weiteren Verkleinerung der freien Kanallichtungen kam. Dies führte allerdings zu einer Zunahme des EOF und einer Verringerung der Abtrenneffizienz für Carbonsäuren.
Zu einem überraschenden Ergebnis führte die Verwendung von Verbindungen in den Lösungen mit reaktiven Mono-/Oligomeren, die eine Polymerisationsreaktion der reaktiven Mono-/Oligomere fördern oder bedingen können. Bei derartigen Zubereitungen entstanden auf planaren Flächen Polymere, die elektronenmikroskopisch irreguläre Strukturen zeigten, welche gewebeartige Texturen aufwiesen, aber als eigenständige Schicht bzw. Masse fragil waren. Sie waren porös und konnten flüssige Carbonsäuren rasch in das dreidimensionale Netzwerk, das sich ausgebildet hatte, aufnehmen. Mit Wasser waren sie nicht benetzbar. Derartige Schichten/Massen ließen sich leicht mechanisch von der Auflagefläche entfernen und zerfielen in kleinste Partikel. Wurden poröse Materialien mit reaktionsfähigen Mono-/Oligomerlösungen befüllt und eine Polymerisationsreaktion durch eine Reaktionsaktivierung gestartet, wie z.B. durch Lösungsmittel oder physikalische Maßnahmen, so entstanden gleichartige gewebeartige Strukturen in den porösen Materialien, die den gesamten Rauminhalt der Kanäle/Spalten ausfüllten, die aber offenporig blieben, da die gewebeartigen Polymerstrukturen nanoskalierte Spalträume ausbildeten. Derartig behandelte Membranen nahmen ebenfalls flüssige Carbonsäuren auf und erlaubten keine Aufnahme von Wasser. Elektronenmikroskopisch zeigte sich, dass bei derartigen Membranen Polymerstrukturen entstanden waren, die Texturen aufweisen, die strangartig sein können und ein dreidimensionales Netzwerk ausbilden aber auch aus flächigen Gewebestrukturen vorliegen können, die parallel oder in einer irregulären Anordnung zueinander vorliegen und bei denen die polymeren Gewebestrukturen den gesamten Raum, der durch die raumbegrenzenden inneren Strukturen der Trägermembran vorgegeben war, ausfüllten. Die entstandenen Polymerstrukturen wurden durch physikalische Maßnahmen, wie durch eine Druckspülung mit Wasser oder organischen Lösungsmitteln sowie durch ein Ultraschallbad nicht in Ihrer Struktur oder ihren Eigenschaften verändert. Mittels Porosimetrie sowie elektronenmikroskopischen Untersuchungen konnte gezeigt werden, dass die dreidimensionalen raumfüllenden Polymerstrukturen irregulär konfigurierte Spalträume begrenzten, die miteinander offen verbunden waren und eine Kommunikation zwischen beiden Außenseiten der behandelten Membranen gewährleisteten. Derartige Membranen waren somit nicht verschlossen. Bei Membranen, die mit raumfüllenden Polymerstrukturen ausgekleidet waren, kam es überraschenderweise bei einer Elektrodialyse von wässrigen Medien mit hierin gelösten Fettsäuren zu praktisch keinem EOF, bei einer gleichzeitig hohen Transportrate für Fettsäuren.

Der Erfinder konnte ferner zeigen, dass durch geeignete Wahl der Polymerisationsbedingungen dreidimensionale gewebeartige Polymerstrukturen, die nanocavitäre Spalträume entstehen lassen, durch eine Selbstassemblierung innerhalb der raumgebenden offenporigen Trägermembran gebildet werden, die offenporig sowie permeabel für Carbonsäuren und Gase sind und einen elektroosmotischen Fluss verhindern. Nicht erfindungsgemäß ist dahingegen das Füllen der raumgebenden offenporigen Trägermembran mit einem Polymer, das durch eine Polymerisation erzeugt wurde, sodass hierdurch die Poren, Kanäle oder Schlitze durch das ausgebildete Polymer verschlossen werden. Ebenso ist das oberflächliche Beschichten der Poren, Kanäle oder Schlitze der raumgebenden offenporigen Trägermembran mit einem Polymer nicht erfindungsgemäß. Die erfindungsgemäße raumfüllenden offenporigen Polymerstrukturen werden vorzugsweise durch eine "Selbstassemblierung" von Mono-/Oligomeren zu polymeren Texturen erreicht und sind mit den Oberflächen der offenporigen Trägermembran sowie untereinander kovalent verbunden.

Überraschenderweise konnte gezeigt werden, dass eine selektive Separation von Carbonsäuren aus einem wässrigen Stoffgemisch durch offenporige Polymerstrukturen, die durch eine Selbst-Assemblierung erhalten wurden, ermöglicht werden kann. Der Begriff "Selbst-Assemblierung", wie hierin verwendet, bezieht sich auf die Ausbildung von dreidimensionalen molekularen zusammenhängenden Strukturen, die durch eine Polymerisationsreaktion entstehen. Dabei kann die Polymerisation ausgehend von Monomeren oder Oligomeren durchgeführt werden.

Anders formuliert betrifft die vorliegende Erfindung ein Verfahren zur Herstellung von offenporigen Membranen zur diffusiven und/oder elektrophoretischen Abtrennung von Carbonsäuren aus flüssigen Stoffgemischen, umfassend die folgenden Schritte:

a) Bereitstellen einer raumgebenden offenporigen Trägermembran,
b) Einbringen einer Mono-und/oder Oligomerlösung in die offenporige Trägermembran aus Schritt a),
c) Bilden raumfüllender nanocavitärer Polymerstrukturen innerhalb der besagten offenporigen Trägermembran mittels Polymerisationsreaktion unter Erhalt der Offenporigkeit der Membran.

Noch anders formuliert betrifft die vorliegende Erfindung ein Verfahren zur Herstellung von offenporigen Membranen zur diffusiven und/oder elektrophoretischen Abtrennung von Carbonsäuren aus flüssigen Stoffgemischen, umfassend die folgenden Schritte:

a) Bereitstellen einer offenporigen Trägermembran,
b) Einbringen einer Mono-und/oder Oligomerlösung in die offenporige Trägermembran aus Schritt a),
c) Bilden von raumfüllenden nanocavitären Polymerstrukturen innerhalb der besagten offenporigen Trägermembran mittels Reaktionsaktivierung der Polymerisationsreaktion.

Bevorzugt ist ein Verfahren, zur Herstellung von nanocavitären Polymerstrukturen, die einen selektiven Stofftransport für Carbonsäuren ermöglichen, mit einem Selektivitätsindex gegenüber dem korrespondierenden Alkohol von > 4.

Folglich sind die erfindungsgemäß hergestellten offenporösen Membranen geeignet zur kontinuierlichen Abtrennung von Carbonsäuren, vorzugsweise von Fettsäuren, aus flüssigen Mischungen, enthaltend die Carbonsäuren und vorzugsweise die Fettsäuren, wobei sich diese Mischung auf der einen Seite einer erfindungsgemäß hergestellten Membran mit durchgängigen Poren, Kanälen oder Schlitzen befindet und die Carbonsäuren und vorzugsweise die Fettsäuren durch diese durchgängigen Poren, Kanäle oder Schlitze auf die andere Seite der Membran transportiert werden, mit einem Selektivitätsindex >4 in Bezug auf den aus der Carbonsäure durch Reduktion erhältlichen Alkohol.

Demnach betrifft die vorliegende Erfindung ein Verfahren zur Herstellung von offenporigen Membranen zur diffusiven und/oder elektrophoretischen Abtrennung von Carbonsäuren aus flüssigen Stoffgemischen, umfassend die folgenden Schritte:

a) Bereitstellen einer porösen offenporigen Trägermembran,
b) Einbringen einer Mono-und/oder Oligomerlösung in die offenporige Trägermembran aus Schritt a),
c) Bilden einer nanocavitären Polymerstruktur innerhalb der besagten offenporigen Trägermembran mittels Polymerisationsreaktion unter Erhalt der offenen Poren,

wobei die offenporigen Membranen einen Selektivitätsindex für Carbonsäuren gegenüber dem korrespondierenden Alkohol >4 aufweisen und einen selektiven Stofftransport von Carbonsäuren ermöglichen.

Als vorteilhaft für die Ausbildung raumfüllender Polymerstrukturen stellte sich eine hohe Konzentration der Monomere in der Reaktionslösung dar. Dies bedingt allerdings eine hohe Viskosität der Reaktionslösung, die eine Einbringung in Nano- oder Mikrokanäle praktisch unmöglich macht. Es konnte gezeigt werden, dass sich Reaktionslösungen mit einem hohen Gehalt an Monomeren in verschiedene Trennmedien, die Kanal- und/oder Schlitzstrukturen im Mikrometer- bis Millimeterbereich aufweisen, einbringen lassen und dass eine radikalische oder nukleophile Polymerisation durch verschiedene Startreaktionen initiiert werden kann, die zur Ausbildung der nanocavitären Polymerstrukturen führt. Nach erfolgter Polymerisation bestehen in derartigen Membranen raumfüllende Strukturgebilde/Texturen, die nanoskalierte Kavitäten ausbilden, welche miteinander verbunden sind.

Derartige Membranen sind offenporig, da ein Gasstrom derartige Membranen passieren konnte. Der Begriff "offenporig", wie hierin verwendet, bezieht sich auf durchgängige Poren, die von einer Seite der Membran zur anderen Seite verlaufen. Das heißt, die Poren besitzen mindestens einen Poreneingang auf der einen Seite der Membran und mindestens einen Porenausgang auf der anderen Seite. Sie stellen somit eine Verbindung von der einen Seite der Membran zur anderen her und ermöglichen einen Stofftransport durch die Membran. Unter offenporig wird demnach nicht verstanden, dass eine Pore nur eine Öffnung (Dead-End-Pore) oder zwei oder mehr Öffnungen auf nur einer Membranseite besitzt. Offenporig schließt neben Poren auch Kanäle, Schlitze und beliebige andere Formen von durchgehenden Verbindungen zwischen den beiden Membranseiten ein.

Elektronenmikroskopisch unterscheiden sich die so hergestellten polymeren Strukturen von denen, die durch eine radikale Startreaktion infolge eines Kontakts mit einer beschichteten Oberfläche, im Sinne eines "grafting from"-Polymerisationsverfahrens initiiert und durch eine selbstunterhaltende Polymerisation erhalten wurden, dadurch, dass die Polymerschicht keine geschlossene Masse, sondern offenporig war, mit Ausbildung von filamentären bis membranösen Strukturen, die raumfüllend irregulär begrenzte Spalträume formierten. Daher sind eine Initiierung einer radikalischen oder nukleophilen Polymerisationsreaktion in einer Monomer- und/oder Oligomerlösung durch Zugabe eines chemischen Initiators und/oder eine physikalische Reaktionsinitiierung sowie Verfahren zur Beschleunigung einer Polymerisationsreaktion einer Lösung mit reaktiven Mono-/Oligomeren besonders bevorzugte Ausführungsformen zur Herstellung der erfindungsgemäßen Membranen. In einer weiteren bevorzugten Ausführungsform der vorliegenden Erfindung umfasst das Verfahren zur Herstellung von offenporigen Membranen zur diffusiven und/oder elektrophoretischen Abtrennung von Carbonsäuren aus flüssigen Stoffgemischen die folgenden Schritte:

a) Bereitstellen einer offenporigen Trägermembran,
b) Einbringen einer Mono-und/oder Oligomerlösung in die offenporige Trägermembran aus Schritt a),
c) Reaktionsaktivierung zur Ausbildung eines offenporigen raumfüllenden nanocavitären polymeren Strukturnetzwerkes,

wobei die Polymerisationsreaktion in Stufe c) durch die Hinzugabe oder Applikation eines chemischen und/oder physikalischen Starters einer radikalischen oder nukleophilen Polymerisation erfolgt.

Bevorzugt ist ein Verfahren, bei dem eine reaktionsfähige Monomerlösung in eine mikro- und/oder makroporöse Trägermembran vollständig eingebracht wird und die die Polymerisation durch die Lösungsmittelphase initiiert wird.

In einer weiteren bevorzugten Ausführungsform der vorliegenden Erfindung umfasst das Verfahren zur Herstellung von offenporigen Membranen zur diffusiven und/oder elektrophoretischen Abtrennung von Carbonsäuren aus flüssigen Stoffgemischen die folgenden Schritte:

a) Bereitstellen einer offenporigen mikro- und/oder makroporösen Trägermembran,
b) Vollständiges Einbringen einer reaktionsfähigen Monomerlösung in die offenporige Trägermembran aus Schritt a),
c) Reaktionsinitiierung mittels Lösungsmittelphase zur Ausbildung eines offenporigen raumfüllenden nanocavitären polymeren Strukturnetzwerkes.

Überraschender Weise kommt es bei der Verwendung von offenporigen Membranen mit raumfüllenden nanocavitären Polymerstrukturen zu keinem oder nur minimalen EOF während einer Elektrodialyse von Carbonsäuren.

Bevorzugt ist die Herstellung von selbst-assemblierten nanocavitären Polymerstrukturen zur Reduktion/Unterbindung eines elektro-osmotischen Flusses.

In einer anderen besonders bevorzugten Ausführungsform werden/wird die erfindungsgemäße Membran und/oder eine separate Membran mit einem Polykation beschichtet. Die Beschichtung kann in Form einer Adsorption und/oder einer kovalenten Bindung an die Oberfläche des Trägermaterials erfolgen. Dabei kann die Beschichtung oberflächlich aufgebracht werden, vorzugsweise auf der Seite, die dem Akzeptormedium zugewandt ist. Die Beschichtung kann aber auch in die Spalträume der Membran eingebracht werden. Verfahren hierzu sind im Stand der Technik bekannt. In einer besonders bevorzugten Ausführungsform, wird eine frei-tragende Schicht, die aus polykationischen

Verbindungen besteht, verwandt, indem diese mit einer erfindungsgemäßen Membran zusammengebracht wird. Dabei bedeutet Zusammenbringen einen engen räumlichen Kontakt, der z. B. dadurch erreicht wird, indem die Membranen durch eine geeignete Haltevorrichtung aneinander gepresst werden, wodurch eine Spaltbildung zwischen beiden Membranen verhindert wird. Eine derartige Anordnung erfolgt auch bei Verwendung einer separaten Membran, die mit einem Polykation beschichtet wurde.

Bevorzugt ist ein Verfahren, bei dem eine Unterbindung eines elektroosmotischen Flusses dadurch erfolgt, indem in der optionalen Stufe d2) polykationische Verbindungen physiosorptiv und/oder chemosorptiv auf die nanocavitären Polymerstrukturen aufgebracht werden.

Bevorzugt ist ein Verfahren zur Unterbindung des elektro-osmotischen Flusses, das dadurch erreicht wird, dass die Separationsmembran und/oder eine weitere Membran adsorptiv und/oder chemosorptiv mit einer/mehreren polykationischer(en) Verbindung(en) beschichtet wird/werden und im Falle einer separaten Membran diese in engen räumlichen Kontakt mit der Separationsmembran gebracht wird. Bevorzugt ist ein Verfahren, bei dem eine Unterbindung des elektroosmotischen Flusses dadurch hergestellt wird, indem eine frei-tragende Membran, die aus polykationischen Verbindungen besteht oder in der polykationische Verbindungen integriert sind, in engen räumlichen Kontakt mit der Separationsmembran gebracht wird.

Ein anderer Aspekt der Erfindung betrifft die Oberflächenladung der nanocavitären Polymerstrukturen in den erfindungsgemäß hergestellten Membranen. Die Oberflächenladung lässt sich durch eine transmembranöse Bestimmung des Zetapotentials ermitteln. Vorzugsweise beträgt das Zetapotential der erfindungsgemäßen Membran 0 mV. In weiteren bevorzugten Ausführungsformen weisen die erfindungsgemäßen Membranen ein Zetapotential auf das ungleich 0 mV ist und in einem Bereich zwischen +40mV und -80mV liegt. In Abhängigkeit von der Zusammensetzung und den Eigenschaften (z.B. pH-Wert) der wässrigen Medien, aus denen Carbonsäuren durch eine erfindungsgemäße Membran separiert werden sollen, kann es erforderlich sein, positive und/oder negative Ladungsgruppen an den Oberflächen der nanocavitären Polymerstrukturen bereitzustellen. Dies kann erreicht werden, indem ionische oder ionisierbare Verbindungen in die Polymerstrukturen integriert werden. Da positive oder negative Ladungsgruppen die Polymerisationsreaktion der Stufe c) stören können, werden in einer Ausführungsform die ionischen oder ionisierbaren Verbindungen mit einer Schutzgruppe versehen und dem Reaktionsgemisch in Stufe b) hinzugegeben. In einer weiteren Ausführungsart werden diese Schutzgruppen in der optionalen Stufe d1) durch geeignete Reagenzien oder physikalische Maßnahmen abgespalten und anschließend aus der Membran entfernt. In einer anderen Ausführungsart werden ionische und/oder ionisierbare Verbindungen in dem optionalen Verfahrensschritt d2) an den Oberflächen der nanocavitären Polymerstrukturen physio- und/oder chemosorptiv angebunden. Hierzu kann es erforderlich sein, zunächst reaktionsfähige Gruppen an den Polymerstrukturen herzustellen. Verfahren hierzu sind im Stand der Technik bekannt. Nach einer nachträglichen physio- und/oder chemosorptiven Anlagerung von ionischen oder ionisierbaren Verbindungen sowie nach der Abspaltung von Schutzgruppen werden derartige Membranen sorgfältig von nicht umgesetzten oder abgespaltenen Verbindungen gereinigt. In diesen optionalen Verfahrensstufen können skalierbare Oberflächenladungen der erfindungsgemäßen Membranen hergestellt werden, die vorzugsweise in einem Bereich von + 0,1 mV bis + 40mV liegen, sofern ein positives Zetapotential gewünscht ist und in einem Bereich von - 0,1 mV bis - 80 mV liegen, sofern ein negatives Zetapotential erforderlich ist. Bevorzugt ist ein Verfahren, bei dem Schutzgruppen der in Stufe c) umgesetzten Mono-/Oligomere in Stufe d1) durch geeignete Reagenzien oder physikalische Maßnahmen abgespalten und anschließend aus der Membran entfernt werden.

In einer bevorzugten Ausführungsform der vorliegenden Erfindung umfasst das Verfahren zur Herstellung von offenporigen Membranen zur diffusiven und/oder elektrophoretischen Abtrennung von Carbonsäuren aus flüssigen Stoffgemischen, die folgenden Schritte:

a) Bereitstellen einer raumgebenden offenporigen Trägermembran,
b) Einbringen einer Mono-und/oder Oligomerlösung in die offenporige Trägermembran aus Schritt a),
c) Reaktionsaktivierung zur Ausbildung eines offenporigen raumfüllenden nanocavitären polymeren geschützten Strukturnetzwerkes,
d1) Abspalten der Schutzgruppen,

wobei die eingebrachte Mono-und/oder Oligomerlösung geschützte Monomere und/oder geschützte Oligomere enthält und die Monomere und/oder Oligomere ionisch und/oder ionisierbar sind.

Bevorzugt ist ein Verfahren, bei dem ionische und/oder ionisierbare Verbindungen in einem Verfahrensschritt d2) an den Oberflächen der nanocavitären Polymerstrukturen physio- und/oder chemosorptiv angebunden werden.

In einer weiteren bevorzugten Ausführungsform der vorliegenden Erfindung umfasst das Verfahren zur Herstellung von offenporigen Membranen zur diffusiven und/oder elektrophoretischen Abtrennung von Carbonsäuren aus flüssigen Stoffgemischen die folgenden Schritte:

a) Bereitstellen einer offenporigen Trägermembran,

b) Einbringen einer Mono- und/oder Oligomerlösung in die offenporige Trägermembran aus Schritt a),

c) Reaktionsaktivierung zur Ausbildung eines offenporigen raumfüllenden nanocavitären polymeren Strukturnetzwerkes, und

d2) Binden einer ionischen und/oder ionisierbaren Verbindung an das offenporige raumfüllende nanocavitäre polymere Strukturnetzwerk.

Ein weiterer Aspekt der Erfindung betrifft die physikalischen Eigenschaften der erfindungsgemäßen Membranen. Derartige Membranen sind einerseits hydrophob und andererseits lipophil. Überraschenderweise kam es auch bei einer längeren Anwendungsdauer (> 200h) zu keiner Reduktion des Transports von Carbonsäuren bei einer Elektrodialyse, wenn im Donormedium Proteine oder Phospholipide gelöst vorlagen. Daher sind die erfindungsgemäßen Membranen auch resistent gegenüber einem Fouling durch Verbindungen, die aufgrund ihrer physiko-chemischen Eigenschaften und ihrer Dimensionen, in die erfindungsgemäßen Membranen eintreten und diese belegen können.
Bevorzugt ist ein Verfahren zur Herstellung von Membranen, durch das ein Fouling der Membranen durch organische Verbindungen verhindert wird.
Ein weiterer überaus vorteilhafter Effekt, der mit den erfindungsgemäßen Membranen bewerkstelligt wird, ist eine sehr gute Biokompatibilität. So konnte gezeigt werden, dass es bei einem Kontakt mit biologischen Flüssigkeiten nur zu einer geringen bis kaum messbaren Anhaftung von Biomolekülen kommt. Insbesondere wurde keine Anhaftung von lebenden Zellen beobachtet. Hierdurch kommt es auch zu einer Hemmung von ansonsten üblichen Fouling-Prozessen, die bei Trennmembranen, die bei biologischen Flüssigkeiten eingesetzt werden, auftreten. Der Anti-Fouling-Effekt wird dadurch unterstützt, indem die Separation von Carbonsäuren ohne eine Druckbeaufschlagung des Donormediums erfolgt.
Die Oberflächen der erfindungsgemäßen Membranen weisen darüber hinaus auch eine sehr gute Bio- und Hämokompatibilität auf. Insbesondere für Membranen, die erfindungsgemäß mit Lösungen, enthaltend Polyphenylalanin-Monomere mit einem der hierin offenbarten Verfahren hergestellt wurden, kam es zu keiner Anhaftung von Blutproteinen oder Blutzellen im Verlauf von 6 Stunden. Daher sind die erfindungsgemäßen Membranen auch bio- bzw. hämokompatibel.
Bevorzugt ist die Herstellung von nanocavitären Polymerstrukturen, deren Untereinheiten bevorzugt zu > 25Gew%, mehr bevorzugt zu > 50Gew%, weiter bevorzugt zu > 75Gew% und besonders bevorzugt zu > 90Gew% aus Biomono-/Oligomeren aufgebaut sind. Bevorzugt ist die Herstellung von nanocavitären Polymerstrukturen, deren Untereinheiten bevorzugt zu > 25Gew%, mehr bevorzugt zu > 50Gew%, weiter bevorzugt zu > 75Gew% und besonders bevorzugt zu > 90Gew% aus Phenylalanin und/oder Phenylalaninderivaten aufgebaut sind.
Bevorzugt ist ein Verfahren zur Herstellung von bio- und hämokompatiblen Membranen.
In einer bevorzugten Ausführungsform der vorliegenden Erfindung umfasst das Verfahren zur Herstellung von offenporigen biokompatiblen und hämokompatiblen Membranen zur diffusiven und/oder elektrophoretischen Abtrennung von Carbonsäuren aus flüssigen Stoffgemischen die folgenden Schritte:

a) Bereitstellen einer raumgebenden offenporigen Trägermembran,

b) Einbringen einer Phenylalanin- und/oder Oligophenylalaninlösung in die offenporige Trägermembran aus Schritt a),

c) Reaktionsaktivierung zur Ausbildung eines offenporigen raumfüllenden nanocavitären Polyphenylalanin-Strukturnetzwerkes.

In einer weiteren bevorzugten Ausführungsform der vorliegenden Erfindung umfasst das Verfahren zur Herstellung von offenporigen Membranen zur diffusiven und/oder elektrophoretischen Abtrennung von Carbonsäuren aus flüssigen Stoffgemischen die folgenden Schritte:

d) Bereitstellen einer offenporigen Trägermembran,

e) Einbringen einer Mono- und/oder Oligomerlösung in die offenporige Trägermembran aus Schritt a),

f) Reaktionsaktivierung zur Ausbildung eines offenporigen raumfüllenden nanocavitären polymeren Strukturnetzwerkes,

wobei die Mono- und/oder Oligomerlösung Biomonomere und/oder Biooligomere enthält.
Ein besonders vorteilhafter Effekt, der sich insbesondere durch die erfindungsgemäße Beschichtung einer Trägermembran mit Aminosäurepolymeren ergibt, ist ihre Beständigkeit gegenüber organischen Lösungsmitteln. So wiesen Membranen, bei denen nach einem erfindungsgemäßen Verfahren eine Polymerisation mit Polyphenylalanin- oder Benzylglutamat Monomeren erfolgt war, elektronenmikroskopisch keine strukturellen Veränderungen auf, wenn sie über 3 Wochen in THF oder Hexan eingelegt worden waren.
Bevorzugt ist ein Verfahren zur Herstellung von lösungsmittelresistenten Membranen.
In einer bevorzugten Ausführungsform werden die erfindungsgemäßen nanocavitären Polymerstrukturen durch einen Polymerisationsvorgang hergestellt, der durch eine nukleophile Reaktionsinitiierung einer Monomerlösung in-situ

gestartet wird. Diese Initiierung wird hier als "Reaktionsaktivierung" oder "Reaktionsinitiierung" bezeichnet. Hierunter fallen nukleophile und radikalische Reaktionen, die eine kovalente Bindung zwischen einem oder mehreren Mono-/Oligomeren oder eine kovalente Bindung mit anderen Verbindungen (z.B. organischen Verbindungen auf Oberflächen der raumgebenden Trägermembran) bewirken. Dabei können Reaktionsprodukte freigesetzt werden. In einer besonders bevorzugten Ausführungsform wird durch die Reaktionsprodukte, die Ausbildung der nanocavitären Polymerstrukturen zusätzlich begünstigt. Unter dem Begriff Reaktionsinitiierung werden ferner geeignete Maßnahmen zu einem multifokalen Start der Polymerisation verstanden, so dass nanocavitäre Raumstrukturen innerhalb der raumgebenden offenporigen Trägermembran gebildet werden. Mit anderen Worten be-/verhindert die Reaktionsinitiierung einen gleichmäßigen Schichtaufbau bzw. die Ausbildung kompakter Verbände der sich ausbildenden Polymere durch eine multifokales Polymerwachstum. Geeignete Maßnahmen zur Reaktionsaktivierung umfassen: Zugabe von Startern von Polymerisationsreaktionen, Erhöhen der Temperatur, Senken der Temperatur, Zugabe eines Lösungsmittels, Bestrahlen, Erhöhung oder Erniedrigung des Druckes, sowie bekannte Verfahren aus dem Stand der Technik. Die Reaktionsaktivierung verhindert die Ausbildung einer kompakten Schicht des Polymers auf den Oberflächen des raumgebenden Stützgewebes sowie eines Verschluss der Poren einer porösen Membran.

Vorzugsweise liegen die Mono- bzw. Oligomere in Form einer Lösung oder Suspension in einem geeigneten Lösungsmittel vor. Bevorzugte Lösungsmittel sind, Acetonitril, THF, 1,4-Dioxan, N,N-Dimethylformamid (DMF) Dichlormethan, Chloroform, Methyl-tert-butylether oder ähnlichen Lösungsmitteln, wie z. B. N,N-Dimethylacetamid (DMA) und N-Methylpyrrolidon (NMP), DMSO, Wasser, Methanol, Toluol, Xylen, Anisol sowie andere organische Lösungsmittel und Kombinationen hiervon oder hiermit. Die Wahl der Monomer-/Oligomerkonzentration hängt von den sich hieraus ergebenden physikalischen Eigenschaften und der Löslichkeit ab. Vorzugsweise ist die Konzentration auszuwählen aus einem Bereich zwischen 1 mmol/l und 3 mol/l, mehr bevorzugt zwischen 10mmol/l und 1 mol/l und weiter bevorzugt zwischen 100mmol/l und 0,5 mol/l.

In einer bevorzugten Ausführungsform der vorliegenden Erfindung umfasst das Verfahren zur Herstellung von offenporigen Membranen zur diffusiven und/oder elektrophoretischen Abtrennung von Carbonsäuren aus flüssigen Stoffgemischen die folgenden Schritte:

d) Bereitstellen einer raumgebenden offenporigen Trägermembran,
e) Einbringen einer Mono-und/oder Oligomerlösung in die offenporige Trägermembran aus Schritt a),
f) Reaktionsaktivierung zur Ausbildung eines offenporigen raumfüllenden nanocavitären polymeren Strukturnetzwerkes,

wobei die Monomer- und/oder Oligomer-Konzentration der besagten Mono-und/oder Oligomerlösung zwischen 1 mmol/l und 3 mol/l liegt.

In einer bevorzugten Ausführungsform erfolgt die Herstellung von nanocavitären Polymerstrukturen, indem eine geeignete und vorbereitete struktur- und raumgebende Membran bis zur Sättigung mit der reaktionsfähigen Mono-/Oligomerlösung befüllt wird. Die Befüllung kann durch Tränken, Aufgießen, Einlegen mit/in der/die Monomer-/Oligomerlösung oder durch einen kontinuierlichen oder diskontinuierlichen Volumenstrom der Mono-/Oligomerlösung durch die Membran erfolgen. Dabei kann es erforderlich sein, die Temperatur abzusenken oder zu erhöhen. Bevorzugt ist die Einbringung der Mono-/Oligomerlösung in die strukturgebende Membran bei einer Temperatur von -10 bis 100°C, mehr bevorzugt zwischen 0°C und 80°C und weiter bevorzugt zwischen 10°C und 40°C.
In einer weiter bevorzugten Ausführungsform werden die erfindungsgemäßen nanocavitären Polymerstrukturen durch einen Polymerisationsvorgang hergestellt, der durch eine radikalische oder nukleophile Reaktionsinitiierung einer Monomer- und/oder Oligomerlösung in-situ gestartet wird.
In einer weiteren bevorzugten Ausführungsform der vorliegenden Erfindung umfasst das Verfahren zur Herstellung von offenporigen Membranen zur diffusiven und/oder elektrophoretischen Abtrennung von Carbonsäuren aus flüssigen Stoffgemischen die folgenden Schritte:

a) Bereitstellen einer raumgebenden offenporigen Trägermembran,
b) Einbringen einer Mono-und/oder Oligomerlösung in die offenporige Trägermembran aus Schritt a),
c) Reaktionsaktivierung zur Ausbildung eines offenporigen raumfüllenden nanocavitären polymeren Strukturnetzwerkes,

wobei die Reaktionsaktivierung radikalisch oder nukleophil *in situ* erfolgt.

Starter einer Reaktion der Mono-/Oligomere sind Substanzen, die eine ringöffnende oder radikalische Polymerisation initiieren können, wie sie aus dem Stand der Technik bekannt sind. Hierzu zählen u.a. Lösungsmittel, wie Wasser, DMF, NMP, DMSO, Tetramethylharnstoff. Nucleophile, wie Amine, Amide, Alkoholate, Hydroxidionen, Thiolate, Triethylamin, Ammoniak, Pyridine, wie 4-Dimethylaminopyridin, Phosphine, Carbene, wie Imidazol-2-ylidene und Imidazolin-2-ylidene oder Thiazol-2-ylidene. Kationische Katalysatoren, wie Trifluormethansulfonsäure und Methyltrifluormethansulfonat, bifunktionale Organokatalysatoren, wie [1-(3,5-bis(trifluoromethyl)phenyl)-3-(2-dimethylamino-cyclohexyl)thiourea] oder [1,5,7-triazabicyclo(4.4.0)dec-5-ene (TBD). Katalysatoren, wie Grubb's Katalysator, Metallionen, wie Kupfer, Zinn, z.B. als Zinnoctanoat ($Sn(Oct)_2$), Aluminumalkoxide $Al(OR)_3$, Titaniumalkoxide ($Ti(OR)_4$), Kobalt oder Nickel, Säuren, wie Ascorbinsäure, Schwefelsäure oder Phosphorsäure, weiterhin Azo-Verbindungen, wie AIBN oder Peroxide, wie Benzoylperoxid.

In einer bevorzugten Ausführungsform werden die Substanzen/Verbindungen, die eine Startreaktion initiieren, unmittelbar vor dem Einbringen der Monomer-/Oligomerlösung in eine Trägerstruktur der Mono-/Oligomerlösung hinzugegeben und/oder wurden zuvor auf die Oberflächen der Trägerstruktur aufgebracht. Die ringöffnende Polymerisation erfolgt vorzugsweise mit einer nukleophil induzierten Polymerisation, vorzugsweise werden zuvor Amine auf die Oberflächen der Trägerstrukturen aufgebracht. In einer Ausführungsform wird der Mono-/Oligomerlösung ein Starter einer radikalischen Polymerisation unmittelbar vor dem Einbringen in das Stützgewebe hinzugemischt.

Besonders bevorzugt ist die Verwendung von Lösungsmitteln, die eine nukleophile Reaktion initiieren können, wie DMF, DMA oder NMP. Weiter bevorzugt ist die Verwendung von Lösungsmittelkombinationen. In einer bevorzugten Ausführungsform wird einer Mono- bzw. Oligomerlösung, die einem Lösungsmittel vorliegt, das keine nukleophile Reaktion initiiert, ein Lösungsmittel das eine nuckeophile Reaktion bewirkt, unmittelbar vor dem Einbringen der Lösung in die offenporige Trägermembran hinzugegeben. So kann beispielsweise eine ringöffnende Polymerisation von Aminosäure-Monomeren, die in THF gelöst vorliegen, durch die Hinzugabe von 10Vol% DMF initiiert werden.

Bevorzugt ist die Initiierung der radikalischen oder ringöffnenden Startreaktion in der Lösungsmittelphase durch physikalische Maßnahmen. Hierzu gehört eine Erwärmung des Substrates, bevorzugt ist eine Erwärmung auf 40 bis 160°C, mehr bevorzugt auf 60° bis 120°C und weiter bevorzugt auf 80° bis 110°C. In einer bevorzugten Ausführungsform erfolgt die Polymerisationsreaktion bei einem Über- oder bei einem Unterdruck. Bevorzugt ist die Beaufschlagung mit einem Druck zwischen 1,1bar und 10bar. Ferner bevorzugt ist die Anlage eines Unterdruckes zwischen 10 und 900mbar. Ferner bevorzugt ist die Anwendung eines Druckverlaufs. So kann in einer Ausführungsform zunächst ein erhöhter Druck beaufschlagt werden und im Verlauf wird dieser kontinuierlich oder stufenweise erniedrigt. In einer besonders bevorzugten Ausführungsform erfolgt die lösungsmittelinduzierte Polymerisationsinitiierung in einem Behältnis mit einem steuerbaren Ein-/Auslassventil. In einer weiterhin besonders bevorzugten Ausführungsform erfolgt die Temperierung der mit der Reaktionslösung gefüllten Membran nach einem 2 oder mehrstufigen oder kontinuierlichen Verlaufsschema mit unterschiedlichen Temperaturen. Bevorzugt ist eine Dauer einer Temperaturerhöhung zwischen 10 Minuten und 72 Stunden, mehr bevorzugt zwischen 30 Minuten und 48 Stunden und weiter bevorzugt zwischen 2 und 24 Stunden.

In einer weiter bevorzugten Verfahrensart erfolgt die Reaktionsinitiierung der Mono-/Polymerlösung in einem raumgebenden Stützgewebe durch eine Polykondensation in Form einer Schmelze. Vorzugsweise werden hierbei Temperaturen gewählt, die um oder oberhalb des individuellen Schmelzpunktes der eingesetzten Monomere liegen. Bevorzugt ist eine Erwärmung auf 40 bis 200°C, mehr bevorzugt auf 80° bis 140°C und weiter bevorzugt auf 90° bis 140°C. Ein weiteres bevorzugtes Verfahren zur Polymerisationsinitiierung ist eine Exposition der mit einer Mono-/Oligomerlösung getränkten offenporigen Membran mit einer lang- oder kurzwelligen Strahlung. Besonders bevorzugt ist die Applikation von Mikrowellen. In einer bevorzugten Ausführungsart des Verfahrens werden die reaktiven Mono-/Oligomere in den inneren Raumstrukturen der porösen Trägermembran vor einer Reaktionsaktivierung aufkonzentriert. In einer Ausführungsart kann dies erfolgen, indem die Lösung mit den reaktionsfähigen Mono-/Oligomeren in die Trägermembran eingebracht wird und anschließend das Lösungsmittel durch geeignete Maßnahmen evaporiert wird, vorzugsweise ohne eine Reaktionsaktivierung zu bedingen. Dies kann z.B. dadurch erreicht werden, indem die Evaporation bei erniedrigter Temperatur und einem angelegten Vakuum erfolgt. Dieser Prozess kann in der erforderlichen Anzahl wiederholt werden. Bevorzugt ist es, im Anschluss hieran eine Schmelze oder Bestrahlung der akkumulierten reaktiven Mono-/Oligomere vorzunehmen. Besonders bevorzugt ist eine ringöffnende Polymerisation, die unter Erwärmung des Substrates erfolgt.

In einer weiteren bevorzugten Ausführungsform der vorliegenden Erfindung umfasst das Verfahren zur Herstellung von offenporigen Membranen zur diffusiven und/oder elektrophoretischen Abtrennung von Carbonsäuren aus flüssigen Stoffgemischen die folgenden Schritte:

g) Bereitstellung einer offenporigen Trägermembran,
h) Einbringen einer Mono-und/oder Oligomerlösung in die offenporige Trägermembran aus Schritt a),
i) Reaktionsaktivierung zur Ausbildung eines offenporigen raumfüllenden nanocavitären polymeren Strukturnetzwerkes,

wobei in Schritt c) die Ausbildung eines offenporigen raumfüllenden nanocavitären polymeren Strukturnetzwerkes durch eine ringöffnende Polymerisation erfolgt, wobei die Mono- und/oder Oligomerlösung erwärmt wird und/oder die Mono- und/oder Oligomerlösung Lösungsmittel enthält, die eine ringöffnende oder radikalische Polymerisation initiieren können. Bevorzugt ist ein Verfahren zur ringöffnenden Polymerisation, bei dem das Substrat mit der Mono-/Oligomerlösung erwärmt wird.

Bevorzugt ist ein Verfahren bei dem raumfüllende nanoskalierte Polymerstrukturen durch eine Polykondensation, die

durch thermische Schmelze einer Mono-/Oligomerlösung erfolgt, erhalten werden.

In einer Ausführungsform erfolgt die Initiierung der Polymerisation durch eine Startreaktion, die von der Oberfläche der Trägermaterialien gestartet wird. Bevorzugt sind Oberflächen, die eine Funktionalisierung mit einem Starter für eine radikalische oder ringöffnende Polymerisation aufweisen. Bevorzugt sind ferner Polymerisationen, die mittels eines "Grafting through"-Verfahren erfolgen. Beim Grafting through-Verfahren werden 2 oder mehr Polymerverbindungen miteinander verbunden. Dabei bildet eine der Verbindungen ein längerkettiges Grundgerüst aus und die anderen Verbindungen werden im Rahmen der Reaktion mit diesem Grundgerüst verbunden. In einer weiteren Ausführungsform erfolgt eine Polymerisation in Form eines "grafting to" - Verfahrens. Dabei werden zunächst Polymere in geeigneten Reaktionsansätzen erzeugt. Vorzugsweise werden die entstandenen Polymere anschließend gereinigt und in einer spezifizierten Form bzw. mit einer definierten Mol-Masse durch geeignete Separationstechniken erhalten. Diese Polymere werden dann mit Oberflächen oder anderen Polymeren in Kontakt gebracht und mit diesen mittels einer Kondensations-, Additions- oder Substitutionsreaktion verbunden.

Polymerisationsverfahren, mit denen die erfindungsgemäßen nanocavitären Polymerstrukturen hergestellt werden können, umfassen Verfahren aus dem Stand der Technik, wie der Atom Transfer Radical Polymerisation (ATRP), der "ringopening metathesis polymerization" (ROMP), der anionischen oder kationischen Polymerisation, sowie der freien lebenden radikalischen Polymerisation, aber auch der strahlungs-induzierten Polymerisation, der ringöffnenden Olefinmetathese Polymerisation, der reversiblen Additions-Fragmentierungs-Kettenübertragungs-polymerisation, der Nitroxidvermittelten Polymerisation, Polykondensationsreaktionen sowie einer iniferter-induzierten Polymerisation.

Die Reaktionsbedingungen, die bei den unterschiedlichen Verfahren zur Herstellung von nanocavitären raumfüllenden Polymerstrukturen erforderlich sind, können sehr unterschiedlich sein. Bevorzugt ist eine Reaktionszeit zwischen 1 Minute und 10 Tagen, mehr bevorzugt zwischen 10 Minuten und 3 Tagen und weiter bevorzugt zwischen 15 Minuten und 24 Stunden. Bevorzugt ist eine Reaktionstemperatur zwischen 0° und 270°C, weiter bevorzugt zwischen 10° und 180°C und weiter bevorzugt zwischen 20° und 130°C.

Bevorzugt ist ein Verfahren, bei dem in Schritt b) eine Lösung mit reaktiven Mono-Oligomeren in eine raumgebende offenporige Trägermembran eingebracht wird und durch eine Polymerisationsinitiierung in Stufe c) es zu einer Ausbildung von Polymerstrukturen kommt, die nanocavitäre Spalträume ausbilden.

Bevorzugt ist ein Verfahren, bei dem die Stufe c) durch eine Polymerisationsinitiierung chemisch, physiko-chemisch oder physikalisch erfolgt. Bevorzugt ist ein Verfahren, bei dem die Polymerisationsinitiierung in Stufe c) durch eine Temperaturerhöhung erfolgt.

In einer Verfahrensausführung können die mono- und/oder oligomeren Verbindungen, die zur Herstellung der nanocavitären Polymerstrukturen eingesetzt werden, funktionelle Seitengruppen aufweisen. Die funktionellen Seitengruppen können polar oder apolar sein und/oder reaktive und/oder ionische Verbindungen enthalten. Beispiele für apolare Gruppen sind aliphatische oder cyclische Kohlenwasserstoffe. Beispiele für polare Seitengruppen sind Alkohole oder Carbonsäuren. Beispiele für reaktive Seitengruppen sind Amine, Thiole, Olefine, Alkine, Aldehyde. Vorzugsweise werden diese funktionellen Seitengruppen durch Schutzgruppen, die im Stand der Technik bekannt sind, während der Polymerisationsreaktion geschützt. Vorzugsweise werden die Schutzgruppen nach der Polymerisationsreaktion abgespalten und durch geeignete Maßnahmen aus der Membran entfernt.

Im Anschluss an die Polymerisation werden die Membranen von hierin noch vorhandenen Resten von Monomeren und/oder Katalysatoren und/oder Nebenprodukten befreit, indem die Membranen mit einer geeigneten Sequenz an Lösungsmitteln mit diesen gespült werden.

Bevorzugt ist ein Verfahren, bei dem in der Stufe d) vorhandene Reste von Monomeren und/oder Katalysatoren und/oder Nebenprodukte aus der Membran entfernt werden.

In einer ganz besonders bevorzugten Ausführungsform handelt es sich bei den erfindungsgemäßen Polymeren, die nanocavitäre Polymernetzwerke ausbilden, um Biopolymere. Diese umfassen physiologische Verbindungen, wie beispielsweise Aminosäuren, Carbonsäuren, Polysacharide, Nukleinsäuren, Polyphenole, Phenylpropanderivate, Sacharide, sowie deren Derivate. Besonders bevorzugt sind Phenylalanin, Lysin, Arginin und Benzylglutamat. Bevorzugt sind Stearinsäure, Ölsäure, Linolsäure und Linolensäure.

Bevorzugt ist ein Verfahren zur Herstellung von nanocavitären Polymerstrukturen mittels Biopolymeren.

Bevorzugt ist ein Verfahren zur Herstellung von nanocavitären Polymerstrukturen aus Polyphenylalanin.

Eine bevorzugte Ausführungsform der vorliegenden Erfindung betrifft Verfahren zur Oberflächenfunktionalisierung der nanocavitären Polymerstrukturen mit Verbindungen, die bifunktional lipophil und kationisch sind. Dies bedeutet, dass die Beschichtung aus amphiphilen Molekülen besteht, welche eine positiv geladene Gruppe aufweisen und einen lipophilen Anteil besitzen, wie beispielsweise einen längeren Alkylrest. Geeignete Moleküle für die Oberflächenfunktionalisierung sind somit insbesondere Tetraalkylammoniumverbindungen oder alkylierte Imidazolverbindungen mit mindestens einem Alkylrest oder einer längeren Kohlenstoffkette, welche auch Aromaten oder Doppelbindungen oder Heteroatome enthalten kann, und dem positiv geladenen Stickstoffatom, so dass lipophile und kationische Eigenschaften in einem Molekül vorliegen. Bevorzugte Verbindungen sind, Tetradecyltrimethylammoniumoxalat, Denatoniumbenzoat oder der Tetramethyl-2-methylen-1 H-imidazol. Eine Alternative ist, zwei verschiedene Moleküle zu verwenden, wobei

eines die kationischen Eigenschaften und das andere die lipophilen Eigenschaften mit sich bringt, wie z.B. Ammoniumpolyole oder eine Trimethylpropylammoniumgruppe, welche kationische Eigenschaften aufweist und ein Dodecylrest, welcher die lipophilen Eigenschaften besitzt.
Bevorzugt ist ein Verfahren, bei dem in der Stufe d2) die Oberflächenfunktionalisierung der Polymerstrukturen, erhältlich aus der Stufe c), mit amphiphilen Verbindungen erfolgt.

In einer bevorzugten Ausführungsform der vorliegenden Erfindung umfasst das Verfahren zur Herstellung von offenporigen Membranen zur diffusiven und/oder elektrophoretischen Abtrennung von Carbonsäuren aus flüssigen Stoffgemischen, die folgenden Schritte:

a) Bereitstellen einer raumgebenden offenporigen Trägermembran,
b) Einbringen einer Mono-und/oder Oligomerlösung in die offenporige Trägermembran aus Schritt a),
c) Reaktionsaktivierung zur Ausbildung eines offenporigen raumfüllenden nanocavitären polymeren geschützten Strukturnetzwerkes, und
d2) Funktionalisieren der Oberfläche der raumfüllenden Polymerstrukturen aus Schritt c), mit mindestens einer amphiphilen Verbindung.

Zur Herstellung der erfindungsgemäßen offenporigen nanocavitären raumfüllenden Polymerstrukturen können prinzipiell alle aus dem Stand der Technik bekannten organischen Verbindungseinheiten, mit denen sich organische Polymere herstellen lassen, verwandt werden. Beispiele solcher organischer Verbindungseinheiten sind Ethylen, Propylen, Vinylchlorid, Caprolactam, Isopren, 1,3-Butadien, Carbonate, Arylate, Caprolacton, Tetrafluorethylen, Oxazoline, Imidazole, Carboxylsäureester oder -ether, 1-thio-2,3-dihydroxypropyl-thioether, 1-diglycerol ether, 1,2 dihydroxy 3-thiopropyl-1-ether, 1-Glycerolether, 1-Glycerolether mit einem Substituenten an Position 3, wie z. B. 1,3 Glycerol.
Ferner Imide, Aramide und aromatische Amide (z. B. m- oder p-Phenylenisophthalamid), Paraphenylene, Terephthalamide, Paraphenylene/3,4'-Diphenylether, Terephthalamid sowie Metaphenylen-isophthalamid, Aminalkohol, Isocyanate, Cyclocarbonat, Triazine, Melamin, Vinylalkohol, Vinylacetat, Vinylbutyral, Acrylat mit freien Hydroxylgruppen, Polyester, vorzugsweise PET, PBT, PTT, p-Hydroxybenzoesäure, 2,6-Hydroxynaphthoesäure, 2,5-Hydroxynahpthoesäure, 2,6-Dihydroxynaphthalen, 2,6-Naphthalendicarboxylsäure, Biphenol, Bisphenol A, Terephthalsäure, Isophthalsäure und Hydrochinone.
Weiterhin aromatische Polyamide, wie Vinylpyrrolidon oder Mischester dieser, Urethan, Harnstoff oder biologisch erzeugte bzw. abbaubaren Mono- bzw. Polymere, vorzugsweise aus Milchsäure, Hydroxyalkanoat, Hydroxybutyrat, Hydroxyvalerat, Cellulose oder deren Gemische oder Copolymere hiervon.
Für die Herstellung der erfindungsgemäßen nanocavitären Polymerstrukturen können verschiedene Polymerisationsmechanismen ausgewählt werden. Hierzu sind Monomere, die radikalisch, kationisch oder anionisch reagierende Zentren enthalten, herzustellen. Techniken zur Herstellung reaktiver Mono-/Oligomere sind im Stand der Technik bekannt. Als reaktive Zentren dienen insbesondere Halogene und Halogenide, wie Brom oder Chlorid, zyklische Olefine, wie Norbornene und Cylopentene, negativ geladene Vinylgruppen, Isocyanate, Amine, Aldehyde, Ketone, Carbonsäuren, Epoxide, elektronenreiche Aromaten, wie Phenole, HydroxyVerbindungen, wie Phenole oder Alkohole, Michael-Systeme, wie $\alpha,\beta$-ungesättigte Ester, Amide, Nitrile, Nitroolefine, Kohlensäuren und -Derivate.
Besonders bevorzugt ist die Verwendung von Monomeren, die physiologisch vorkommen und/oder biologisch abbaubar sind. Hierfür sind insbesondere geeignet Aminosäuren und Derivate hiervon. Dabei kann es sich um Carbaminsäure, alpha-, beta- oder gamma-Aminosäuren handeln sowie um L- oder D-Formen sowie Mischungen dieser. Insbesondere handelt es sich dabei um Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin, Valin, sowie Thyroxin, Dopamin, 5-Hydroxytrytophan, Histamin. Ferner Aminosäurederivate, wie z. B. Tyrosin, Zimtsäure oder 3-(4-Hydroxyphenyl)propionsäure. Besonders bevorzugt sind Phenylalanin, Arginin und Lysin. Sofern Monomere reaktive Zentren/Seitengruppen aufweisen, die eine nicht gewünschte Reaktion während der Polymerisation verursachen können, so sind diese reaktiven Gruppen mit Schutzgruppen zu passivieren. Techniken hierzu sind aus dem Stand der Technik bekannt.
In einer weiteren bevorzugten Verfahrensdurchführung werden geschützte Aminosäuren verwendet, d.h. reaktionsfähige Haupt oder Seitengruppen werden durch eine reversible Sättigung mit einer nicht reaktionsfähigen Verbindung von einem Reaktionsumsatz geschützt. Als Schutzgruppe für die zu schützende Funktionalität werden bevorzugt Fluorenylmethoxycarbonyl- (Fmoc), tert-Butyloxycarbonyl- (Boc), Benzyloxycarbonyl- (Cbz, Z) oder Acetylgruppen verwendet, weiter bevorzugt werden Triphenylmethyl- (Trt), Benzyloxymethyl- (Bom), Benzyl-, Phenyl-, Dinitrophenyl- (Dnp), Toluolsulfony-l (Tos), Mesitylensulfonyl- (Mts), Acetamidomethyl- (Acm), tert-Butylmercaptogruppen (tBum) eingesetzt. Im Stand der Technik sind Verfahren bekannt, mit denen sich die Schutzgruppen im Anschluss an eine erfolgte Polymerisation wieder entfernen lassen.
Besonders bevorzugt sind Monomere von alpha-Aminosäure N-Carboxyanhydriden. Besonders bevorzugt ist Phenylalanin-NCA als reaktives Monomer. Weiter bevorzugt sind Di-, Tri- oder Polypeptide mit mindestens einer reaktiven

Gruppe, die eine Polymerisation ermöglicht.-
Als "reaktive Monomere" werden hierin auch verstanden Di-, Tri- oder Oligomere sowie Co- oder Blockpolymere, die eine oder mehrere reaktive Gruppe(n) aufweisen, die eine Polymerisation mit anderen reaktiven Monomeren ermöglicht/ermöglichen. Bevorzugt ist ferner die Verwendung von Copolymeren, wie Styrol.
Die erfindungsgemäßen Polymerisationsreaktionen lassen sich mit reaktiven Monomeren oder alleine oder zusammen aus/mit reaktiven Oligomeren herstellen. Unter Oligomer wird hierin verstanden, ein Molekül, das aus 2, 3 oder mehr gleichen oder ähnlichen Moleküleinheiten, die miteinander kovalent gebunden sind, besteht. Die erfindungsgemäßen Oligomere haben vorzugsweise bis 500 Untereinheiten, mehr bevorzugt bis 250 und weiter bevorzugt bis 100 Untereinheiten. Die Moleküluntereinheiten der erfindungsgemäßen Oligomere bestehen vorzugsweise aus den gleichen Verbindungen, wie die hierin beschriebenen reaktiven Monomere. Besonders bevorzugt sind Polypeptide, die aus 2, 3 oder mehr gleichen oder unterschiedlichen Aminosäuren bestehen, die linear in Form einer Kette verbunden sind oder in einer zyklischen Formation vorliegen und ebenfalls unter den hierin gebrauten Begriff Oligomer fallen. Die reaktiven Oligomere haben dabei 1 oder mehrere reaktive Zentren, die eine nukleophile oder radikalische Reaktion ermöglichen, vorzugsweise bestehen diese aus einer oder mehreren der hierin aufgeführten Verbindungen. Insofern können die reaktiven Oligomere mit den gleichen Verfahren, wie die reaktiven Monomere, polymerisiert werden.
In einer besonders bevorzugten Ausführungsart erfolgt die Polymerisation von Mono-/Oligomeren in einem raumgebenden Stützgewebe. Bei dem Stützgewebe handelt es sich vorzugsweise um eine offenporige Trägermembran. "Raumgebend" bedeutet in diesem Zusammenhang, dass die offenporige Trägermembran porös und offenporig ist und dass die Poren, Kanäle oder Schlitze einen Durchmesser zwischen 10nm bis 300$\mu$m, mehr bevorzugt zwischen 100nm und 10$\mu$m und weiter bevorzugt zwischen 200nm und 5$\mu$m aufweisen. Folglich besitzt die raumgebende offenporige Trägermembran eine große spezifische Oberfläche bzw. große innere Oberfläche, die raumbegrenzend und formgebend ist für die nanocavitären Polymerstrukturen, die den inneren Oberflächen anliegen, bzw. mit denen sie verbunden sind. D. h. die raumgebende offenporige Trägermembran besitzt offene Kavitäten in Form von offenen durchgängigen Poren, Kanälen oder Schlitzen und ist damit nicht nur in der Lage, Stoffe und Moleküle in seine offenen Kavitäten aufzunehmen, sondern auch viskose Lösungen mit einer Konzentration von 1 mmol/L bis 3 mol/L. Die offenporige Trägermembran ist zudem dadurch charakterisiert, dass sie eine hohe mechanische Stabilität besitzt. Die offenen Kavitäten bleiben bei äußeren Druckeinwirkungen stabil in ihrer Form und fallen insbesondere nicht zusammen. Besonders geeignet hierfür sind frei-tragende Membranen, Gewebe oder Texturen sowie poröse Materialien, die in einer Verbundstruktur vorliegen.
Geeignete offenporige Stützgewebe zur Beschichtung können jedwede beständige Membranen, Gewebe oder Materialien sein, mit einer bevorzugten Porosität von > 15%, mehr bevorzugt von > 25%, weiter bevorzugt von > 50%, noch weiter bevorzugt von > 70% und am meisten bevorzugt von > 80%. Als Materialien kommen sowohl organische als auch anorganische Materialien in Frage, sofern diese beständig sind gegen die zur Lösung von Monomeren eingesetzten Lösungsmittel sowie gegen wässrige Medien, mit denen sie bei einer Anwendung in Kontakt gebracht werden. Geeignete organische Materialien umfassen natürliche sowie synthetische Polymere, insbesondere Cellulose sowie Cellulosederivate, z.B. Celluloseacetate, Melamine, Polyethylen, Polypropylen, PMMA, Polycarbonat, Polyurethan, Nafion, Polyethylen, Terephthalate (PET) oder Polysulfone.
Polymere Materialien können in Form von Fasern, Geweben oder Schäumen vorliegen und in beliebiger Anordnung ein Strukturnetzwerk ausbilden. Besonders bevorzugt sind Hohlkammerfasern. Ferner sind Polymer-Membranen geeignet, die zunächst als geschlossene Folie hergestellt und anschließend mechanisch oder chemisch perforiert wurden. Insofern sind auch "Kernspurmembranen" geeignet, bei denen nach einem Elektronenbeschuss Kanäle, die durch die Membran gehen, hergestellt werden, indem eine chemische Ätzung entlang des durch den Elektronenbeschuss aufgelösten Polymerverbandes erfolgt. Weiterhin geeignet sind Folien aus Polymeren, die durch ein mehrdimensionales Verziehen porös werden. Besonders geeignet hierfür ist PTFE. Außerdem geeignet sind Folien aus BlockCopolymeren, bei denen nach der Herstellung Polymeranteile durch physikalische oder chemische Verfahren aufgelöst werden, wodurch die Folien porös werden. Vorteilhaft bei derartigen Membranen ist, dass die entstehenden Raumstrukturen skalierbar sind und die auch chemische Bindungsgruppen bereits enthalten können, sodass eine Oberflächenfunktionalisierung für die erfindungsgemäße Herstellung von raumfüllenden Polymeren nicht mehr erforderlich ist.
In einer Ausführungsart erfolgt die Herstellung der nanoskalierten Polymerstrukturen, indem Folien oder Streifen mit der Monomerlösung beaufschlagt und in mehrere Lagen übereinander gebracht werden. Die Folien dienen dabei als Trägergerüst, der Verbund kann nach der Polymerisation in Scheiben geschnitten werden. In diesem Fall erfolgt der Transport von Carbonsäuren nicht durch die Trägerfolie, sondern entlang dieser durch die raumfüllenden Polymerstrukturen. Mit einem solchen Verbund von gestapelten Folien, die nanocavitäre Polymerschichten umschließen, lassen sich verschiedene Geometrien eines Trennmediums herstellten. So können Ringe oder Röhren durch Ausschneiden hergestellt werden. Andererseits lassen sich Streifen herstellen, die übereinandergelegt werden und zu einem festen Verbund zusammengefügt werden. In einer anderen Ausführungsform kann es dabei vorteilhaft sein, ein Trägermaterial ein- oder beidseitig mit der Monomerlösung zu beaufschlagen und nach Herstellung einer Polymerschicht mit nanoskalierten Polymerstrukturen mit gleichartig hergestellten Trägern in Schichten zusammenzufügen oder sie zu schneiden und zu räumlichen Gebilden, wie z. B. einer Scheibe, zusammenzuführen. In einer besonders bevorzugten Ausführungsform

besteht das Trägermaterial aus Carbonfasern. Diese haben den Vorteil einer hohen chemischen und thermischen Beständigkeit sowie einer sehr guten mechanischen Belastbarkeit. Carbonfasern können in dünnen Streifen oder Fasern hergestellt werden und lassen sich z. B. zu Geweben verarbeiten.

Geeignete anorganische Materialien umfassen vorzugsweise keramische oder metallische Materialien, wie beispielsweise Aluminiumoxid, Aluminium, Titanoxid, Titan, Tantal, Zirkonium, Zirkonoxid, Zeolithe oder Glas. Geeignete keramische Membranen können dabei hergestellt sein aus einer Pressung oder Sinterung von Partikeln, die anschließend thermisch behandelt werden, wodurch ein stabiler Verbund entsteht. Dabei kann es erforderlich sein, organische oder anorganische Zuschlagstoffe zu verwenden, die nach oder bei der Herstellung der Membran physikalisch und/oder chemisch ganz oder teilweise wieder ausgetragen bzw. abgebaut werden. Keramische Membranen können auch durch eine Schmelze von Partikel erhalten werden. Dieses Verfahren eignet sich besonders für Silizium-Verbindungen. Andererseits lassen sich auch Fasertexturen aus anorganischen Materialien herstellen. Diese lassen sich zu Gewebeverbänden zusammenfügen, z. B. als Glasfasergewebe.

Die Spalträume geeigneter poröser Materialien können eine beliebige Konfiguration aufweisen, solange sie in der Pluralität miteinander verbunden sind und die Passage eines flüssigen Mediums durch das Material erlauben. Die Spaltmaße betragen vorzugsweise für minimale Durchmesser zwischen 10nm bis 100$\mu$m, mehr bevorzugt zwischen 100nm und 10$\mu$m und weiter bevorzugt zwischen 200nm und 1$\mu$m. Bevorzugt sind maximale Durchmesser der Spaltmaße zwischen 1$\mu$m und 3mm, mehr bevorzugt werden Durchmesser zwischen 10$\mu$m und 1mm und weiter bevorzugt zwischen 10$\mu$m und 100$\mu$m. Die Länge der Verbindungen zwischen beiden Außenseiten beträgt vorzugsweise zwischen 5$\mu$m und 10mm, mehr bevorzugt zwischen 50$\mu$m und 3mm und weiter bevorzugt zwischen 100$\mu$m und 1mm. Die Struktur, bzw. der Strukturaufbau des raumgebenden Stützgewebes, resp. der offenporigen Trägermembran, ist beliebig. Dabei kann es sich um einen schichtweisen Aufbau aus Fasern oder Geweben handeln oder aus einem gesinterten Material bestehen oder aus einem gegossenen oder verpressten kontinuierlich oder diskontinuierlich zusammenhängenden Material.

Die Dicke des Trägermaterials, resp. der offenporigen Trägermembran, beträgt vorzugsweise zwischen 5$\mu$m und 10mm, mehr bevorzugt zwischen 50$\mu$m und 3mm und weiter bevorzugt zwischen 100$\mu$m und 1mm. Die äußere Form der raumgebenden Stützstruktur kann eine beliebige Geometrie aufweisen, bevorzugt sind eine planebene Bauform sowie eine Röhrenform. In einer besonders bevorzugten Ausführungsform ist die raumgebende offenporige Trägermembran eine Hohlfaserkapillare.

Bevorzugt ist ein Verfahren, bei dem die raumgebende offenporige Trägermembran der Stufe a) aus einer/einem porösen frei-tragenden Membran, Gewebe oder Texturen sowie poröse Materialien besteht, die in einer Verbundstruktur vorliegen. Bevorzugt ist ein Verfahren, bei dem die raumgebende offenporige Trägermembran der Stufe a) aus anorganischen und/oder organischen Verbindungen besteht. Bevorzugt ist ein Verfahren, bei dem die raumgebende offenporige Trägermembran der Stufe a) die Form von planaren Membranen, Röhren oder Hohlkammerkapillaren aufweist.

In einer bevorzugten Ausführungsform der vorliegenden Erfindung umfasst das Verfahren zur Herstellung von offenporigen Membranen zur diffusiven und/oder elektrophoretischen Abtrennung von Carbonsäuren aus flüssigen Stoffgemischen die folgenden Schritte:

a) Bereitstellen einer offenporigen Trägermembran,
b) Einbringen einer Mono- und/oder Oligomerlösung in die offenporige Trägermembran aus Schritt a),
c) Reaktionsaktivierung zur Ausbildung eines offenporigen raumfüllenden nanocavitären polymeren Strukturnetzwerkes,

wobei die offenporige Trägermembran aus einer porösen frei-tragenden Membran, einem Gewebe oder Texturen sowie porösen Materialien besteht, die in einem Verbund vorliegen.

Um eine äußerst vorteilhafte Stofftrennung durch die erfindungsgemäße Membranherstellung zu erhalten, ist es bevorzugt, dass die nanocavitären Polymerstrukturen raumfüllend sind sowie auch ein vollflächiger Verbund mit den inneren Oberflächen des Raumstrukturgewebes erreicht wird. Daher ist es vorteilhaft, eine vollflächige Belegung/Anbindung der inneren raumgebenden Grenzflächen der offenporigen Trägermembran mit den erfindungsgemäßen Polymerstrukturen zu erreichen. Sofern an der raumgebenden Stützstruktur nicht bereits reaktionsbildende Gruppen vorhanden sind, können diese mit Techniken aus dem Stand der Technik auf die inneren Oberflächen des Stützmaterials aufgebracht werden. Geeignete Verbindungen, die an der Oberfläche zur Reaktionsbildung in hoher Dichte zur Verfügung stehen sollten, weisen funktionelle Gruppen auf, wie z. B. Amine, Epoxide, Thiole, Alkylhalogenide oder Carboxylgruppen. Ferner können auch andere reaktionsfähige Gruppen verwandt werden, wie Cyanate, Thiocyanate, Alkene, Azide oder aktivierte Carbonsäuren.

Zur Beschichtung sind die Oberflächen der offenporigen Trägermembran mit geeigneten Maßnahmen aus dem Stand der Technik vorzubereiten. Die Anbindung kann chemosorptiv oder physiosorptiv erfolgen. Bevorzugte Verbindungen, mit denen eine vollflächige Belegung der Oberflächen der offenporigen Trägermembran mittels einer kovalenten Anbindung erfolgen kann, sind beispielsweise Aminosilane, wie (3-Aminopropyl)triethoxysilan (APTS) oder und (3-Trimethoxysilylpropyl)diethylentriamin (TAPTES). Beispiele für Verbindungen, mit denen eine physiosorptive Belegung der Oberflächen möglich ist, sind beispielsweise Dopamin, Polyethylenimin, Polyvinylamin, Polyvinylimidazol, Polyvinylpyridin, Polyvinylpyrrolidon, Polylysin oder Polyacrylsäure.
In einer besonders vorteilhaften Ausführungsform des Verfahrens erfolgt in der optionalen Stufe a1) eine Vorbehandlung der Oberflächen der raumgebenden offenporigen Trägermembran mit einer vollflächigen Beschichtung mit Verbindungen, die eine Reaktion mit der Monomerlösung in der Stufe b) ermöglicht oder bedingt. Bevorzugt ist ein Verfahren, bei dem die raumgebende offenporige Trägermembran der Stufe a) in der Stufe a1) einer Oberflächenbehandlung unterzogen wird. Bevorzugt ist ein Verfahren, bei dem die Oberflächenbehandlung in der Stufe a1) mit einem Starter für eine radikalische oder nukleophile Polymerisation erfolgt.

Nach der Ausbildung der nanocavitären Polymerstruktur können Rückstände von Lösungsmittel und/oder Nebenprodukte der Reaktion in der erfindungsgemäß hergestellten offenporigen Membran verbleiben. Diese Rückstände können in einem separaten Reinigungsschritt, z.B. durch Spülen oder Waschen, aus der Membran entfernt werden. Dabei kann es erforderlich sein, die Membranen mit verschiedenen Lösungsmitteln und in subsequenter Abfolge zu reinigen, bevorzugt sind Methanol, $H_2O_2$ oder THF. Ferner kann es erforderlich sein, Reste der Lösungsmittel zu entfernen, wozu z.B. das Einlegen in einen Vakuum-Trocknungsschrank oder eine Erhitzung geeignete Verfahren sind.

Somit umfasst in einer bevorzugten Ausführungsform der vorliegenden Erfindung das Verfahren zur Herstellung von offenporigen Membranen zur diffusiven und/oder elektrophoretischen Abtrennung von Carbonsäuren aus flüssigen Stoffgemischen die folgenden Schritte:

a) Bereitstellen einer raumgebenden offenporigen Trägermembran,
b) Einbringen einer Mono-und/oder Oligomerlösung in die offenporige Trägermembran aus Schritt a),
c) Reaktionsaktivierung zur Ausbildung eines offenporigen raumfüllenden nanocavitären polymeren Strukturnetzwerkes,
d) Reinigen der Membran aus Stufe c) von Lösungsmitteln und/oder Reaktionsnebenprodukten.

Die erfindungsgemäßen Membranen zeichnen sich insbesondere durch verschiedene physikalische Eigenschaften aus. Hierzu zählt insbesondere, dass sie für Gase und Carbonsäuren permeabel sind.
Die erfindungsgemäßen Membranen sind ferner charakterisiert durch messbare Oberflächeneigenschaften, wie beispielsweise einer Lipophilie, respektive einer Hydrophobie, bzw. einer Ladungsträgerdichte, die durch die Bestimmung der Kontaktwinkel für Wasser oder eine Carbonsäure angegeben werden. Die Messmethode zielt auf die Benetzbarkeit von Oberflächen ab, wobei der Winkel zwischen einem Flüssigkeitstropfen auf einer Oberfläche und der festen Oberfläche gemessen wird.

Die erfindungsgemäßen Membranen wiesen sowohl an den Außenflächen sowie an den Oberflächen von Bruchkanten Wasserkontaktwinkel von vorzugsweise > 70°, mehr bevorzugt von > 100° und weiter bevorzugt von > 120° auf. Die erfindungsgemäßen Membranen wiesen ferner bevorzugt an äußeren und inneren Oberflächen Kontaktwinkel für Ölsäure auf, die vorzugsweise < 30°, mehr bevorzugt < 20° und weiter bevorzugt <10° sind.
Eine weitere bevorzugte Ausführungsform der vorliegenden Erfindung betrifft ein Verfahren, wobei die funktionalisierten oder beschichteten Oberflächen der Poren, Kanäle oder Schlitze einen Kontaktwinkel für Wasser von > 70°, bevorzugt > 100°, insbesondere bevorzugt > 120° und einen Kontaktwinkel für die abzutrennende Carbonsäure von < 30° aufweisen.
In einer Ausführungsart zeichnen sich die erfindungsgemäßen Membranen dadurch aus, dass sie Carbonsäuren gegen einen hydrostatischen Gradienten aufnehmen. Dieser Gradient ist vorzugsweise > 10mm $H_2O$, weiter bevorzugt > 30mm $H_2O$ und weiter bevorzugt > 60mm $H_2O$. Dies kann bestimmt werden, indem an ein geschlossenes und mit einer Carbonsäure vollständig gefülltes Behältnis mit einer hierin liegender Membran, ein entsprechender Unterdruck angelegt wird.
In einer besonders bevorzugten Ausführungsform sind die erfindungsgemäßen Membranen resistent gegenüber den meisten organischen Lösungsmitteln. Bevorzugt ist eine Resistenz gegen Toluol, Ethanol, Methanol, Xylen, Acetonitril, THF, Dimethylformamid, Aceton, Methylester, Ethylester, Propylencarbonat, NMP, DMSO, Dichlormethan, Chloroform, Dichlorethan, Perchlorethylen, Trichlorethylen, Tetrachlorkohlenstoff, Chlorbenzol, Benzylalkohol, Glycole, Glycolether, Isopropylacetat, Butanon, Methylisobutylketon, Methoxypropylacetat, Butylacetat, Tetrahydrofuran, Diethylether, Diisopropylether, MTBE, Butanol, Isopropanol, Trifluorethanol, Hexafluorisopropanol. Resistent bedeutet dabei, dass sie sich in den Lösungsmitteln nicht zersetzen.
Bevorzugt ist ein Verfahren zur Herstellung von Membranen mit nanocavitären Polymerstrukturen, die resistent sind gegenüber organischen Lösungsmitteln.

In einer weiteren besonders bevorzugten Ausführungsform weist die erfindungsgemäße Membran eine hohe Biokompatibilität auf. Eine hohe Kompatibilität wird insbesondere dadurch gewährleitet, indem eine geringe Aktivierung des Gerinnungs- und Komplementsystems bei einem Kontakt mit der erfindungsgemäßen Membran auftritt. Ein weiterer Aspekt einer hohen Biokompatibilität ist begründet durch eine geringe Adsorption von Proteinen und lebenden Zellen. Bevorzugt ist ein Verfahren zur Herstellung von Membranen mit nanocavitären Polymerstrukturen, die biokompatibel sind.

Verfahren zur kontinuierlichen Separation von deprotonierten Carbonsäuren, die in wässrigen Medien vorliegen, sind nicht bekannt. Mit den erfindungsgemäßen Membranen ist eine derartige kontinuierliche Abtrennung von Carbonsäuren möglich. Dabei kann eine Selektivität für die Separation von in wässrigen Emulsionen gelösten Carbonsäuren gegenüber gleichartigen organischen Verbindungen, die keine Carboxylgruppe aufweisen, erreicht werden.
So konnte z. B. gezeigt werden, dass eine Selektivität für Fettsäuren gegenüber Fettalkoholen oder Fettsulfaten, die eine gleiche Anzahl von Kohlenstoffatomen aufweisen, besteht, mit einem Selektivitätsindex $\alpha_{OH}$ bezogen auf Fettalkohole von 400 - 800 und einem Selektivitätsindex $a_I$ bezogen auf Fettsulfate von 160, bei Verwendung eines wässrigen Mediums (Beispiel 3).
Mit den erfindungsgemäßen Membranen wird auch eine Selektivität gegenüber anderen organischen Verbindungen, die negative Ladungsgruppen aufweisen gefunden. Proteine weisen unter physiologischen Bedingungen zumeist eine negative Oberflächenladung auf und können elektrophoretisch transportiert werden. Bei unbeschichteten porösen Membranen ist die erzielbare Transportmenge von Carbonsäuren und Proteinen durch die Membran zueinander proportional. Bei Diffusion- und Elektrodialysen, die mit den erfindungsgemäßen Membranen durchgeführt wurden, kam es zu praktisch keinem Transport von Proteinen durch die Membran, während im Vergleich zu unbeschichteten Membranen die Transportrate für Carbonsäuren gesteigert war (Beispiel 3). Es wurden aber auch kleine anionische Verbindungen zurückgehalten, wie Chlorid- und Sulfat-Ionen. Somit gewährleisten die erfindungsgemäßen Membranen eine hohe Selektivität für die Separation von Carbonsäuren aus wässrigen Medien bzw. wässrig gelösten Stoffgemischen. Bevorzugt ist ein Verfahren zur Herstellung von Membranen mit nanocavitären Polymerstrukturen, mit denen ein Stofftransport von anionischen hydrophilen Verbindungen unterbunden wird.

In einer weiteren Ausführungsform der vorliegenden Erfindung hat es sich als besonders vorteilhaft erwiesen, dass eine Solubilisierung von Carbonsäuren vor der Abtrennung der Carbon- und/oder Fettsäuren durchgeführt wird. Effektive Solubilisierungstechniken von Carbon- und/oder Fettsäuren sind in der Literatur beschrieben. Besonders geeignet für deren Lösung in wässrigen Medien sind Laugenbildner, wie NaOH, kationische wasserlösliche Verbindungen, wie Guadinin- oder Amidingruppen-tragende Verbindungen oder quartäre Ammoniumverbindungen.

Beispiele für Lösungen oder Emulsionen, die Carbonsäuren enthalten und getrennt werden können, umfassen industrielle Öle, Pflanzenöle, Milchprodukte, Flüssigkeiten der Phospholipid- und Glycolipidaufreinigung, Kraftstoffaufreinigung, Biodieselherstellung, Biomasse-Auftrennung, Flüssigkeiten aus der biotechnologischen Prozesstechnik, Blut, Blutplasma, Prozessflüssigkeiten, Abwässer, pharmazeutische Synthesegemische und Flüssigkeiten der chemischen Analytik sowie Abwässer.

Da die erfindungsgemäßen Trennmembranen nicht auf dem Prinzip einer Molekülgrößenselektion basiert und die effektiven Kanaldurchmesser zumeist deutlich größer sind, als die Moleküle des abzureichernden Stoffgemisches, ist es bevorzugt, keinen oder nur einen geringen transmembranen Druckgradienten anzulegen. Um dies zu erreichen, kann es erforderlich sein, den Druck an der Donor- und Akzeptorseite zu überwachen und für einen Druckausgleich zu sorgen. Eine bevorzugte Anwendungsform der erfindungsgemäßen Membranen betrifft ein Separationsverfahren, bei dem kein erhöhter Druck auf die flüssige Mischung, enthaltend mindestens eine Carbonsäure, zum Durchtritt der mindestens einen Carbonsäure durch die Membran angelegt wird.
In einer Ausführungsart erfolgt die Anlage eines hydrostatischen Druckes an die Donorkammer. Dies ist besonders vorteilhaft, um einem ggf. vorhandenen EOF entgegen zu wirken.
Der Transport von Carbonsäuren durch die erfindungsgemäßen Membranen erfolgt durch eine Diffusion aufgrund eines Konzentrationsgradienten oder eines thermischen Gradienten und/oder elektro-kinetisch durch einen elektrischen Gradienten. Ein elektrischer Gradient wird vorzugsweise durch die Anlage einer Gleichspannung zwischen der Donor- und der Akzeptorseite hergestellt. Dabei ist bevorzugt eine Spannungsanlage zwischen 1$\mu$V und 500V, mehr bevorzugt zwischen 100mV und 100V und weiter bevorzugt zwischen 1 V und 50 V. Bevorzugt ist eine Stromstärkte zwischen 0,01mA und 5 A, mehr bevorzugt zwischen 0,1mA und 0,5A und weiter bevorzugt zwischen 1 mA und 0,5A.
Ein bevorzugtes Verfahren zur Durchführung der Diffusionsdialyse sind Cross-Flow-Verfahren, unter Verwendung eines geeigneten Akzeptormediums, in das eine Aufnahme der zu separierenden Carbonsäuren erfolgen kann.
Eine besonders bevorzugte Ausführungsform betrifft ein erfindungsgemäßes Verfahren, wobei zum Abtrennen der mindestens einen Carbonsäure ein elektrochemischer Gradient von der Eintrittsseite zur Austrittsseite der Membran angelegt wird.
Die Erfindung betrifft daher auch ein Verfahren zur kontinuierlichen Abtrennung von Carbonsäuren aus flüssigen Mischungen, umfassend die Schritte:

(i) Bereitstellen einer flüssigen Mischung enthaltend mindestens eine Carbonsäure,
(ii) in Kontakt bringen der flüssigen Mischung enthaltend mindestens eine Carbonsäure mit einer offenporigen Membran mit raumfüllenden nanocavitären Polymerstrukturen,
(ii) Abtrennen der mindestens einen Carbonsäure aus der flüssigen Mischung durch selektives Durchtreten der mindestens einen Carbonsäure durch die offenporige Membran mit raumfüllenden nanocavitären Polymerstrukturen, wobei die Abtrennung einen Selektivitätsindex von >4 gegenüber dem Alkohol erhältlich durch Reduktion der entsprechenden Carbonsäure aufweist.

Ein anderer Aspekt der Erfindung betrifft Verfahren, die optional eingesetzt werden können, um der Entstehung eines elektro-osmotischen Flusses, der bei hohen elektrischen Spannungen entstehen kann, entgegenzuwirken. Überraschenderweise wurde gefunden, dass die Anwesenheit von gelösten polykationischen Verbindungen in dem Akzeptormedium, in dem die transportierten Carbonsäuren aufgenommen werden, dies ermöglicht. Dabei kann es zur Ausbildung von viskösen Schichten auf der Membranoberfläche kommen, die Carbonsäuren, die aus dem Donormedium stammen enthalten. Diese Schichten sind für Carbonsäuren permeabel. Bevorzugt sind hochmolekulare polykationische Verbindungen, wie Polyethylenemin, Polyvinylamin, Polyvinylimidazol, Polyvinylpyridin, Polyvinylpyrrolidon, Polylysin, Polyarginin.
Die Konzentration der polykationischen Verbindungen im Akzeptormedium ist dabei bevorzugt zwischen 0,1 μmol/l bis 2mol/l, mehr bevorzugt zwischen 1μmol/l und 0,1 mol/l und weiter bevorzugt zwischen 10μmol/l und 50 mmol/l.
Bevorzugt ist ein Verfahren, bei dem eine Unterbindung des elektro-osmotischen Flusses während einer Elektrodialyse durch die Verwendung von polykationischen Verbindungen im Akzeptormedium erfolgt.

Carbonsäuren sind eine sehr häufig vorkommende Verbindungsklasse, die in vielen industriellen Bereichen aus organischen Substraten extrahiert bzw. gewonnen werden. Die Carboxylgruppen bedingen im Wesentlichen die chemischen aber auch die physiko-chemischen Eigenschaften der Carbonsäuren. Lipophile Kohlenstoffverbindungen erhalten hierdurch amphiphilen Eigenschaften. Hierdurch wird die Löslichkeit in wässrigen oder organischen Gemischen entscheidend gegenüber einer gleichen Molekülstruktur ohne eine Carboxylgruppe verändert. Dies bedingt in der Regel auch eine reduzierte Extrahierbarkeit dieser Moleküle aus einem Stoffgemisch, insbesondere wenn diese in Emulsionen vorliegen. Viele Carbonsäuren sind wichtige Grund- und Baustoffe in verschiedenen Industriebereichen, wozu sie häufig in einem hohen Reinheitsgrad vorliegen müssen. Die Gewinnung erfolgt durch Aufschluss von organischen Bioprodukten und fossilen Materialien oder durch eine Stoffsynthese. Abtrennungstechniken für Carbonsäuren nach dem Stand der Technik sind zumeist aufwendig oder eignen sich nicht für die kontinuierliche Abtrennung großer Stoffmengen. Bei stetig ansteigendem Bedarf an Carbonsäuren ist die Herstellung einer kostengünstigen Verfahrenstechnik, die eine kontinuierliche Abtrennung von Carbonsäuren in großen Mengen ermöglicht, wünschenswert. Dies wird durch die erfindungsgemäßen Membranen in hohem Maße nunmehr ermöglicht. Besonders vorteilhat ist, dass andere organische und anorganische Verbindungen zurückgehalten werden, ohne dass eine Änderung des pH erfolgen oder ein Extraktionsmittel dem Stoffgemisch hinzugegeben werden muss. Besonders vorteilhaft ist ferner, dass durch die Offenporigkeit der Membranen große Stoffmengen pro Flächeneinheit transportiert werden können. Ferner vorteilhaft ist, dass der hierfür erforderliche Energieaufwand im Vergleich zu thermischen Trennverfahren minimal ist. Überaus vorteilhaft ist, dass es zu keinem Fouling der Membran durch organische Verbindungen, die die nanoskalierten Spalten verschließen, kommt, wodurch eine Langlebigkeit der erfindungsgemäßen Membranen resultiert. Die erfindungsgemäßen Membranen sind ferner bio- und hämokompatibel, sodass sie bei biologischen Lösungen oder bei Blut und Blutprodukten eingesetzt werden können. Besonders vorteilhaft ist, dass die erfindungsgemäßen Membranen mit unterschiedlichen Trägermaterialien, die aus dem Stand der Technik erhältlich sind, hergestellt werden können. Somit sind verschiedene Bauformen einfach zu realisieren und die verschiedenen Anforderungen bei unterschiedlichen Anwendungen können gewährleistet werden.

## Definitionen

### Offenporige Trägermembran

Unter dem Begriff "offenporige Trägermembran", wie hierin verwendet, werden raum- und strukturgebende poröse Stützgewebe oder Festkörper verstanden, die durchgehende offene Verbindungen zwischen beiden (oder mehreren) Außenseiten aufweisen. Der Begriff raumgebend bezieht sich hierbei auf die Geometrien der inneren Spalt- bzw. Hohlräume der Trägermembran, die von nanocavitären Polymerstrukturen ausgefüllt werden sollen. Der Begriff strukturgebend bezieht sich auf die äußere Form und Geometrie der offenporigen Trägermembran. Die offenen Verbindungen können plan-parallele bis völlig irregulär verlaufende Wandkonturen bzw. Verlaufsformen der inneren Grenzflächen bzw. Hohlräume aufweisen. Die Verbindungen können beliebige Dimensionen aufweisen, bevorzugt sind minimale Durchmesser der inneren Hohlräume zwischen 10nm bis 100μm, mehr bevorzugt zwischen 100nm und 10μm und weiter bevorzugt zwischen 200nm und 1μm. Vorzugsweise sind die Raumgebilde der offenporigen Trägermembran nicht nanocavitär. Bevorzugt sind maximale Durchmesser der inneren Hohlräume zwischen 1μm und 3mm, mehr bevorzugt werden Durchmesser zwischen 10μm und 1mm und weiter bevorzugt zwischen 10μm und 100μm. Die Dicke der Trägermembran und/oder die Länge der offenen Verbindungen zwischen den Außenseiten beträgt vorzugsweise zwischen 5μm und 10mm, mehr bevorzugt zwischen 50μm und 3mm und weiter bevorzugt zwischen 100μm und 1mm. Die Struktur, bzw. der Strukturaufbau der raumgebenden offenporigen Trägermembran sind beliebig. Dabei kann es sich um einen schichtweisen Aufbau aus Fasern oder Geweben handeln oder sie bestehen aus einem gesinterten oder

gegossenen oder verpressten, kontinuierlich oder diskontinuierlich zusammenhängenden Material. Das Material der raumgebenden offenporigen Trägermembran kann eine beliebige äußere oder innere Oberflächenbeschaffenheit aufweisen (z.B. glatt oder rau) und aus Substanzen bzw. Verbindungen bestehen, die u.a. einschließen: natürliche Polymere, wie Cellulose, synthetische Polymere, wie Polyethylenglycol, ferner anorganische Verbindungen, wie Aluminiumoxid oder Siliziumoxid.

In einer bevorzugten Ausführungsform der vorliegenden Erfindung umfasst das Verfahren zur Herstellung von offenporigen porösen Membranen zur diffusiven und/oder elektrophoretischen Abtrennung von Carbonsäuren aus flüssigen Stoffgemischen die folgenden Schritte:

a) Bereitstellen einer offenporigen Trägermembran
b) Einbringen einer Mono-und/oder Oligomerlösung in die besagte offenporige Trägermembran aus Schritt a),
c) Reaktionsaktivierung zur Ausbildung eines offenporigen raumfüllenden nanocavitären polymeren Strukturnetzwerkes.

Nanocavitäre Polymerstrukturen

Unter dem Begriff "Nanocavitäre Polymerstrukturen" werden Polymerstrukturen verstanden, die nanocavitäre Spalträume ausbilden bzw. solche umschließen und begrenzen. Die nanocavitären Spalträume werden hierin auch als "nanocavitäre Raumstrukturen" bezeichnet, da sie verschiedene geometrische Formen aufweisen können, wobei sie rund, polygonal oder schlitzförmig konfiguriert sein können. "Nanocavitär" bedeutet hierbei, dass der minimale Abstand zwischen zwei polymeren Grenzflächen vorzugsweise zwischen 0,01 und 100nm, mehr bevorzugt zwischen 0,1 und 50nm und weiter bevorzugt zwischen 1 und 10nm liegt. Der maximale Abstand zwischen zwei polymeren Grenzflächen ist dabei vorzugsweise zwischen 1 und 500nm, mehr bevorzugt zwischen 5 und 200nm und weiter bevorzugt zwischen 10 und 100nm. Vorzugsweise sind die nanocavitären Raumstrukturen miteinander verbunden und können durch ein Gas oder Carbonsäuren durchströmt werden. Die nanocavitäre Polymerstrukturen sind offenporig, da die nanocavitären Spalträume miteinander durchgängig und offen verbunden sind und somit offene Verbindungen zwischen äußeren Grenzflächen ermöglichen. Bevorzugt beträgt die Länge einer transmembranösen Wegstrecke durch die miteinander verbundenen oder nicht unmittelbar miteinander verbundenen nanocavitären Raumstrukturen zwischen 1$\mu$m und 30mm, weiter bevorzugt zwischen 5$\mu$m und 3mm und weiter bevorzugt zwischen 50 $\mu$m und 1 mm.

Die Polymerstrukturen können in verschiedenen Konfigurationen vorliegen und kategorisiert werden, vorzugsweise als faden/bandförmig, netzartig, flächig oder nodulös, aber auch andere Geometrien und Kombinationen hiervon sind möglich. Die Polymerstrukturen sind durchgängig miteinander kovalent verbunden und mit den Grenzflächen der offenporigen Trägermembran physio- und/oder chemosorptiv verbunden.

In einer bevorzugten Ausführungsform der vorliegenden Erfindung umfasst das Verfahren zur Herstellung von offenporigen Membranen zur diffusiven und/oder elektrophoretischen Abtrennung von Carbonsäuren aus flüssigen Stoffgemischen die folgenden Schritte:

a) Bereitstellen einer offenporigen Trägermembran,
b) Einbringen einer Mono-und/oder Oligomerlösung in die offenporige Trägermembran aus Schritt a),
c) Reaktionsaktivierung zur Ausbildung offenporiger raumfüllender nanocavitärer Polymerstrukturen,

wobei die offenporige Membran durchgängige Verbindungen mit minimalen Durchmessern zwischen 0,01 nm und 100nm und
das offenporige raumfüllende nanocavitäre polymere Strukturnetzwerk durchgängige Verbindungen mit maximalen Durchmessern zwischen 1 nm und 500nm aufweisen.

Die erfindungsgemäß hergestellten nanocavitären Polymerstrukturen sind raumfüllend. Der Begriff "raumfüllend" bedeutet in diesem Zusammenhang, dass die Polymerstrukturen dreidimensional den Raum innerhalb der raumgebenden offenporigen Trägermembran einnehmen. Eine Beschichtung von Oberflächen ist beispielsweise nicht raumfüllend. Demnach handelt es sich bei den erfindungsgemäß hergestellten nanocavitären Polymerstrukturen nicht um Oberflächenbeschichtungen, obgleich sie die Oberfläche der raumgebenden offenporigen Trägermembran bedecken, mit ihr in Kontakt stehen oder an sie direkt oder indirekt physio- und/oder chemosorptiv gebunden sein können. Die Polymerstrukturen begrenzen dabei die Spalt- bzw. Hohlräume innerhalb der offenporigen Trägermembran.

Selektivitätsindex

Der Selektivitätsindex $\alpha$, wie hierin definiert, bezieht sich auf die Stoffmenge einer Carbon- oder Fettsäure im Vergleich zu einer organischen Verbindung (ref) mit einem vergleichbaren Molekulargewicht ($\pm$30%), die keine Carbon- oder Fettsäure ist, die durch eine erfindungsgemäße Membran pro Zeiteinheit transportiert wird unter Anlegen eines

elektrischen Gradienten oder eines Konzentrationsgradienten.

$$\alpha = \frac{v_{COOH}}{v_{ref}} = \frac{n_{COOH}/t_{COOH}}{n_{ref}/t_{ref}}$$

wobei v die Transportgeschwindigkeit in mol/s, n die transportierte Stoffmenge und t die Zeit ist.
Bei gleicher Ausgangskonzentration ($n_{0,COOH} = n_{0,ref}$) an Carbonsäure und organischer Verbindung und gleicher Zeit ($t_{COOH} = t_{ref}$), kann der Selektivitätsindex aus der relativen transportierten Stoffmenge $n/n_0$ bestimmt werden. Die Gleichung lautet wie folgt:

$$\alpha = \frac{v_{COOH}}{v_{ref}} = \frac{n_{COOH}/n_0}{n_{ref}/n_0}$$

Bevorzugt sind erfindungsgemäße Membranen, die einen Selektivitätsindex $\alpha$ von >4, vorzugsweise von >6, mehr bevorzugt von >8 und am meisten bevorzugt von >10 aufweisen.
Vorzugsweise wird der Selektivitätsindex $\alpha_{OH}$ zwischen einer Carbonsäure und dem korrespondierenden Alkohol bestimmt:

$$\alpha_{OH} = \frac{v_{COOH}}{v_{OH}} = \frac{n_{COOH}/t_{COOH}}{n_{OH}/t_{OH}}$$

Bevorzugt sind erfindungsgemäße Membranen, die einen Selektivitätsindex $\alpha_{OH}$ von >4, vorzugsweise von >6, mehr bevorzugt von >8 und am meisten bevorzugt von >10 aufweisen.
Der Selektivitätsindex $\alpha_h$ charakterisiert den Transport von Carbon- oder Fettsäuren durch eine erfindungsgemäße Trennmembran im Vergleich zu einem hydrophilen Molekül ($K_{OW}$ < 1) mit einem vergleichbaren Molekulargewicht (+/- 30%).

$$\alpha_h = \frac{v_{COOH}}{v_h} = \frac{n_{COOH}/t_{COOH}}{n_h/t_h}$$

Der Selektivitätsindex $\alpha_l$ charakterisiert den Transport von Carbon- oder Fettsäuren durch eine erfindungsgemäße Trennmembran im Vergleich zu einem hydrophoben Molekül ($K_{OW}$ > 1) mit einem vergleichbaren Molekulargewicht (+/- 30%), das keine Carboxylgruppe trägt. $K_{OW}$ bezieht sich dabei auf den Verteilungsquotienten einer Verbindung in einem Gemisch aus Octanol und Wasser.

$$\alpha_l = \frac{v_{COOH}}{v_l} = \frac{n_{COOH}/t_{COOH}}{n_l/t_l}$$

Bevorzugt sind erfindungsgemäße Membranen, die einen Selektivitätsindex $\alpha_h$ von >8, mehr bevorzugt von >12 und am meisten bevorzugt von >20 aufweisen.
Bevorzugt sind erfindungsgemäße Membranen, die einen Selektivitätsindex $\alpha_l$ von >8, mehr bevorzugt von >10 und am meisten bevorzugt von >15 aufweisen.

Carbonsäuren

Unter dem Begriff "Carbonsäuren" werden organische Moleküle zusammengefasst, die als gemeinsames Merkmal eine oder mehrere Carboxylgruppe(n) (-COOH) aufweisen. Die häufigsten Formen von Carbonsäuren umfassen Verbindungen der allgemeinen Formel R-COOH, wobei R einen aliphatischen Rest $CH_3$-$(CH_2)_n$-darstellt. Allerdings können genauso gut verzweigte und substituierte Alkyl-, Alkenyl- oder Alkinylreste sowie zyklische Kohlenstoffreste eine Carboxylgruppe tragen. Ferner umfasst der Begriff Verbindungen mit mehreren Carboxylgruppen.
Als Fettsäuren werden Carbonsäuren mit mindestens 4 Kohlenstoffatomen bezeichnet. Gesättigte Fettsäuren haben für gewöhnlich eine gerade Kette und eine gerade Anzahl an Kohlenstoffen (n = 4 - 30). Sie unterliegen der allgemeinen Formel $CH_3(CH_2)_nCOOH$. Sie umfassen u. a. Buttersäure, Capronsäure, Caprylsäure, Caprinsäure, Laurinsäure, Myristinsäure, Palmitinsäure, Margarinsäure, Stearinsäure, Arachinsäure, Behelsäure und Lignocerinsäure.
Monoolefinische Fettsäuren sind einfach ungesättigte Fettsäuren und haben die allgemeine Struktur $CH_3(CH_2)_xCH=CH(CH_2)_yCOOH$. Sie können die einzige Doppelbindung an einer Reihe von verschiedenen Positionen haben. Einige wichtige monoolefinische Säuren sind Myristoleinsäure, Palmitoleinsäure, Petroselinsäure, Ölsäure, Vaccensäure, Gadoleinsäure, Gondoinsäure, Erucinsäure und Nervonsäure. Polyolefinfettsäuren werden auch vielfach ungesättigte Fettsäuren (polyunsaturated fatty acids, PUFA) genannt. Diese Fettsäuren haben zwei oder mehr cis-Doppelbindungen, die am häufigsten durch eine einzelne Methylengruppe voneinander getrennt sind (Methylen-getrennte Polyolefine). Linolsäure ist ein typischer Vertreter dieser Gruppe. Bei einigen anderen vielfach ungesättigten Fettsäuren tritt eine Verschiebung einer ihrer Doppelbindungen auf, die nicht wieder durch eine Methylen-Gruppe getrennt sind und als konjugierte Fettsäuren bekannt sind. Einige ungewöhnliche Fettsäuren haben nicht die reguläre Struktur mit einer Methylengruppe zwischen zwei Doppelbindungen, sondern sind durch mehrere Methylengruppen getrennte Polyolefine.
Die wichtigsten Polyenfettsäuren können in 2 Gruppen mit einem gemeinsamen strukturellen Merkmal unterteilt werden: $CH_3(CH_2)_xCH=CH$- mit x=4 für die (n-6) Gruppe und mit x=1 für die (n-3) Gruppe. Vertreter dieser Gruppe sind Linolsäure, Linolensäure, Arachidonsäure, Stearidonsäure, EPA, DPA, DHA und Meadsäure. Die häufigsten Polyolefinsäuren sind Octadecatriensäuren.
Von den ungesättigten durch mehrere Methylengruppen getrennten Fettsäuren, die im Pflanzenreich vorkommen, findet man diejenigen mit einer cis-5 Ethylen-Bindung an verschiedenen Fundorten. Die drei häufigsten Fettsäuren mit dieser Struktur sind Taxoleinsäure (all-cis-5,9-18:2), Pinolensäure (all-cis-5,9,12-18:3) und Sciadonsäure (all-cis-5,11,14-20:3). Einige Isoprenoid-Fettsäuren sind bekannt, z. B. die Retinsäure. Weitere Beispiele für verzweigte Fettsäuren sind Pristansäure und Phytansäure. Einige Fettsäuren enthalten entweder in der Kette einen Cyclopropan-Ring oder einen Cyclopropen-Ring oder am Ende der Kette einen Cyclopenten-Ring. Weitere Carbonsäuren umfassen z. B. die Cyclopropansäuren, wie z. B. die Lactobacillinsäure (11,12-Methylen-Octadecansäure), weiterhin Cyclopropansäuren, Epoxysäuren, z. B. 9,10-Epoxystearin- und 9,10-Epoxy-octadec-12-en-(Coronarinsäure) säure. Ferner Acetylen-Fettsäuren, auch bekannt als Ethinsäuren, wie die Taririnsäure (6-Octadecinsäure). Des weiteren Hydroxyfettsäuren, wobei die Hydroxylgruppe an verschiedenen Positionen in der Kohlenstoffkette auftreten kann, die gesättigt oder einfach ungesättigt sein können. Beispiele sind Ricinoleinsäure (12-Hydroxy-9-octadecensäure), und Lesquerolinsäure, das C20-Homolog der Ricinoleinsäure (14-Hydroxy-11-eicosensäure). Ebenso Di- oder Tricarbonsäuren, Beispiele hierfür sind Adipinsäure, Pimelinsäure, Suberinsäure, Azelainsäure, Sebacinsäure, Brassylinsäure und Thapsinsäure.

**Methoden**

Oberflächenfunktionalisierung

Unter dem Begriff Oberflächenfunktionalisierung wird für die vorliegende Erfindung die Veränderung der Oberflächeneigenschaften durch Einbringung chemischer Reste, vorzugsweise mit funktionalen Gruppen, auf die Oberfläche der raumbegrenzenden Strukturen der offenporigen Trägermembran, sowohl auf dessen äußere Oberfläche als auch auf die Oberfläche der raumgebenden Strukturen, verstanden. Die Einbringung chemischer Reste kann physio- oder chemosorptiv sowie als Kombination aus beidem geschehen. Hiermit können Mono- bis Multilagen von Molekülen auf den Oberflächen einer Stützstruktur aufgebracht werden.

*1. Physisorption*

Hierbei erfolgt die Anbindung einer Verbindung an eine Substratoberfläche durch elektrostatische Wechselkräfte. Eine physiosorptive Aufbringung ist vorteilhaft, da die Beschichtungstechnik, durch einfaches Benetzen mit der in Lösung befindlichen Substanz (z.B. durch Dip-Coating) vollflächig aufgebracht werden kann. Es können Schichtdicken von wenigen Nanometern bis in den Millimeterbereich hergestellt werden. Typische Verbindungen für physiosorptive Anbindungen sind z.B. Polymere mit vielen unterschiedlichen Ladungsgruppen und amphiphile Moleküle, wie z.B. Phospholipide oder Carbonsäuren sowie hydrophile Verbindungen, wie Elektrolyte oder Polyelektrolyte.

*2. Chemisorption*

Hierunter wird eine kovalente Anbindung einer Verbindung an die Substratoberfläche verstanden. Eine kovalente Anbindung von organischen Molekülen kann z. B. durch ein SAM auf verschiedenen Substraten (z.B. Gold, Oxiden von Aluminium, Zirconium, Titan oder Silizium) über reaktive Gruppen an den anzubindenden Molekülen HS-R (Thiole), R-SS-R' (Disulfide), R-S-R (Dialkylsulfide), $S_2CO$-R (Alkylxanthate), R(4-n)-Si-$(R^1)_n$, $R^1$: -OMe, -OEt, -Cl, -H, -$NH_2$ (n = 1-3), Carbonsäuren (R-COOH), Phosphonsäuren (R-P(O)$(OH)_2$) erfolgen.

*3. Gasphasenabscheidungsverfahren*

Zu den Verfahren, bei denen die aufzubringenden Moleküle aus einer Gasphase auf Oberflächen abgeschieden und zur Reaktion gebracht werden, gehören die Atomic Layer Deposition (ALD), Chemical Vapour Deposition (CVD), Plasma-Enhanced Chemical Vapour Deposition (PECVD), Chemical Vapour Infiltration (CVI). Gasabscheideverfahren sind besonders geeignet zur Aufbringung von Monolagen der in der Gasphase befindlichen Verbindung, es können aber auch mehrere Schichten und Polymerisationsprozesse erreicht werden. Bekannt sind auch Beschichtungen mit Silanen und organischen Molekülen.

a) Methoden zur Oberflächenvorbehandlung/ -aktivierung

Verschiedene Membranmaterialien müssen vor einer Beschichtung mit Funktionsmolekülen chemisch verändert bez. aktiviert werden. So ist es z. B. bei den Metallen Aluminium, Titan, Tantal oder Zirkonium, aber auch bei Silizium, erforderlich, die oberflächlichen Schichten zu oxidieren, um eine ausreichende Anzahl an reaktiven Gruppen (OH) zur Anbindung der Beschichtungsmoleküle zu erhalten. Die hierfür geeigneten Methoden sind bekannt und sind daher nur kurz dargestellt.

Eine häufig verwandte Methode ist die Kombination aus einer Säure (z.B. Schwefelsäure) und einem Oxidationsmittel (z.B. $H_2O_2$), wodurch durch das hohe Oxidationspotential und den extrem niedrigen pH-Wert sowohl eine Reinigung der Oberfläche als auch eine Oxidation der Substratoberfläche erreicht wird. Ferner kann dies auch mit einer starken Base (z.B. $NH_3$) und einem Oxidationsmittel erreicht werden.

Ein weiteres Verfahren, um die Substratoberflächen zu reinigen und zu aktivieren, ist die Anwendung von Strahlungsenergie, beispielsweise im UV-Bereich.

Ferner können sowohl zur Reinigung von Oberflächen als auch zu deren Aktivierung Plasmaverfahren eingesetzt werden. Plasma ist ein komplexer Gaszustand von Materie, der aus freien Radikalen, Elektronen, Ionen, Photonen etc. besteht. Plasma kann durch kontinuierliche elektrische Entladung entweder in einem Inertgas oder einem reaktiven Gas gebildet werden. Für die Anwendung bei einer Membran kann Plasma die Eigenschaften sowohl der porösen Substanz als auch eines Polymerfilms für die Gastrennung verbessern. Poröse Membranen können einer Plasmabehandlung unterzogen werden unter Erreichung der folgenden Effekte:

(i) Vernetzung der obersten Schicht und Reduzierung der Porengröße;
(ii) Einführung von funktionalen Gruppen auf der Oberfläche oder
(iii) Abscheidung und Ablagerung einer dünnen selektiven Schicht auf dem porösen Substrat.

Für eine Plasmabehandlung sind Inertgase, wie Argon oder Helium geeignet.

Eine zweite Anwendung ist die Einführung von funktionalen Gruppen. Plasmabehandlung mit Luft, Sauerstoff oder Wasserdampf führt sauerstoffhaltige funktionale Gruppen auf der Oberfläche ein. Stickstoff-, Ammoniak- und Alkylaminplasma führen stickstoffhaltige funktionale Gruppen ein. Ammoniakplasma wurde dafür verwendet, den Durchfluss und die Selektivität von UF-Polysulfonmembranen zu verbessern. Stickstoff- und Sauerstoffplasma wurde verwendet, um die Hydrophilie von Polyvinylchloridmembranen zu verbessern. Hydrophilisierung kann auch durch plasmainduzierte Abscheidungspolymerisation unter Einschluss hydrophiler Monomere erreicht werden. Das Plasma wird dann von gasförmigen organischen Molekülen gebildet, die polymerisieren und auf der Membranoberfläche vernetzen.

In einer bevorzugten Ausführungsart wird Plasma zur Entfernung von Polymerstrukturen an einer oder beiden Außenseiten der erfindungsgemäß hergestellten Membranen verwandt.

<u>Polymerisationsverfahren</u>

In einer bevorzugten Ausführungsform erfolgt die Herstellung nanocavitärer Polymerstrukturen durch eine insitu-Polymerisation innerhalb der Raumstrukturen offenporiger Trägermembranen. Hierzu ist es in der Regel erforderlich,

die Oberflächen der Raumstrukturen innerhalb der offenporigen Trägermembran, einer Vorbehandlung zu unterziehen. Die Art der Einbringung einer Lösung/Suspension mit reaktiven Mono-/Oligomeren in die Raumstrukturen der offenporigen Trägermembran hängt u.a. von deren Viskosität, den Dimensionen der Raumstrukturen und den Reaktionsbedingungen ab und kann daher erheblich variieren. Die Art der Einbringung der Monomer-/Oligomerlösung kann durch Tränken, Aufgießen, Einlegen der offenporigen Trägermembran erfolgen oder erfolgt durch einen kontinuierlichen oder diskontinuierlichen Volumenstrom der Mono-/Oligomerlösung, der durch die Membran geleitet wird. Dabei ist es vorteilhaft alle Raumstrukturen der offenporigen Trägermembran vollständig mit der Mono-/Oligomerlösung zu befüllen. Die Befüllung kann auch mehrmals nacheinander erfolgen. Dies ist insbesondere dann erforderlich, wenn aufgrund der Viskosität der Lösung eine niedrige Konzentration der Mono-/Oligomere gewählt werden muss. Der Polymerisationsvorgang kann unter statischen Bedingungen in einem geeigneten Gefäß erfolgen oder unter dynamischen Bedingungen (Durchfluss) in einer Durchflussapparatur. Vorzugsweise erfolgen die Beschickung der offenporigen Membran mit der Lösung sowie der Polymerisationsvorgang unter Schutzgasbedingungen. Die Einbringung der Mono-/Oligomerlösungen erfolgt bevorzugt bei einer Temperatur von -10 bis 100°C, mehr bevorzugt ist diese zwischen 0°C und 80°C und weiter bevorzugt zwischen 10°C und 40°C.

Die Polymerisation erfolgt durch eine radikalische oder nukleophile Reaktion reaktiver Monomer- und/oder Oligomere. Vorzugsweise wird der Polymerisationsvorgang unmittelbar vor, während und/oder nach dem Einbringen der Mono-/Oligomerlösung in die offenporige Trägermembran initiiert. Die Polymerisation zu nanocavitären Polymerstrukturen erfolgt dann in-situ. Sie wird initiiert und/oder unterhalten durch eine chemische Reaktion, die mit Verbindungen, die sich an den Oberflächen der offenporigen Trägermembran befinden und/oder in der Mon-/Oligomerlösung enthalten sind, erfolgt. Derartige Verbindungen sind u.a. Nucleophile, wie Amine, Amide, Alkoholate, Hydroxidionen, Thiolate, Triethylamin, Ammoniak, Pyridine, wie 4-Dimethylaminopyridin, Phosphine, Carbene, wie Imidazol-2-ylidene und Imidazolin-2-ylidene oder Thiazol-2-ylidene. Kationische Katalysatoren, wie Trifluormethansulfonsäure und Methyltrifluormethansulfonat, bifunktionale Organokatalysatoren, wie [1-(3,5-bis(trifluoromethyl)phenyl)-3-(2-dimethylamino-cyclohexyl)thiourea] oder [1,5,7-triazabicyclo(4.4.0)dec-5-ene (TBD). Katalysatoren, wie Grubb's Katalysator, Metallionen, wie Kupfer, Zinn, wie Zinnoctanoat (Sn(Oct)2), Aluminumalkoxide Al(OR)3, Titaniumalkoxide (Ti(OR)4), Kobalt oder Nickel, Säuren, wie Ascorbinsäure, Schwefelsäure oder Phosphorsäure, weiterhin Azo-Verbindungen, wie AIBN oder Peroxide, wie Benzoylperoxid. Initiatoren einer Polymerisationsreaktion sind ferner Lösungsmittel, wie Wasser, DMF, NMP, DMA, NMP, DMSO, Tetramethylharnstoff.

Bevorzugte Polymerisationsverfahren, mit denen nanocavitäre Polymerstrukturen erhalten werden können, umfassen Verfahrensschritte, die unter den Begriffen "Grafting from"-Verfahren, "Grafting through"-Verfahren oder "grafting to" - Verfahren bekannt sind.

Besonders bevorzugt sind Polymerisationsverfahren auf der Basis einer nukleophilen Reaktionsinitiierung, wie der Atom Transfer Radical Polymerisation (ATRP), der "ring-opening metathesis polymerization" (ROMP), der anionischen oder kationischen Polymerisation, sowie der freien lebenden radikalischen Polymerisation, aber auch der strahlungs-induzierten Polymerisation, der ringöffnenden Olefinmetathese Polymerisation, der reversiblen Additions-Fragmentierungs-Kettenübertragungspolymerisation, der Nitroxid-vermittelten Polymerisation, Polykondensationsreaktionen sowie einer iniferter-induzierten Polymerisation.

Bevorzugte reaktive Mono-/Oligomere sind Benzyl-L-Glutamat-NCA, Phenylalanin-NCA, H-Lys(Z)-NCA, Alanin-NCA, Valin-NCA, oder Kombinationen hiervon.

Die reaktiven Mono-/Oligomere werden in Form von Lösungen oder Suspensionen in mit einer bevorzugten Konzentration zwischen 1 mmol/l und 3 mol/l in die offenporigen Trägermembranen eingebracht.

Die Initiierung/Katalyse der Polymerisationsreaktion, die zu nanocavitären Polymerstrukturen führt, kann auch durch bzw. in Kombination mit physikalische(n) Reaktionsbedingungen erfolgen. Hierzu zählt insbesondere die Erwärmung des Substrates (offenporige Trägermembran, enthaltend die Mono-/Oligomerlösung), bevorzugt ist eine Erwärmung auf 40 bis 160°C, mehr bevorzugt auf 60° bis 120°C und weiter bevorzugt auf 80° bis 110°C. Die Polymerisationsreaktion kann bei unterschiedlichen Umgebungsdrücken erfolgen. Ein Überdruck kann beispielsweise bei einer Temperaturerhöhung des Substrates verwandt werden. Bevorzugt ist die Beaufschlagung mit einem Druck zwischen 1,1bar und 10bar. Ferner bevorzugt ist die Anlage eines Unterdruckes zwischen 10 und 900mbar.

Die Temperierung und der Umgebungsdruck des Substrates können im Verlauf der Polymerisation variiert werden. So kann in einer Ausführungsform das Substrat zunächst mit einem erhöhten Druck beaufschlagt werden und im Verlauf wird dieser kontinuierlich oder stufenweise erniedrigt. Die Dauer eines gegenüber der Umgebungstemperatur und der Umgebung veränderten Druckes erfolgt vorzugsweise über 10 Minuten bis 72 Stunden, mehr bevorzugt über 30 Minuten und 48 Stunden und weiter bevorzugt über 2 und 24 Stunden.

Die erfindungsgemäße Herstellung von nanocavitären Polymerstrukturen kann auch durch eine Polykondensation der in die Raumstrukturen der offenporigen Trägermembran eingebrachten Mono-/Oligomere, in Form einer Schmelze, erzielt werden. Vorzugsweise werden hierbei Temperaturen gewählt, die um oder oberhalb des individuellen Schmelzpunktes der eingesetzten Monomere liegen. bevorzugt ist eine Erwärmung auf 40 bis 200°C, mehr bevorzugt auf 80° bis 140°C und weiter bevorzugt auf 90° bis 140°C. Ein weiteres bevorzugtes Verfahren zur Polymerisationsinitiierung

ist eine Exposition der mit einer Mono-/Oligomerlösung getränkten Membran mit einer lang- oder kurzwelligen Strahlung. Besonders bevorzugt ist die Applikation von Mikrowellen.

Die Aufreinigung der Membranen nach erfolgter Polymerisation erfolgt durch Einlegen in bevorzugt THF, DMF oder DCM. Die erfolgreiche Polymerisation wird durch analytische Methoden, wie Kontaktwinkelmessung, Rasterelektronenmikroskopie oder Infrarotspektroskopie nachvollzogen.

In einer bevorzugten Ausführungsform der vorliegenden Erfindung umfasst das Verfahren zur Herstellung von offenporigen biokompatiblen und hämokompatiblen Membranen zur diffusiven und/oder elektrophoretischen Abtrennung von Carbonsäuren aus flüssigen Stoffgemischen die folgenden Schritte:

a) Bereitstellen einer raumgebenden offenporigen Trägermembran,
b) Einbringen einer Monomer- und/oder Oligomerlösung in die offenporige Trägermembran aus Schritt a),
c) Reaktionsaktivierung zur Ausbildung eines offenporigen raumfüllenden nanocavitären Polypeptid-Strukturnetzwerkes,

wobei die eingebrachte Monomer- und/oder Oligomerlösung mindestens eine Aminosäure und/oder mindestens ein Oligopeptid enthält und das gebildete offenporige raumfüllende nanocavitäre Polypeptid-Strukturnetzwerk aus einer oder verschiedenen Aminosäuren besteht.

Post-Polymerisationsfunktionalisierung

Nach Herstellung der erfindungsgemäßen nanocavitären Polymerstrukturen können in weiteren bevorzugten Ausführungsformen funktionelle Gruppen /Verbindungen auf die Polymerstrukturen aufgebracht oder in die Hohlräume eingebracht und/oder auf/an eine oder beide äußeren Oberflächen der Membranen gebracht werden. Bevorzugte Verbindungen, die kationische bzw. polykationische Oberflächeneigenschaften durch Physio- oder Chemisorption ermöglichen, sind Amine. Hierzu gehören u.a. aliphatische und cycloaliphatische Amine, vorzugsweise Methylamin, Ethylamin, Propylamin, Butylamin, Pentylamin, Hexylamin, Heptylamin, Octylamin, Nonylamin, Decylamin, Undecylamin, Dodecylamin, Tridecylamin, Stearylamin, Palmitylamin, 2-Ethylhexylamin, Isononylamin, Hexamethylenimin, Dimethylamin, Diethylamin, Dipropylamin, Dibutylamin, Dihexylamin, Ditridecylamin, N-Methylbutylamin, N-Ethylbutylamin; alicyclische Amine, vorzugsweise Cyclopentylamin, Cyclohexylamin, N-Methylcyclohexylamin, N-Ethylcyclohexylamin, Dicyclohexylamin; Diamine, Triamine und Tetraamine, vorzugsweise Ethylendiamin, Propylendiamin, Butylendiamin, Neopentyldiamin, Hexamethylendiamin, Octamethylendiamin, Imidazol, 5-Amino-l, 3-trimethylcyclohexylmethylamin, Diethylentriamin, Dipropylentriamin, Tripropyltetraamin, 4,4' - Methylenbiscyclohexylamin, 4, 7-Dioxadecyl-l, 10-diamin, 4, 9-Dioxadodecyl-l, 12-diamin, 4,7, 10-Trioxatridecyl-l, 13-diamin, 2- (Ethylamino) ethylamin, 3-(Methylamino)propylamin, 3- (Cyclohexylamino) propylamin, 3- (2-Aminoethyl) aminopropylamin, 2- (Diethylamino) ethylamin, 3- (Dirnethylamino) propylamin, Dirnethyldipropylentriamin, 4-Aminomethyloctan-l , 8-diamin, 3- (Diethylamino) propylamin, N,N-Diethyl-1, 4-pentandiamin, Diethylentriamin, Dipropylentriamin, Bis (hexamethylen) triamin, Aminoethylpiperazin, Aminopropylpiperazin, N,N-Bis (aminopropyl)methylamin, N,N-Bis (aminopropyl) ethylamin, N,N-Bis (aminopropyl) hexylamin, N,N-Bis (aminopropyl) octylamin, N,N-Dimethyldipropylentriamin, N,N-Bis (3-dimethylaminopropyl) amin, N,N' 1, 2-Ethandiylbis (1,3-propandiamin), N-(Aminoethyl)piperazin, N- (2-Imidazol)piperazin, N-Ethylpiperazin, N-(Hydroxyethyl)piperazin, N- (Aminoethyl) piperazin, N- (Aminopropyl) piperazin, N-(Aminoethyl) morpholin, N- (Aminopropyl) morpholin, N- (Aminoethyl) imidazol, N-(Aminopropyl) imidazol, N- (Aminoethyl) hexamethylendiamin, N- (Aminopropyl) hexamethylendiamin, N (Aminopropyl) ethylendiamin, N (Aminoethyl) butylendiamin, N (Aminopropyl )butylendiamin, Bis (aminoethyl) piperazin, Bis (aminopropyl) piperazin, Bis (aminoethyl) hexamethylendiamin, Bis (aminopropyl) hexamethylendiamin, Bis (aminoethyl) ethylendiamin. Bis (aminopropyl) ethylendiamin, Bis (aminoethyl) butylendiamin, Bis (aminopropyl) butylendiamin, aliphatische Aminoalkohole, Diethanolamin, Bis (hydroxyethyl) aminoethylamin, Bis (hydroxypropyl) aminoethylamin und andere aminogruppenhaltige Verbindungen, wie z.B. Melamin, Harnstoff, Guanidin, Polyguanide, Amidine, Piperidin, Morpholin, 2,6-Dimethylmorpholin und Tryptamin. Bevorzugte Amine werden unter Hexamethylendiamin, Octylamin, Monoethanolamin, Octamethylendiamin, Diaminododecan, Decylamin, Dodecylamin, Betaine, wie Polycarboxybetaine, Arginin und deren Mischungen ausgewählt. Polykationisch bedeutet in diesem Zusammenhang, dass eine Funktionalisierung mit einer Vielzahl von kationischen Ladungsträgern erfolgt, wobei eine einzelne Verbindung oder Einzelverbindungseinheit eine singuläre kationische Ladungsgruppe enthalten kann. Für eine chemosorptive Anbindung von Polykationen an Verbindungen, wie z.B. Aminosilane (Aminopropyl)triethoxysilan (APTS), aminofunktionalisierte Polymere (Polylysin, Polyvinylamin), Polycarbonsäuren (Polyacrylsäure, Polyglutaminsäure), Polyamide und Polyacrylsäureester, sind bevorzugt weitere Funktionalisierungen und entsprechende Kopplungen vorzunehmen. Dies erfolgt durch Umwandlung der Aminofunktionalitäten mit cyclischem Säureanhydrid (z.B. Glutarsäureanhydrid) zur Carbonsäure. Die Carboxylgruppen benötigen eine Aktivierung durch verschiedene Reagenzien, wie z.B. Pentafluorphenol, N-Hydroxysuccinimid, Thionylchlorid, 1-Ethyl-3-(3-dimethylaminopropyl)carbodiimid. Alternativ kann die Anbindung direkt an funktionelle Silane (wie z.B. (3-Glycidooxypropyl)trimethoxysilan, (3-Iodopropyl)trimethoxysila-

ne, (3-Triethoxysilyl)succinimid-anydrid) erfolgen.

Verwendungen der Membranen

Die erfindungsgemäßen Membranen werden vorzugsweise in Verbindung mit einem diffusiven oder elektrokinetischen Separationsverfahren eingesetzt. Hierzu sind Techniken und Vorrichtungen aus dem Stand der Technik bekannt. Die Verfahren können unter den Begriffen Diffusion, Elektrodialyse und Elektroosmose zusammengefasst werden. Die Diffusionsdialyse (DD) basiert auf dem Prinzip eines spontanen Konzentrationsausgleiches von gelösten Verbindungen aufgrund ihrer Eigenbewegung. Bei Verwendung einer diffusions-offenen Membran erfolgt ein Konzentrationsausgleich einer Verbindung aus einer Lösung mit einer erhöhten Konzentration in eine Lösung mit einer niedrigeren Konzentration dieser Verbindung. Die Elektrodialyse (ED) ist ein elektrochemisch getriebener Membranprozess, in dem ionenselektive Membranen in Kombination mit einer elektrischen Potentialdifferenz benutzt werden, um ionische Spezies von ungeladenen Verbindungen aus einer Lösung zu entfernen. Die Ausgangslösung wird dabei in eine Kammer (Donorkammer), die vorzugsweise eine möglichst große Kontaktfläche des Raumvolumens mit den sie mindestens zweiseitig begrenzenden Membranen aufweist, gefüllt. Die Trennmembranen weisen vorzugsweise eine Selektivität auf, die auf der anodenwärts gerichteten Seite gegenüber Anionen und auf der kathodenwärts gerichteter Seite gegenüber Kationen selektiv ist. Diese Membranen sind vorzugsweise parallel zueinander ausgerichtet und erlauben einen vorzugsweise geradlinigen Elektronenfluss zwischen einer Anode und einer Kathode und sind nicht direkt miteinander elektrisch verbunden. In den mit einer Flüssigkeit gefüllten Räumen, die anodenseitig an die für Anionen permeablen Membran angrenzen und kathodenseitig an die für Kationen permeablen Membran angrenzen, findet eine Aufkonzentration der Anionen bzw. Kationen, die die ionenselektiven Membranen passieren, statt, daher wird das Medium Konzentrat oder Akzeptormedium und der Zellenraum, in dem dies erfolgt, Akzeptorzelle genannt. Sofern eine Abreicherung von Kationen und Anionen gewünscht ist, kann eine ED-Einheit aus sich wiederholenden Einheiten einer Kationenakzeptorkammer, einer Donorkammer und einer Anionenakzeptorkammer, die in einer seriellen Abfolge zwischen einen Gleichspannungsgeber angeordnet werden können, bestehen. Die Abtrennung der Ionen aus der Ausgangslösung erfolgt durch Anlage einer Gleichspannung an eine Anode und eine Kathode. Das Verfahren kann bei einem kontinuierlichen Durchflussbetrieb erfolgen oder unter statischen Bedingungen.

In einer besonders bevorzugten Verfahrensanwendung der erfindungsgemäßen Membranen befinden sich die Kathode und/oder die Anode in flüssigkeitsgefüllten Räumen, der Kathoden- bzw. der Anodenkammer, die vorzugsweise durch eine anionen- bzw. kationenselektive Membran, mit einem cut-off-Bereich, der vorzugsweise < 100D beträgt, von der Donor- bzw. der Akzeptorkammer elektrisch getrennt werden. Zwischen der Donor- und der Akzeptorkammer befindet sich die erfindungsgemäße Membran. In einer weiter bevorzugten Ausführungsart erfolgt eine serielle Anordnung, bestehend aus einer kationenselektiven Membran, die kathodenwärts eine Donorkammer begrenzt, einer erfindungsgemäßen Membran, die anodenwärts die Donorkammer und kathodenwärts eine Akzeptorkammer begrenzt. Bevorzugt ist eine Mehrzahl dieser Sequenz seriell anzuordnen. Die letzte Anordnung mit einer solchen Sequenz wird anodenseitig von einer anionenselektiven Membran, die an eine Akzeptorkammer angrenzt, abgeschlossen. Die Akzeptor- und/oder die Donorkammern können dabei über äußere Anschlüsse jeweils miteinander verbunden sein oder einzeln aus einem Vorlagegefäß der Ausgangslösung bzw. des Akzeptormediums befüllt werden. Bei einer seriellen Verbindung der Kammern untereinander, ist es besonders vorteilhaft, die Ausgangslösung (Donorlösung) vorzugsweise auf der Kathodenseite in das Donorkammersystem der ED-Einheit einzuleiten und die Akzeptorlösung auf der Anodenseite in das Akzeptorkammersystem einzulassen. Hierdurch wird eine besonders vorteilhafte Cross-Flow -Separation erreicht, bei der Carbonsäuren aus der Ausgangslösung in das Akzeptormedium transportiert werden.

Eine weiter besonders bevorzugte Verfahrensanwendung der erfindungsgemäßen Membranen erfolgt in Form eines Hohlkammer-Dialysators, wie er beispielsweise in der Medizintechnik bekannt ist. In einer Ausführungsart wird eine Ausgangslösung über ein Verteilersystem in Röhren oder Kapillaren geleitet, die aus einem erfindungsgemäßen Herstellungsprozess hervorgegangen sind und als Separationsmembran eingesetzt werden können, indem diese mit der Verteilervorrichtung abschließend verbunden sind. Die bevorzugten Röhren oder Kapillaren haben vorzugsweise einen Durchmesser zwischen 50μm und 3cm, mehr bevorzugt zwischen 100μm und 1 cm und weiter bevorzugt zwischen 1 mm und 5mm und weisen eine Länge auf, die vorzugsweise zwischen 1cm und 10m beträgt, mehr bevorzugt liegt diese zwischen 5cm und 2m und weiter bevorzugt zwischen 10cm und 100cm. Die Röhren oder Kapillaren sind an dem anderen Ende mit einem oder mehreren Auffangvorrichtung(en) abschließend verbunden. An das Flüssigkeitsmedium in der Verteiler- und/oder der Auffangvorrichtung kann ein elektrisches Feld eingebracht werden, bevorzugt durch eine Verbindung zu einer Kathodenkammer oder durch deren Platzierung innerhalb des Verteiler- bzw. Auffangsystems. Die Kathodenkammern sind dabei durch eine kationenselektive Membran von dem Ausgangsmedium elektrisch getrennt. Die Röhren/Kapillaren befinden sich in einem offenen oder geschlossenen Behältnis, das ein Akzeptormedium aufnimmt bzw. welches durch das Behältnis durchgeleitet werden kann. Das Akzeptormedium ist elektrisch mit einer Anode verbunden, die vorzugsweise durch eine anionenselektive Membran gegen das Akzeptormedium elektrisch getrennt ist. Unter Anlage einer Spannung zwischen einer oder mehrerer Anode(n) und Kathode(n) erfolgt ein Transport von

Carbonsäuren aus dem Ausgangsmedium, das die Rohre/Kapillaren durchströmt, in das Akzeptormedium, welches sich an den Außenseiten der Rohre/Kapillaren befindet. Die Flussgeschwindigkeit(en) des Ausgangs- und/oder Akzeptormediums beträgt vorzugsweise zwischen 1 mm/min und 100m/min, mehr bevorzugt zwischen 5cm/min und 50m/min und weiter bevorzugt zwischen 10cm/min und 1 m/min. Aber auch eine umgekehrte Abtrennrichtung für Carbonsäuren ist vorstellbar. So können in der Verteiler- bzw. Auffangvorrichtung eine oder mehrere Kathoden und in dem umgebenden Behältnis eine oder mehrere Anoden platziert sein und/oder das Ausgangsmedium wird an den Außenseiten der Rohre oder Kapillaren vorbeigeführt und das Akzeptormedium durchströmt die Rohre/Kapillaren.
Derartige Anwendungen erfolgen vorzugsweise bei Temperaturen der Lösungen zwischen 5 und 120°C mehr bevorzugt zwischen 10° und 75°C und weiter bevorzugt zwischen 15° und 45°C. Vorzugsweise wird der in den Kammersystemen herrschende Druck, z.B. durch Drucksensoren, überwacht und der Differenzdruck zwischen den Kammersystemen monitoriert. Vorzugsweise erfolgt ein Druckausgleich zwischen dem Donor- und dem Akzeptorkammersystem. In einer Verfahrensausführung ist ein Differentialdruck zwischen den Kammersystemen einstellbar. In einer Verfahrensausführung werden weitere Parameter des Akzeptor- und/oder Donormediums monitoriert, wie z. B. der pH, die Leitfähigkeit, die Temperatur, Ionenkonzentrationen oder die Viskosität.
Das Material, aus dem die einsetzbaren Elektroden bestehen, kann dem Stand der Technik entnommen werden. Bevorzugt sind allerdings Elektroden, bestehend aus oder mit einer dauerhaften Beschichtung aus Kohlenstoff, Platin, Gold oder Silber. Die Lösungen, mit denen die Anoden- und Kathodenkammern gefüllt sind, enthalten vorzugsweise ionische oder ionisierbare Verbindungen, die für einen Ladungstransport geeignet sind. Derartige Verbindungen sind im Stand der Technik bekannt.
In einer Verfahrensausführung wird ein Konzentrationsgradient, der zwischen dem Ausgangs- und Akzeptormedium besteht, zum Transport von Carbonsäuren durch eine der erfindungsgemäßen Membranen verwendet. In einer anderen bevorzugten Ausführungsform ist das Akzeptormedium ein organisches Lösungsmittel.
In einer weiter bevorzugten Ausführungsart werden die erfindungsgemäßen Membranen zusammen mit kationischen Verbindungen, die in der Akzeptorkammer gebunden und/oder ungebunden vorliegen, durchgeführt. Dies ist deshalb besonders vorteilhaft, da Carbonsäuren, die durch die Membran in die Akzeptorkammer gelangen, von diesen Verbindungen gebunden werden können, wodurch die Löslichkeit der Carbonsäuren erhöht (z.B. durch Arginin sowie Triethylamin) oder erniedrigt (z. B. durch Natrium, Calcium, Polykationen) werden kann. Besonders bevorzugt sind dabei polykationische Verbindungen, die in der Lage sind, eine Vielzahl von Carbonsäuren gleichzeitig zu binden. Besonders bevorzugt ist Polyethylenimin. In einer besonders bevorzugten Verfahrensausführung, können Carbonsäuren, die aus der Donorlösung durch die Separationsmembran geleitet wurden, mittels kationischer und/oder polykationischer Verbindungen immobilisiert/gebunden werden und in komplexierter Form unmittelbar aus dem Akzeptormedium entnommen und erhalten werden.
In einem weiter bevorzugten Verfahren werden die erfindungsgemäßen Membranen in einem DD- oder ED-Verfahren verwandt, um Carbonsäuren aus einem Donormedium in ein Akzeptormedium zu transportieren und die separierten Carbonsäuren in ein oder mehreren weiteren Verfahrensstufen zu gewinnen. Hierzu kann z.B. die mit Carbonsäuren angereicherte Akzeptorlösung separiert und die hierin gelösten Carbonsäuren durch eine pH-Erniedrigung, die mittels einer Säure eingestellt werden kann, protoniert werden, wodurch eine Phasenseparation erreicht wird und die Carbonsäurephase abgetrennt werden kann.
Bevorzugt ist eine Verwendung von Membranen mit nanocavitären Polymerstrukturen zur Separation von Carbonsäuren, bei der im Akzeptormedium kationische Verbindungen in gelöster und/oder ungelöster Form vorliegen.

**Anwendungen**

Eine selektive Abtrennung von Carbonsäuren ist in vielen Anwendungsbereichen erforderlich. Erfindungsgemäße Membranen können für eine Produktgewinnung oder eine Aufreinigung von Medien angewendet werden. So lassen sich beispielsweise Carbonsäuren, die durch einen chemischen oder bio-technologischen Prozess oder durch eine Aufreinigung in einem wässrigen Medium vorliegen, abtrennen. Hierdurch lassen sich beispielsweise Fettsäuren selektiv gewinnen, die eine Anwendung beispielsweise als Nahrungsmittel finden oder einer Biodiesel-, Schmierstoff- oder Seifen-Produktion zugeführt werden. Aber auch Edukte oder Produkte aus chemischer oder pharmazeutischer Synthese können in reiner Form gewonnen bzw. abgetrennt werden. Carbonsäuren sind fernerhin abzutrennen beispielsweise in der Milchindustrie, Getränkeindustrie, aus Gemischen einer chemischen Umsetzung oder Synthese, aus Reinigungslösungen, die z. B. bei der wässrigen Aufreinigung von Ölen und Fetten entstehen, Abwässern industrieller oder kommunaler Herkunft. Beispiele für Öle sind Industrieöle, Pflanzenöle, Kraftstoffe, Altspeiseöle. Weitere Anwendungen sind möglich bei der Bio-Dieselherstellung, Biomasse-Auftrennung, bio-technologischen Prozesstechnik, Aufreinigung von Blutplasma, Prozessflüssigkeiten, pharmazeutischen Synthesegemischen sowie bei Flüssigkeiten in der chemischen Analytik.
Das erfindungsgemäße Verfahren eignet sich insbesondere, um im Wege der Dialyse Carbonsäuren und insbesondere Fettsäuren aus dem Blut eines Menschen abzutrennen.

Somit ist ein weiterer Aspekt der vorliegenden Erfindung auf ein Verfahren gerichtet, das ein Dialyseverfahren ist und zum Abtrennen von Fettsäuren aus Blut dient. Somit können als Ausgangsmedium Flüssigkeiten verwandt werden, wie Blut oder Blutprodukte, Milchprodukte, Getränke, wässrige Synthesemedien oder wässrige Extraktionen von Synthesegemischen, Reinigungsmedien oder Abwässer industrieller oder kommunaler Herkunft, aber auf diese nicht beschränkt sind. Folglich betrifft ein weiter Aspekt der vorliegenden Erfindung die Verwendung der erfindungsgemäß hergestellten offenporigen Membranen zur diffusiven und/oder elektrophoretischen Abtrennung von Carbonsäuren aus flüssigen Stoffgemischen.
In einer Ausführungsform der vorliegenden Erfindung werden die erfindungsgemäß hergestellten offenporigen Membranen zur elektrophoretischen Abtrennung von Carbonsäuren aus flüssigen Stoffgemischen verwendet.
In einer weiteren Ausführungsform der vorliegenden Erfindung werden die erfindungsgemäß hergestellten offenporigen Membranen zur diffusiven Abtrennung von Carbonsäuren aus flüssigen Stoffgemischen verwendet.
In einer bevorzugten Ausführungsform der vorliegenden Erfindung werden die erfindungsgemäß hergestellten offenporigen Membranen zur Separation von Carbonsäuren aus wässrigen Medien verwendet.
In einer besonders bevorzugten Ausführungsform der vorliegenden Erfindung werden die erfindungsgemäß hergestellten offenporigen Membranen zur Separation von Carbonsäuren aus wässrigen Medien, wobei es sich bei den wässrigen Medien um Blut und Blutprodukte handelt, verwendet.

**Beispiele**

Die folgenden Beispiele sind hier angeführt, um bevorzugte Ausführungsformen der Erfindung zu zeigen. Weitere Modifikationen und alternative Ausführungsformen hinsichtlich der verschiedenen Aspekte der vorliegenden Erfindung sind ersichtlich für den Fachmann. Demnach ist die Beschreibung lediglich darauf ausgelegt die generelle Lehre und die Ausführung der vorliegenden Erfindung näher zu bringen. Es sollte jedoch verstanden werden, dass die gezeigten und beschriebenen Formen der vorliegenden Erfindung als Beispiele der Ausführungsformen angesehen werden sollten. Elemente oder Materialien können ausgetauscht werden gegenüber denen, die in den Beispielen verwendet wurden.

**Beispiel 1:**

Synthese von reaktiven Monomer-Lösungen und Pentafluorphenylester-verbindungen.

A) α-Aminosäure-N-carboxyanhydride (NCA), die für eine ringöffnende Polymerisation (ROP) geeignet sind, wurden nach der Fuchs-Farthing-Methode hergestellt. Dabei erfolgte das Suspendieren von 5g der freien α-Aminosäure (Phenylalanin, Arginin (Schutzgruppe 2,2,4,6,7-Pentamethyldihydrobenzofuran-5-sulfonyl), Lysin (Schutzgruppe tert-Butyloxycarbonyl), Benzylglutamat) in 60 mL THF. Im Anschluss wurden 1/3 Äquivalente Bis(trichloromethyl) carbonat hinzugegeben. Die Reaktion erfolgte unter Schutzgas bei 50°C unter kontinuierlichem Rühren. Nach 2 Stunden wurden 180 mL n-Pentan hinzugegeben und der Feststoff über einer Umkehrfritte abfiltriert und mit n-Pentan gewaschen. Zur Aufreinigung wurde der getrocknete Feststoff in benötigter Menge von THF gelöst und solange mit n-Pentan versetzt bis eine leichte Kristallisation einsetzte. Die Kristallisation wurde für 15 Stunden bei - 28°C vervollständigt. Der Feststoff wurde erneut abfiltriert und wiederholt mit n-Pentan gewaschen. Der Aufreinigungsschritt wurde zweimal wiederholt. Die Reinheit des Produktes wurde über deren Schmelzpunkt überprüft.

B) Synthese der N-(Methoxycarbonyl)-aminosäuren

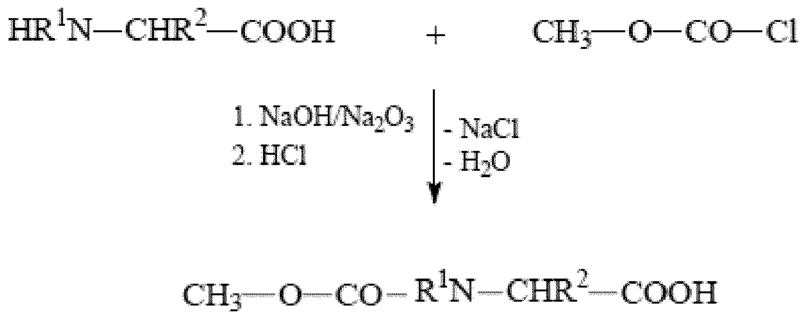

In einem 2-1-Dreihalskolben, ausgestattet mit einem KPG-Rührer werden nacheinander 1 mol Aminosäure und 0.5 mol (53 g) Natriumcarbonat in 1 l NaOH (1 N) gelöst. Die Lösung wird mit Eis/Kochsalz-Mischung auf 0°C gekühlt, dann wird 1 mol (94.6 g) Chlorameisensäuremethylester (ebenfalls vorgekühlt) langsam innerhalb von 30 min unter Rühren zugetropft. Es wird noch 1 h unter Kühlung und 1 h bei RT weitergerührt. Danach wird vorsichtig mit konz. HCl auf pH 2-3 angesäuert. Das Produkt wird durch mehrmaliges Ausschütteln mit insgesamt 1.4 l Ethylacetat/THF (5:2) extrahiert. Die wässrige Phase wird noch zweimal mit jeweils 200 ml Essigester nachextrahiert. Bei der Herstellung von N-MOC-D,L-phenylalanin und N-MOC-L-phenylalanin wird nur mit Dichlormethan extrahiert. Nach Trocknung der vereinigten organischen Phasen mit Natriumsulfat wird das Lösungsmittel vollständig am Rotationsverdampfer entfernt. Das Rohprodukt wird ohne weitere Reinigung weiterverwendet.

C) Die Synthese von Pentafluorophenylestern, die für eine chemosorptive SAM-Formation geeignet sind erfolgte nach dem folgenden Schema:

In einem 100 ml Einhalskolben werden unter Eiskühlung 1 Eq Carbonsäure, 1,1 Eq Pentafluorphenol und 1,1 Eq DCC in 50 ml trockenem Dichlormethan (bei Malon-, Naphthen-, Terephthal- und Phthalsäure in DMF) gelöst. Nach Zugabe des Katalysators bildet sich schnell ein weißer Feststoff (Dicyclohexylharnstoff) aus. Die Suspension wird für 48 h bei Raumtemperatur gerührt, anschließend mit 20 ml Hexan versetzt und im Vakuum vom Feststoff filtriert (G2-Filtertiegel). Nach dem Verdampfen der Lösemittel am Rotationsverdampfer wird das Rohprodukt vom neu gebildeten Feststoff über eine kurze Kieselgel-Säule mit dem Laufmittel Hexan/Chloroform 3:1 getrennt. Abrotieren der Lösemittel mit guten Ausbeuten (in Klammern) der aktivierten Carbonsäuren: PentansäurePfP (86%), HexansäurePfP (83%), OctansäurePfP (71%), HexadecansäurePfP (73%), Eliadinsäure-PfP (78%), PhenylessigsäurePfP (98%), ZimtsäurePfP (64%), β-Naphtolsäure-PfP (84%), PhthalsäurePfP (84%).

D) 3-(Guanidinopropyl-chlorid)-trimethoxysilan

Synthese: 10,0g APTS (55mmol, 1 Eq) und 8,2g 1-Aminopyrazol-HCl (55mmol, 1 Eq) werden in 30ml $MeOH_{abs}$. gelöst und für 48h bei Raumtemperatur mit aufgesetzten Trockenrohr ($CaCl_2$-Füllung) gerührt. Anschließend wird das Lösemittel im Vakuum bei 65°C abdestilliert. Das orange Rohprodukt wird mittels Kurzwegdestillation im Vakuum (10 mbar) bei 150 C gereinigt. Als Produkt erhält man ein orange gefärbtes, hochviskoses Öl. Zur Überführung in das Probengefäß wird es in Methanol gelöst und im Vakuumtrockenschrank bei 80 C über mehrere Stunden getrocknet.

E) Alkyl-Imidazolsilane

Synthese: In einem 100 ml-Einhalskolben werden 13,72 g (0,05 mol, 1 Eq) Imidazolsilan und 2,5 Eq Halogenalkan (C8 und C16) unter N2-Atmosphäre für 24 h am Rückfluss gekocht (100-140 °C). Nach dem Abkühlen auf Raumtemperatur wird das Gemisch dreimal mit Hexan gewaschen. Dazu werden entweder das Hexan zur Reaktionslösung geben, 15 min heftig rühren gelassen und dann das Hexan abpipettieren oder das Reaktionsgemisch wird mit DCM aufgenommen und dreimal mit Hexan ausschüttelt. Die untere Phase wird am Rotationsverdampfer von restlichem Lösungsmittel getrennt.

**Beispiel 2:**

<u>Oberflächenfunktionalisierung von Membranen und Kopplungsreaktionen mit aktivierten Carbonsäuren und reaktiven Monomeren.</u>

Die folgenden Membranmaterialien (Trägermembranen) wurden verwandt: Ultrafiltrationsmembranen aus (a) Zirkonoxid-Keramik mit einer asymmetrischen Porengrößenverteilung (Kerafol, Deutschland), (b) PTFE-Membran, mittlerer Porendurchmesser 1,0 μm (Emflon, Pall, Deutschland), (c) Glasfritte, Sorte D (Schott, Deutschland), (d) Polyethersulfon-Membran (Supor, 0,8μm, Pall, USA) sowie (e) anodisierte Aluminium-oxid-Membranen mit Kanaldurchmessern von 200nm (Anodisc, Whatman, Deutschland).

A) Reinigung der Substrate für eine Oberflächenfunktionalisierung.

Die Membranen (a) - (d) wurden mittels einer basischen Reinigungslösung, bestehend aus deionisiertem $H_2O$, $H_2O_2$ (35%ig) und $NH_3$ (30%ig) im Verhältnis 5:1:1 (v:v:v) bei 80 °C für 10 Minuten gereinigt. Die Prozedur wurde 5mal wiederholt, abschließend Reinigung mit Methanol. Hiernach wurden die Membranen in einer Lösung aus $H_2SO_4$ und $H_2O_2$ (7:3) bei 80 °C über 10 Minuten aufbewahrt. Abschließende Reinigung mit deionisiertem $H_2O$ und Methanol. Die Membranen (e) wurden mit einer $H_2O_2$ (35%ig) bei 80°C für 10 Minuten gereinigt. Abschließende Reinigung mit deio-

nisiertem $H_2O$ und Trocknung bei 70 °C für 2 Stunden.

B) Oberflächenfunktionalisierung.

Es wurden die Aminosilane 3-Aminopropyl-triethoxysilan (APTS) und 3-Trimethoxysilylpropyl-diethylentriamin (TAPTES) (Sigma-Aldrich, USA) in Toluol gelöst und auf 75 °C erhitzt. Hierin wurden die Beschichtungsobjekte für 4 Minuten aufbewahrt und anschließend mit Toluol und Dichlormethan abgespült und dann in einem Vakuumtrockenschrank bei 80 °C über 24 Stunden getrocknet. Qualitätskontrolle mittels Kontaktwinkelmessungen.

C) Kovalente Bindung von Funktionsverbindungen.

Die Membranen wurden in eine Aminosilan-Lösung mit einer Konzentration von 1 Vol% bis 5 Vol% in Toluol bei 120 °C für 4 Stunden eingelegt. Danach reinigen der Membranen mit Toluol und trocknen im Vakuumschrank bei 70 °C.

Die kovalente Kupplung der aktivierten Carbonsäuren an die Membranoberflächen erfolgte durch Einlegen in eine 60 mmolare Lösung des Pentafluorophenylesters in DMF über 15 Stunden und 24 Stunden bei Temperaturen zwischen 0 °C und 80 °C. Bei den Durchflussbeschichtungen erfolgte ein Volumenstrom durch die Membran mit 1 mL/h bis 2 mL/h.

C) Polykondensation von aktivierten Aminosäuren-Monomeren und chemosorptive Anbindung von höhermolekularen Verbindungen.

Diese erfolgte mit reaktiven Monomeren an den Aminosilanen über eine ringöffnende Polymerisation (ROP) von $\alpha$-Aminosäure-N-carboxyanhydriden.
Die Oberflächenfunktionalisierung mit Aminosäure-NCAs (Konzentrationen 40 bis 80 mmol in THF) erfolgte bei den anodisierten Aluminium-Membranen mit einem Durchflussverfahren. Hierzu wurden die Membranen in eine geschlossene Haltevorrichtung eingelegt, die eine kontinuierliche Durchspülung der Membran erlaubte. In eine Glasspritze wurden die Monomerlösungen aufgezogen und durch eine Vorrichtung zum automatischen Stempelvorschub mit einer konstanten Geschwindigkeit über eine Schlauchleitung der Haltevorrichtung zugeführt. Die Reaktionsdauer erfolgte über 6, 12, 24 und 36 Stunden. Ferner wurden Kombinationen der Aminosäuren-NCA's zur Herstellung von Polymerstrukturen verwandt. Die Qualitätskontrolle des Polymerisationsergebnisses erfolgte mittels Kontaktwinkelmessungen und REM.

D) Eine thermisch induzierte Polykondensation der reaktiven Monomere erfolgte mit Phenylalanin, Sarkosin-, Alanin-, Cystein-, Valin- und Lysin-NCAs. Hierzu wurde in einen 50-ml-Erlenmeyerkolben unter Argon 1 g von einem oder mehreren NCA eingewogen. Der Erlenmeyerkolben wurde mit einem durch eine Stahlfeder gesicherten Glasstopfen verschlossen und für 12 h bei 120 °C temperiert. Es entstand eine Feststoffmasse, die gemörsert und in THF aufgenommen wurde. Hierin wurden lineare und cyclischen Oligomere und Polypeptide nachgewiesen.
Für die Beschichtungen wurden 250 mM Lösungen aus Polyphenylalanin-, Arginin-, Lysin- und Benzylglutamat-NCA sowie in weiteren Versuchen zusammen mit Oligomeren und Polypeptiden, die durch eine Polykondensation erzeugt worden waren, in Methyl-tert-butylether (MTBE) hergestellt. 0,5 mL der Lösung wurden auf das 1,33 $cm^2$ große poröse Trägermaterial (mit oder ohne Aminofunktionalität) getropft und der MTBE bei 25 °und 60°C i Vakuum verdampft. Dieser Prozess wurde 8-mal und 16-mal wiederholt. Das Trägermaterial wurde im Anschluss auf 110°C für 15 Stunden erhitzt. Die Membranen wurden in TBME mehrfach gespült. Die erfolgreiche Funktionalisierung wird mittels Kontaktwinkelmessung und REM überprüft.
Wiederholungsversuche erfolgten mit 0,5molaren Lösungen der Mono- und Oligomere, bei denen das Lösungsmittel NMP, DMSO sowie DMF war und die Membranen solange mit den Lösungen wiederholt getränkt wurden, bis keine Aufnahme der Lösung mehr zu erkennen war. Im Anschluss erfolgte eine thermische Umsetzung bei Temperaturen zwischen 80° und 164°C über 8 und 16 Stunden. Anschließend Reinigung der Membranen, wie vorbeschrieben. Eine Feststoffanalyse zeigt das Vorliegen von zyklischen Polypeptiden.

E) Lösungsmittel induzierte Polykondensationen erfolgten, indem 250 mmolare Lösungen aus Phenylalanin-, Arginin-, Lysin-, Benzylgutamat- und Valin-NCAs sowie Kombinationen aus diesen in Dimethylformamid hergestellt wurden. Das Trägermaterial (mit oder ohne Aminofunktionalität) wurde in Inertgasatmosphäre in die Lösungen eingetaucht. Nach 72 Stunden bei Raumtemperatur oder 60 °C wurden die Membran mit 5 mL THF abgespült. Ergänzende Untersuchungen erfolgten in gleicher Weise mit Lösungsmittelgemischen aus THF und DMF (80:20 v:v, sowie 50:50 v:v). Die erfolgte Polymerisation wurde mittels Kontaktwinkelmessung, Infrarotspektroskopie und Rasterelektronenmikroskopie untersucht. Bei Membranen, bei denen sich im REM Polymerstrukturen auf den äußeren Oberflächen der Membranen darstellten, wurden diese mit einem Argonplasma behandelt.

F) Funktionalisierung mit Polykationen.

Membranen, die gemäß der Schritte A) bis E) vorbehandelt worden waren oder native Membranen wurden in eine 10 Gew%igen Polyethylenimin Lösung (Methanol) bei 25°C für 24 Stunden eingelegt. Zur Aufreinigung wurden die Membranen in Methanol eingelegt und anschließend getrocknet. Dieser Schritt wurde dreimal wiederholt. Die Funktionalisierung wurde mit Infrarotspektroskopie überprüft.
Ferner erfolgten Funktionalisierungen mit Mischungen aus Polyethylenimin und Öl-sowie Stearinsäure, die in Pentan gelöst wurden. Die Lösung wurde auf einer Seite der Membran mehrfach aufpipettiert.

Die nach den o. a. Behandlungen erhaltenen Membranen wurden gebrochen oder geschnitten und die Bruchkanten sowie die äußeren Oberflächen elektronenmikroskopisch beurteilt. An den Bruchkanten (sofern möglich) und den äußeren Oberflächen der Membranen erfolgten Wasser- sowie Ölsäure-Kontaktwinkelmessungen.

Ergebnisse:

Beispiele von Membranen, die durch die aufgeführten Herstellungsarten erhalten wurden, sind in Tabelle 1 dargestellt.

Tabelle 1: Beispiele von Membranen, deren Oberflächenfunktionalisierung und Polymerisationsverfahren.

| Bezeichnung | Träger-Membran | Oberflächenfunktionalisierung | Polymersisationsverfahren | Reaktions dauer [h] | Zusätzliche Funktion-alisierung | NC-Strukture-n[a] | Wasser-Kontak-twinkel [°] |
|---|---|---|---|---|---|---|---|
| M1.0 | a | -- | -- | -- | | -- | 36 |
| M1.1 | a | Eliadinsäure-PfP | -- | 8 | | -- | 79 |
| M1.2 | a | PhenylessigsäurePfP | -- | 8 | | -- | 68 |
| M1.3 | a | C16-Imidazolsilan | -- | 8 | | -- | 92 |
| M1.4 | a | Phenylalanin-NCA | ROP/Grafting-from | 36 | | ++ | 88 |
| M1.5 | a | Lysin-NCA | ROP/Grafting-from | 24 | | + | 82 |
| M1.6 | a | Arginin-NCA | ROP/Grafting-from | 24 | | + | 75 |
| M1.7 | a | Benzylglutamat-NCA | ROP/Grafting-from | 12 | | -- | 61 |
| M1.8 | a | Phenylalanin-NCA | LM-Induktion | 2 | | +++ | 83 |
| M1.9 | a | Lysin-NCA | LM-Induktion | 3 | | ++ | 85 |
| M1.10 | a | Arginin-NCA | Thermokatalyse | 2 | | ++ | 79 |
| M1.11 | a | Benzylglutamat-NCA | LM-Induktion | 4 | PEI[b] | ++ | 80 |
| M1.12 | a | Phenylalanin-NCA | Thermokatalyse | 1 | PEI | +++ | 117 |
| M2.0 | b | -- | -- | -- | -- | | 121 |
| M2.1 | b | Phenylalanin-NCA | Thermokatalyse | 2 | | +++ | 111 |
| M2.2 | b | Lysin-NCA | Thermokatalyse | 2 | | +++ | 95 |
| M2.3 | b | Arginin-NCA | LM-Induktion | 4 | | +++ | 93 |
| M2.4 | b | Benzylglutamat-NCA | Thermokatalyse | 2 | | ++ | 82 |
| M2.5 | b | Phenylalanin-NCA | Thermokatalyse | 2 | PEI | +++ | 92 |
| M3.0 | c | -- | -- | -- | -- | -- | 26 |
| M3.1 | c | Eliadinsäure-PfP | -- | 24 | | -- | 77 |
| M3.2 | c | PhenylessigsäurePfP | -- | 24 | | -- | 65 |
| M3.3 | c | C16-Imidazolsilan | -- | 24 | | -- | 82 |
| M3.4 | c | Phenylalanin-NCA | ROP/Grafting-from | 8 | | + | 100 |
| M3.5 | c | Lysin-NCA | ROP/Grafting-from | 8 | | -- | 92 |

(fortgesetzt)

| Bezeichnung | Träger-Membran | Oberflächenfunktionalisierung | Polymersisationsverfahren | Reaktions dauer [h] | Zusätzliche Funktion-alisierung | NC-Strukture-n[a] | Wasser-Kontak-twinkel [°] |
|---|---|---|---|---|---|---|---|
| M3.6 | c | Arginin-NCA | ROP/Grafting-from | 8 | | + | 65 |
| M3.7 | c | Benzylglutamat-NCA | ROP/Grafting-from | 8 | | -- | 62 |
| M3.8 | c | Phenylalanin-NCA | Thermokatalyse | 2 | | +++ | 105 |
| M3.9 | c | Lysin-NCA | LM-Induktion | 3 | | ++ | 99 |
| M3.10 | c | Arginin-NCA | Thermokatalyse | 2 | | ++ | 92 |
| M3.11 | c | Benzylglutamat-NCA | LM-Induktion | 3 | PEI | ++ | 93 |
| M3.12 | c | Phenylalanin-NCA | Thermokatalyse | 1 | PEI | +++ | 92 |
| M4.0 | d | -- | -- | -- | -- | -- | 41 |
| M4.1 | d | Eliadinsäure-PfP | -- | 24 | | -- | 78 |
| M4.2 | d | C16-Imidazolsilan | -- | 24 | | -- | 81 |
| M4.3 | d | Phenylalanin-NCA | ROP/Grafting-from | 24 | | + | 76 |
| M4.5 | d | PA-Lysin-NCA[c] | ROP/Grafting-from | 24 | | + | 70 |
| M4.6 | d | PA-Arginin-NCA | ROP/Grafting-from | 24 | | + | 71 |
| M4.7 | d | Benzylglutamat-NCA | ROP/Grafting-from | 12 | | -- | 66 |
| M4.8 | d | Phenylalanin-NCA | Thermokatalyse | 2 | | +++ | 85 |
| M4.9 | d | Lysin-NCA | LM-Induktion | 3 | | ++ | 80 |
| M4.10 | d | PA-Arginin-NCA | Thermokatalyse | 2 | | +++ | 88 |
| M4.11 | d | Arginin-Benzylglutamat-NCA | LM-Induktion | 3 | | ++ | 81 |
| M4.12 | d | PA-Arginin-Benzylglutamat-NCA | Thermokatalyse | 2 | PEI | +++ | 83 |
| M4.13 | d | Phenylalanin-NCA | Thermokatalyse | 2 | PEI | +++ | 93 |
| M5.0 | e | -- | -- | -- | -- | -- | 25 |
| M5.1 | e | Eliadinsäure-PfP | -- | 12 | | -- | 75 |
| M5.2 | e | PhenylessigsäurePfP | -- | 12 | | -- | 70 |
| M5.3 | e | C16-Imidazolsilan | -- | 12 | | -- | 76 |

(fortgesetzt)

| Bezeichnung | Träger-Membran | Oberflächenfunktionalisierung | Polymersisationsverfahren | Reaktions dauer [h] | Zusätzliche Funktion-alisierung | NC-Strukture-n[a] | Wasser-Kontak-twinkel [°] |
|---|---|---|---|---|---|---|---|
| M5.4 | e | Phenylalanin-NCA | ROP/Grafting-from | 36 | | ++ | 71 |
| M5.5 | e | Lysin-NCA | ROP/Grafting-from | 36 | | + | 68 |
| M5.6 | e | Arginin-NCA | ROP/Grafting-from | 36 | | ++ | 60 |
| M5.7 | e | Benzylglutamat-NCA | ROP/Grafting-from | 36 | | + | 58 |
| M5.8 | e | Phenylalanin-NCA | Thermokatalyse | 2 | | +++ | 88 |
| M5.9 | e | Lysin-NCA | LM-Induktion | 4 | | +++ | 79 |
| M5.10 | e | Arginin-NCA | Thermokatalyse | 2 | | +++ | 83 |
| M5.11 | e | Benzylglutamat-NCA | LM-Induktion | 1 | PEI | +++ | 82 |

[a]NC-Strukturen = nanocavitäre Polymerstrukturen mit: -- keiner, + überwiegend partieller , ++ überwiegend vollständiger und +++ vollständiger Raumfüllung;
[b]PEI = Polyethylenimin;
[c]PA = Phenylalanin

Nach einer Oberflächenbeschichtung mit aktivierten Carbonsäuren sowie nach einer ring-öffnenden-Polymerisation (ROP) von Aminosäure-Monomerlösungen, die durch eine Oberflächenaktivierung eingeleitet wurden, waren unter den gewählten Reaktionsbedingungen keine strang- oder gewebeartigen Strukturen innerhalb der Spalträume der Membranen entstanden. Bei langen Reaktionszeiten der ROP mit Aminosäure-NCA's kam es zum partiellen oder vollständigen Verschluss der Innenräume der Trägermembranen. Bei thermokatalytischen und lösungsmittelinduzierten Polymerisationsreaktionen der eingesetzten Aminosäure-NCA's kam es zur Ausbildung raumfüllender Polymerstrukturen mit nanocavitären Spalten und Kanälen, bei einer vollständigen Bedeckung der inneren Membranoberflächen.
Beschichtungen mit Alkylsilanen erreichten Wasserkontaktwinkel (WKW) von 59° bis 79°. Die WKW der Aminosäurepolymere lagen zwischen 68° und 117° und die der Alkyl-Imidazolsilane zwischen 66° und 92°. Die Guanidinsilane wiesen WKW um 68° auf. Für die Kombination von Aminosäurepolymeren oder Guanidinsilanen zusammen mit Alkylsilanen resultierten WKW von 95 - 110°.
Die Fettsäure-Kontaktwinkel (FKW) für Beschichtungen mit Alkansilanen betrugen zwischen 10° und 25°, die für Aminosäure-Polymere zwischen 0° bis 20°, für die Alkyl-Imidazolsilanen-Funktionalisierung zwischen 0 und 10° und für Oberflächen mit Guanidinsilanen um 30°. Für Beschichtungskombinationen aus Alkansilanen und Aminosäure-Polymeren lagen die FKW unter 10°.

**Beispiel 3**

Untersuchungen zur Selektivität des Stofftransports von Carbonsäuren.

Runde Membranstücke mit einem Durchmesser von 12mm von behandelten Membranen aus Versuch 2 mit raumfüllenden nanocavitären Polymerstrukturen (M1.8 und M2.5) sowie die korrespondierenden nativen Membranen (M1.0 bzw. M2.0) wurden druckdicht in ein Teflonseptum eingebracht, welches sich in einer Elektrodialyseeinheit zwischen der Donor- und der Akzeptorkammer befand und diese voneinander elektrisch isolierte. Die beiden Kammern hatten ein Füllungsvolumen von je 25 ml und waren von einer außenseitig angrenzenden Anolyt- bzw. Katholytkammer durch weitere Teflonsepten getrennt, in die eine kationen- bzw. anionenselektive Membran (Fumasep FKS bzw. FAS, Fumatech, Deutschland) druckdicht eingebracht war. In der Anolyt- und Katholytkammer befanden sich Platinelektroden, die mit einer Gleichspannungsquelle verbunden waren. Ein Deckel dichtete die Kammern gegeneinander und gegen die Atmosphäre ab. Über eine pneumatische Verbindung, die durch den Deckel im Bereich der Donorkammer bestand, konnte der Druck in dieser Kammer mittels eines Druckaufnehmers monitoriert werden. Die Versuche erfolgten mit verschiedenen Testlösungen, mit denen die Donorkammer jeweils befüllt wurde: Versuch D1) NaCl 1 Gew% + Capronsäure 100mmol/l in 2%iger ammoniakalischer Lösung; Versuch D2) Capryl-, Myristin-, Myristoleinsäure-, Palmitin-, Öl-, Linol- und Linolensäure jeweils 20mmo/l, die in einer 250 mmolaren Arginin-Lösung nanoemulgiert waren; Versuch D3) Ölsäure, 1-Octadecanesulfonyl Chlorid + Octadecyl Sulfat (je 10mmol)+ DMSO 2 Gew% in 20%iger ethylalkoholischer Lösung; Versuch D4) $NaSO_4$ 1 Gew% + Albumin 100mmol/l + Kreatinin 50mol/l und Linolensäure 50mmol/l in wässriger 100mmlolarer Argininlösung. Die Akzeptorkammer wurde bei den Versuchen D1 und D2 mit einer 100mmolaren Argininlösung und bei den Versuchen D3 und D4 mit einer NaOH-Lösung (0,1%), in der 0,5Gew% Polyethylenimin gelöst war, befüllt. In beiden Kammern waren Magnetrührstäbe eingelegt, die durch ein externes Magnetrührwerk während der Untersuchungen mit 100rpm rotiert wurden.
Es erfolgten Versuche zur Diffusion (DD) sowie zur Elektrophorese (ED) der Testverbindungen. Die Diffusionsversuche erfolgten über eine Dauer von 6 Stunden. Für den elektrophoretischen Stofftransport wurde eine Spannung von 40 V zwischen den Elektroden für die Dauer von 60 Minuten angelegt. Aus der Akzeptorkammer wurden Proben alle 60 Minuten bei den Diffusionsversuchen und alle 15 Minuten bei den Elektrodialyseversuchen entnommen. Ferner wurden Proben der Ausgangslösung sowie der Lösung in der Donorkammer nach Versuchsende entnommen. Die Proben wurden auf die Konzentration der jeweils eingesetzten Substanzen analysiert. Hieraus wurde der Anteil der Verbindungen, der durch die Membran transportiert wurde, berechnet.

Ergebnisse:

Die Ergebnisse der Transportversuche sind in Tabelle 2 zusammengefasst.

Tabelle 2: Ergebnisse der Transportversuche zur Diffusion (DD) und zur Elektrophorese (ED).

| **Versuch D1** | M1.8-DD | M1.8-ED | M2.5-DD | M2.5-ED | M1.0-DD | M1.0-ED | M2.0-DD | M2.0-ED |
|---|---|---|---|---|---|---|---|---|
| Chlorid | 0[a] | 0,1 | 0 | 0,1 | 6,5 | 63 | 7 | 78,5 |
| Capronsäure | 4,5 | 72 | 5 | 80 | 4 | 55 | 4 | 66 |

(fortgesetzt)

| **Versuch D2** | M1.8-DD | M1.8-ED | M2.5-DD | M2.5-ED | M1.0-DD | M1.0-ED | M2.0-DD | M2.0-ED |
|---|---|---|---|---|---|---|---|---|
| Caprylsäure | 7,5 | 75 | 6,5 | 80 | 6 | 68 | 4 | 65 |
| Myristinsäure | 6 | 72 | 7 | 78 | 5 | 66 | 3,5 | 64 |
| Myristoleinsäure | 6,5 | 76 | 6 | 76 | 5,5 | 58 | 4 | 64 |
| Palmitinsäure | 6 | 70 | 5 | 72 | 4 | 52 | 2,5 | 50 |
| Ölsäure | 5,5 | 75 | 6 | 76 | 4,5 | 58 | 3 | 60 |
| Linolsäure | 6 | 76 | 6,5 | 75 | 4 | 56 | 3 | 58 |
| Linolensäure | 6 | 75 | 6 | 76 | 4 | 55 | 2,5 | 55 |
| **Versuch D3** | M1.8-DD | M1.8-ED | M2.5-DD | M2.5-ED | M1.0-DD | M1.0-ED | M2.0-DD | M2.0-ED |
| Ölsäure | 5 | 79 | 6 | 82 | 4 | 35 | 2,5 | 38 |
| 1-Octadecansulfonylchlorid | 0 | 0 | 0 | 0 | 4,5 | 48 | 3 | 50 |
| Octadecylsulfat | 0 | 0,5 | 0 | 0,5 | 5 | 56 | 3,5 | 54 |
| 1-Octadecanol | 0,1 | 0,2 | 0,1 | 0,1 | 11 | 9 | 10 | 9,5 |
| **Versuch D4** | M1.8-DD | M1.8-ED | M2.5-DD | M2.5-ED | M1.0-DD | M1.0-ED | M2.0-DD | M2.0-ED |
| $SO_{4-}$ | 0 | 0,1 | 0 | 0,1 | 5,5 | 86 | 6 | 89 |
| Albumin | 0 | 0 | 0 | 0 | 3,5 | 56 | 3,5 | 58 |
| Kreatinin | 0 | 0 | 0 | 0 | 6 | 7 | 5 | 6 |
| Linolensäure | 4,5 | 78 | 5,5 | 82 | 2 | 39 | 2,5 | 35 |
| [a]Alle Werteangaben als relative Menge (%) der Ausgangsmenge, die in das Akzeptormedium transportiert worden ist. | | | | | | | | |

Membranen mit nanocavitären Polymerstrukturen waren für hydrophile Verbindungen nicht durchlässig, während diese Membranen sowohl für den diffusiven als auch den elektrophoretischen Stofftransport von Carbonsäuren durchlässig waren. Der elektrophoretische Stofftransport für Carbonsäuren war im Vergleich zu den nativen Trägermembranen gesteigert. Für Carbonsäuren bestand gegenüber Sulfaten, die ein vergleichbares Molekulargewicht hatten, ein selektiver Stofftransport mit einem Selektivitätsindex $\alpha_I$ von 158 für M1.8 und 164 für M2.5.

$$\alpha_I = \frac{v_{COOH}}{v_{Sulfat}} = \frac{n_{COOH}/n_0}{n_{Sulfat}/n_0}$$

wobei v die Transportgeschwindigkeit durch die Membran, n die transportierte Stoffmenge und $n_0$ die Stoffmenge in der Donorlösung zum Zeitpunkt t = 0 ist. Bezogen auf den korrespondierenden Alkohol, 1-Octadecanol, war die Selektivität bei der Elektrodialyse sogar noch höher. Der Selektivitätsindex $\alpha_{OH}$ betrug 395 für M1.8 und 820 für M2.5.

$$\alpha_{OH} = \frac{v_{COOH}}{v_{OH}} = \frac{n_{COOH}/n_0}{n_{OH}/n_0}$$

Für das Sulfonylchlorid konnte für die Membranen M1.8 und M2.5 kein Selektivitätsindex berechnet werden, da die transportierte Stoffmenge an Sulfonylchlorid durch die Membran zu klein war und nicht bestimmt werden konnte. Im Vergleich zur Elektrodialyse wurde bei der diffusiven Dialyse ein deutlich geringerer Stofftransport beobachtet. Die transportierte Stoffmenge an Sulfonylchlorid und Sulfat war wiederum so niedrig, dass für die Membranen M1.8 und M2

kein Selektivitätsindex berechnet werden konnte.
Im Gegensatz dazu wurden bei den nativen Membranen M1.0 und M2.0 sowohl bei der elektrophoretischen als auch bei der diffusiven Abtrennung Selektivitätsindizes für Sulfonylchlorid und Sulfat zwischen 0,6 und 0,8 beobachtet. Für den Alkohol wurden niedrige Selektivitätsindizes von 0,36 und 0,25 für M1.0 und M2.0 bei der diffusiven Trennung sowie 3,9 und 4 für M1.0 und M2.0 bei Elektrodialysen bestimmt. Bei Elektrodialysen mit den nativen Membranen M1.0 bzw. M2.0 kam es durch einen EOF zu einem Druckanstieg in der Donorkammer bis auf 152 bzw. 166mbar, 235 bzw. 243mbar, 195 bzw. 205mbar und 165 bzw. 188mbar in den Versuchen D1 bis D4. Im Gegensatz hierzu erhöhte sich der Druck in der Donorkammer nur minimal bei Verwendung der Membranen M1.8 und M2.5 auf 3 bzw. 2mbar bei D1, 5 bzw. 4mbar bei D2, 2 bzw. 1 mbar bei D3 und 3 bzw. 3mbar bei D4.

**Beispiel 4:**

Untersuchungen zum "Membranfouling" durch organische Medien.

Für die Untersuchungen wurden Membranstücke mit einem Durchmesser von 12mm, von Polyethersulfon- und anodisierten Aluminiumoxid-Membranen, die in Versuch 2 behandelt wurden und raumfüllende nanocavitäre Polymerstrukturen aufwiesen (M4.13 und 5.11) sowie die nativen Membranen (M4.0 bzw. M5.0) verwandt. Ferner wurden behandelte Membranen aus Beispiel 2 untersucht, die mit Funktionsverbindungen beschichtet worden waren, aber keine raumfüllenden Polymerstrukturen aufwiesen (M4.2 und M5.2).

Es wurden Versuche zur Diffusion und zum elektrophoretischen Stofftransport analog und mit dem gleichen Versuchsaufbau wie in Bsp. 3 durchgeführt. Zur Prüfung einer Veränderung des Stofftransportes durch Foulingprozesse, wurde 6-Lauroleinsäure (C12:1) den folgenden organischen Medien in einer Konzentration von 100mmol/l hinzugegeben: V1 Kälberserum, V2 Klärschlamm (10 Gew% Trockenmasse), V3 Buttermilch und V4 Bier-Fermentierungslösung. Die Akzeptorkammer war jeweils mit einer 200mmolaren Arginin-Lösung gefüllt.

Die Elektrodialysen erfolgten bei einer Spannung von 15 V mit einer Stromstärke von 100mA über 6 Stunden. Alle 15 Minuten wurde eine Probe aus der Akzeptorkammer zur Bestimmung der Lauroleinsäurekonzentration sowie zum Nachweis von Proteinen entnommen. Es wurde die Dauer bis zum Erreichen einer Lauroleinsäurekonzentration von 10mmol/l ermittelt (Dauer 1). Anschließend wurden beide Kammern geleert und dann mit den gleichen Ausgangslösungen, wie in dem zuvorigen Versuch, befüllt sowie ein identischer Versuchsablauf gestartet. Auch hier wurde die Dauer bis zum Erreichen einer Lauroleinsäurekonzentration von 10mmol/l ermittelt (Dauer 2). Hiernach wurden die Membranen in einem Wasserbad geschwenkt und danach getrocknet. Bei den getrockneten Membranen sowie bei 2 Membranen mit und ohne Polymerstrukturen, die nicht bei den Versuchen eingesetzt worden waren (Referenzproben), erfolgte eine Färbung mit Fluoreszinisothiocyanat. Die fluoreszenzmikroskopisch ermittelte Färbungsintensität bei den Membranen, die in den Versuchen eingesetzt worden waren, wurde mit der der Referenzproben verglichen.

Ergebnisse: Die numerischen Ergebnisse sind in Tabelle 3 angegeben.

Tabelle 3: Membranfoulinq-Versuche.

| Versuch | Membran | Dauer 1[a] (Min) | Protein 1[c] | Dauer 2[b] (Min) | Protein 2[d] |
|---|---|---|---|---|---|
| V1 | M4.13 | 60 | -- | 75 | -- |
| | M5.11 | 75 | -- | 75 | -- |
| | M4.0 | 180 | +++ | 345 | ++ |
| | M5.0 | 165 | +++ | 330 | ++ |
| | M4.2 | 150 | +++ | 315 | +++ |
| | M5.2 | 120 | +++ | 300 | +++ |
| V2 | M4.13 | 45 | -- | 60 | -- |
| | M5.11 | 75 | -- | 60 | -- |
| | M4.0 | 180 | ++ | 315 | ++ |
| | M5.0 | 195 | ++ | 330 | ++ |
| | M4.2 | 150 | +++ | 315 | +++ |
| | M5.2 | 165 | +++ | 330 | +++ |
| V3 | M4.13 | 75 | -- | 75 | -- |

(fortgesetzt)

| Versuch | Membran | Dauer 1[a] (Min) | Protein 1[c] | Dauer 2[b] (Min) | Protein 2[d] |
|---|---|---|---|---|---|
| | M5.11 | 90 | -- | 90 | -- |
| | M4.0 | 150 | +++ | 210 | ++ |
| | M5.0 | 165 | +++ | 240 | +++ |
| | M4.2 | 195 | +++ | 345 | ++ |
| | M5.2 | 210 | +++ | > 360 | +++ |
| V4 | M4.13 | 60 | | 60 | -- |
| | M5.11 | 75 | | 60 | -- |
| | M4.0 | 210 | +++ | 300 | ++ |
| | M5.0 | 195 | +++ | 330 | ++ |
| | M4.2 | 210 | +++ | 345 | +++ |
| | M5.2 | 240 | +++ | 360 | ++ |

[a]Dauer 1 = Dauer in Minuten bis zum Erreichen einer Lauroleinsäurekonzentration in der Akzeptorkammer von 10mmol/l beim ersten Versuchsdurchlauf;
[b]Dauer 2 = Dauer in Minuten bis zum Erreichen einer Lauroleinsäurekonzentration in der Akzeptorkammer von 10mmol/l beim zweiten Versuchsdurchlauf;
[c]Protein 1 = Semiquatitativer Nachweis von Protein in der Akzeptorkammerlösung am Ende des ersten Versuchsdurchlaufs, Nachweisgrenze 0,5$\mu$g/l;
[d]Protein 2 = Semiquatitativer Nachweis von Protein in der Akzeptorkammerlösung am Ende des zweiten Versuchsdurchlaufs, Nachweisgrenze 0,5$\mu$g/l

Die Separationsleitung der Membranen mit nanocavitären Polymerstrukturen war bei den untersuchten Ausgangslösungen vergleichbar. Diese blieb, nach erfolgter Exposition der Membran mit einem organischen Medium, unverändert bei einer wiederholten Separation über weitere 6 Stunden. Auflagerungen organischer Verbindungen waren auf diesen Membranen praktisch nicht nachweisbar. Im Gegensatz hierzu bestanden deutliche bis starke Auflagerungen bei Membranen, die eine hydrophobe Funktionalisierung erhalten hatten sowie bei den nativen Membranen nach den Elektrodialyse-Versuchen. Bei diesen Membranen war bei dem wiederholten Elektrodialyseversuch die Transportleistung für Fettsäuren gegenüber dem ersten Versuch deutlich verringert als Ausdruck eines Membran-Foulings. Bei den Versuchen, die mit Membranen mit hydrophober Oberflächenfunktionalisierung sowie nativen Membranen erfolgten, war der Proteinnachweis in den Akzeptorlösungen positiv, während Proteine nicht in den Akzeptorlösungen bei den Versuchen mit Membranen, die raumfüllende Polymerstrukturen aufwiesen, bestimmbar waren.

**Beispiel 5:**

Untersuchung zur Bio- und Hämokompatibilität.

Die folgenden Membranen aus Beispiel 2 wurden untersucht: M1.1, M1.12, M4.2, und M4.8 sowie die nativen Membranen M1.0 und M4.0. Die Versuchsdurchführung erfolgte gemäß Beispiel 3. Die Donorkammer wurde bei der Versuchsreihe A) mit Vollblut, das mit Heparin in einer Konzentration von 1,0 IU/ml antikoaguliert war und bei der Versuchsreihe B) mit Blutserum befüllt. Die Untersuchungen erfolgten bei einer Temperatur von 37°C. Die Akzeptorkammer enthielt eine 100mmolare Arginin-Lösung. Es wurde eine Gleichspannung von 5V bei 50mA an die Elektroden angelegt. Die Versuchsdauer betrug 120 Minuten.

Nach dem Untersuchungsende wurden Proben aus der Akzeptorkammer entnommen und auf den Gehalt an Fettsäuren und Proteinen analysiert. Die Bestimmung der Fettsäuren erfolgte mittels Gaschromatographie. Alle Untersuchungen wurden 2-mal wiederholt und der Mittelwert der erhaltenen Werte berechnet.

Das Vollblut (VB) bzw. das Serum (S) aus der Donorkammer wurden separiert und weiter analysiert. Dem VB wurden EDTA oder Citrat beigemischt, sodass Endkonzentrationen von 4 bzw. 13 mM erreicht wurden. Vor einer Zentrifugation wurde die Anzahl der Blutplättchen mit einem Coulter AcT diffTM haematology analyzer (Coulter Corporation, USA) bestimmt. Das mit EDTA versehene VB wurde mit 2,200 g über 10 Min bei 41 °C zentrifugiert und das citratbehandelte VB wurde über 10 Min bei 1,000g sowie über 10 min bei 10,000 g bei 41 °C zentrifugiert. Der Gehalt an

Thrombin-anti-thrombin-Komplex (TAT) und Faktor XIIa-AT -

Komplexformationen wurde quantifiziert mit einem Enzym-immunoassay (EIA) (Enzygnosts, Behringwerke, Germany). Ferner erfolgte eine Quantifizierung durch ein EIA für ß-Thromboglobulin (ß-TG) und die Komplementfaktoren C5b und sC5b-9. Die erhaltenen Werte wurden in Relation zu denen aus einer Referenzprobe gesetzt, bei der das VB bzw. S in einem PTFE-Behältnis mit gleicher Dauer und Agitation, wie dies in der Elektrodialysevorrichtung erfolgte, gelagert worden waren.

Die eingesetzten Membranen wurden nach den Versuchen in einem Wasserbad kurz geschwenkt und anschließend geteilt. Ein Teil der Membranstücke wurde getrocknet und auf die erfolgte Proteinadsorption untersucht. Der andere Teil der Membranstücke wurde für die Analyse der Zellbelegung der Oberflächen unmittelbar weiterverarbeitet.

Der Gehalt an adsorbiertem Protein wurde mittels enzyme linked immunosorbent assays für Fibrinogen, Fibronectin und Albumin bestimmt. Die mittleren Belegungsdichten bei hydrophobisierten Membranen und bei Membranen mit nanocavitären Polymerstrukturen wurden in Relation gesetzt zum Färbungsindex, der bei einer nicht eingesetzten Ausgangsmembran ermittelt wurde. Das Ausmaß der Adhäsion von Thrombozyten und Leukozyten wurden mittels einer fluoreszenzmikroskopischen Analyse nach Färbung mit Calcein-AM und Propidiumjodid ermittelt und ist angegeben als relativer Oberflächenbelegungsgrad zur Gesamtoberfläche. Die Zytotoxizität wurde ermittelt durch die Bestimmung der LDH-Konzentrationen des VB nach einer Elektrodialyse und ist angegeben als relativer Anstieg gegenüber dem Wert einer Bestimmung bei einer Referenzprobe, die unter Agitation in einem PTFE-Behältnis über die Versuchsdauer gelagert worden war.

Ergebnisse: Die Ergebnisse sind in Tabelle 4 zusammengefasst.

Membranen mit nanocavitären Polymerstrukturen bedingten während einer Elektrodialyse eine im Vergleich zu einer nativen Membran oder einer Membran mit einer hydrophoben Oberflächenfunktionalisierung nur minimale Aktivierung des Gerinnungs- und Komplementsystems. Ferner stellte sich eine erheblich geringere oberflächliche Belegung mit Serumproteinen und Blutzellen dar, als dies bei nativen Membranen und Membranen mit einer hydrophoben Oberflächenfunktionalisierung der Fall war. Während bei Elektrodialysen mit nativen Membranen und Membranen mit einer hydrophoben Oberflächenfunktionalisierung Hinweise auf eine Zytotoxizität der Oberflächen bestanden, lagen solche bei Membranen mit nanocavitären Polymerstrukturen nicht vor.

Tabelle 4: Ergebnisse der Bio- und Hämokompatibilitätsuntersuchungen.

| | **TAT *[a]** | **ß-TG *[b]** | **sC5b-9 *** | **C5b *** | **Albumin *** | **Fibrinogen *** | **Fibronectin *** | **Thrombozyten** | **Leukozyten** | **LDH *** |
|---|---|---|---|---|---|---|---|---|---|---|
| M1.1 | 1.250 | 11,5 | 1,9 | 5,4 | 264 | 175 | 241 | 98 | 8 | 30 |
| M1.12 | 1,2 | 0,1 | 0,2 | 0,3 | 22 | 10 | 8 | 10 | < 1 | 2 |
| M1.0 | 1.375 | 15,3 | 2,2 | 6,2 | 374 | 210 | 211 | 100 | 9 | 44 |
| M4.2 | 1,5 | 0,2 | 0,1 | 0,1 | 18 | 14 | 6 | 8 | < 1 | 3 |
| M4.8 | 1.422 | 14,1 | 2,4 | 7 | 366 | 156 | 314 | 100 | 6 | 38 |
| M4.0 | 1,3 | 0,1 | 0,2 | 0,2 | 15 | 8 | 9 | 12 | < 1 | 2 |

*Werteangaben in % als relative Änderung gegenüber der Referenzprobe;
[a]TAT: Thrombin-Antithrombin-Komplex;
[b]ß-TG = ß-Thromboglobulin;
[c]Thrombozyten/Leukozyten = mit Thrombozyten bzw. Leukozyten belegte Fläche in Relation zur Gesamtfläche

Die adhärierten Zellen auf den Oberflächen von Membranen mit nanocavitären Polymerstrukturen waren zu 97 - 99% als lebend bewertet, während dies auf Oberflächen der nativen Membranen nur bei 79 bis 84% der Fall war.

Während der Elektrodialysen war es zu einem Transport von Fettsäuren aus dem Serum und Vollblut in die Akzeptorkammern gekommen. Die Konzentrationen betrugen bei den nativen Membranen zwischen 8 und 13mmol/l, bei Membranen mit einer hydrophoben Oberflächenfunktionalisierung 12 und 16mmol/l und bei Membranen mit nanocavitären Polymerstrukturen 22 bis 34mmol/l. Proteine wurden nur bei den Versuchen mit nativen Membranen und Membranen mit einer hydrophoben Oberflächenfunktionalisierung auf der Akzeptorseite gefunden.

**Beispiel 6**

Untersuchung zur Verwendung von Membranen mit nanocavitären Spalträumen zur selektiven Abtrennung von Fettsäuren aus wässrigen Stoffgemischen.

Keramische Zikoniumoxid-Sintermenbranen mit einer mittleren Kanalweite von 0,5μm, einer Materialdicke von 3 mm und Abmessungen von 30 x 30cm wurden einer Oberflächenvorbehandlung gemäß Beispiel 2 (M1.12) zugeführt. Das Vorliegen von nanocavitären gewebeartigen Polymerstrukturen, die sich raumfüllend über das gesamte Hohlraumsystem der Membranen erstreckten, konnte elektronenmikroskopisch dokumentiert werden.

Es wurden 10 der hergestellten Membranen in eine Elektrodialyse-Vorrichtung eingebracht, indem diese in eine Rahmenvorrichtung aus PTFE eingeklebt wurden. Ferner wurden kationenselektive Membranen (Fumasep, FKS, Fumatech, Deutschland) in eine gleichartige Rahmenvorrichtung eingebracht. Die Rahmenvorrichtungen wurden gestapelt, sodass alternierend eine der hergestellten Separationsmembranen und eine kationenselektive Membran eine Donorkammer bzw. eine Akzeptorkammer begrenzten, die jeweils eine Tiefe von 1 cm hatten. Die Anolytkammer war mit einer anionenselektiven Membran gegen die letzte Akzeptorkammer elektrisch abgeschlossen. Die erste Donorkammer grenzte an die kationenselektive Membran der Katholytkammer. Der Stapel sowie die außen befindlichen Anolyt- und Katholytkammern wurden durch eine externe Vorrichtung zusammengepresst, sodass die Kammern gegen die Umgebung druckdicht abgeschlossen waren. Eine weitere baugleiche ED-Einheit wurde mit nativen keramischen Zirkoniumoxid-Membranen gefertigt. Während der ED wurde eine Gleichspannung von 40V mit 0,3A angelegt. Die Donor- und die Akzeptorkammern waren jeweils durch ein Schlauchsystem seriell miteinander verbunden. Der Einleitungsort und die Flussrichtung der Kammersysteme waren entgegengesetzt, die Einleitung des Ausgangsmediums erfolgte in die erste Zelle, die an die Kathodenkammer angrenzte. Beide Systeme wurden mit einem Volumenstrom von 1 Liter/min. beaufschlagt. Die Akzeptorlösung bestand aus einer 0,5 molaren Arginin-Lösung, die aus einem Vorratsgefäß in das Akzeptorkammersystem eingeleitet wurde. Nach dem Austritt aus der ED-Einheit wurde die mit Fettsäuren angereicherte Akzeptorlösung in ein Gefäß aufgefangen und das Volumen bestimmt. Ferner wurden hieraus Proben zur Analyse entnommen. Es erfolgte eine quantitative Bestimmung von Eisen, Magnesium und Phosphor, sowie eine semiquantitative Bestimmung von Glycolipiden.

Ein hexan-extrahiertes Rapspressöl wurde mit einer Arginin-Lösung wässrig entschleimt. Die wässrige Phase (WP) enthielt 12Gew-% freie Fettsäuren, 8Gew-% Phospholipide und 6Gew-% Glykolipide, ferner Lipoproteine und Farbstoffe, sowie Eisen-, Magnesium-, Natrium-, Calcium- und Kaliumionen. Diese WP lag als grünliche trübe Emulsion vor und wurde mit einer Schlauchpumpe in das Donorkammersystem eingeleitet.

Jeweils 50Litern der aufgefangenen Akzeptorlösungen wurde HCl (37Gew%) hinzugegeben, bis der pH der Lösung 2,5 betrug. Die sodann aufschwimmende Ölfraktion wurde in ein weiteres Sammelgefäß überführt. Die restliche Lösung wurde anschließend mit NaOH wieder auf ein pH-Niveau von 12 angehoben und nach einer Abtrennung von Natrium und Chloridionen wieder eingesetzt.

Aus dem abgereicherten wässrigen Reinigungsmedium wurden Proben zur Analytik genommen. Die Fettsäureanalytik erfolgte durch GC der Methylester. Hierzu erfolgte eine Probenaufarbeitung durch Hinzugabe von HCl und Extraktion der Fettsäuren mit Hexan. Anschließend erfolgte eine Methylierung.

Ergebnisse: Bei unbeschichteten Membranen kam es zu einem elektroosmotischen Fluss, der dazu führte, dass das Volumen der Akzeptorlösung sich rasch verringerte und das Volumen der abgereicherten Ausgangslösung um den entsprechenden Betrag zunahm, sodass der Versuch vorzeitig abgebrochen wurde. Bei der ED mit Membranen, die nanocavitäre Polymerstrukturen aufwiesen, blieben die Volumina der Donor- und der Akzeptormedien gleich. Ferner kam es zu einer Abreicherung des Fettsäuregehalts des wässrigen Reinigungsmediums auf 0,1 Gew% durch die Elektrodialyse. Im klaren und farblosen Akzeptormedium lagen zum Versuchsende keine bestimmbaren Konzentrationen von Phosphor, Eisen-, Magnesium-, Natrium-, Calcium- oder Kalium vor. Glycolipide konnten ebenfalls nicht nachgewiesen werden. Die ölige Fraktion, die von dem Akzeptormedium abgetrennt worden war, bestand im Wesentlichen aus Ölsäure, ferner enthielt sie Linolsäure, Linolensäure und Stearinsäure.

**Beispiel 7**

Untersuchung zur selektiven Abtrennung von Fettsäuren aus einer wässrigen Albuminlösung.

Einer wässrigen (0,9% NaCl) Humanalbumin-Lösung mit einem Gewichtsanteil von 20% wurden, bezogen auf den Gewichtsanteil des Albumins, 2% Ölsäure hinzugegeben. Die Lösung wurden über 2 Stunden bei 25°C gerührt. Die Donorkammer wurde mit der Lösung randvoll gefüllt. Der Versuchsaufbau entsprach dem aus Beispiel 3. Die Akzeptorkammer wurde in der Versuchsserie V1 mit einer 150mmolaren Argininlösung befüllt. In der Versuchsserie V2 waren in der Argininlösung 0,5 Gew% Polyethylenimin gelöst. Die Elektrodialyse erfolgte in der Versuchsreihe I. mit einer Spannung von 5 V und in der Versuchsreihe II. mit 50V, jeweils über 2 Stunden. Es wurden der Stromflusses sowie der Druck in der Donorkammer kontinuierlich gemessen. In Abständen von 15 Minuten sowie am Untersuchungsende wurden Proben für die Bestimmung von Fettsäuren und zum Albuminnachweis aus der Akzeptorkammer entnommen. Die Fettsäureanalytik erfolgte mittels GC, die Bestimmung von Albumin spektroskopisch mit einem Bromcresolgrün-Reagenz.

Als Separationsmembranen wurden 12mm durchmessende Stücke der anionenselektiven Polymermembranen Fumasep FAA-3-PK-130 (Fumatech, Deutschland) und PC 400D-250-250 sowie PC 200D-250-250 (PCA, Deutschland) untersucht. Ferner wurden Nanofiltrationsmembranen, bei denen gemäß Beispiel 2 eine Hydrophobisierung der Kanaloberflächen erfolgt war (M5.1 und M5.3) untersucht.

Desweitern wurde eine Track-etch-Polycarbonat Membran (Nuclepore, Whatman, USA, Porendurchmesser 20 nm, Dicke 5μm), deren innere und äußeren Oberflächen vollflächig gemäß Beispiel 2 M1.1 beschichtet worden waren, untersucht (PC1.1). Die Selektivität der Transporteigenschaften wurde verglichen mit Membranen, die raumfüllende nanocavitäre Polymerstrukturen aufwiesen (M5.12 und M1.8 aus Beispiel 2). Die hydrophobisierten Nanofiltrationsmembranen wiesen einen Wasserkontaktwinkel zwischen 110° und 118° auf. Nach den Versuchen wurden alle Membranen entnommen und visuell beurteilt.

Ergebnisse: Die numerischen Ergebnisse sind in Tabelle 5 zusammengefasst. Geschlossene anionen-selektive Membranen sind für Fettsäuren undurchlässig. Die Membranen quollen während der Elektrodialyse auf und verformten sich. Die nativen Ultrafiltrationsmembranen ermöglichten einen Transport von Fettsäuren unter Ausbildung eines elektroosmotischen Flusses. Dies war auch der Fall bei Nanofiltrationsmembranen, deren Oberflächen hydrophobisiert worden waren. Bei den vorgenannten Membranen wurde auch Albumin durch die Membranen transportiert. An allen vorgenannten Membranen bestanden donorkammerseitig gelartige Auflagerungen, die Albumin enthielten. Die Membranen mit nanoskalierten raumfüllenden Polymerstrukturen wiesen eine deutlich höhere Transportrate für Fettsäuren auf, als die anderen offenporigen Membranen. Gleichzeitig fand ein Transport von Albumin nicht statt und es entstand auch praktisch kein elektroosmotischer Fluss bei der geringeren Spannung, die an der Membran anlag. Bei einer hohen Spannung bestand ein geringer elektroosmotischer Fluss, der durch die Anwesenheit von Polykationen in der Akzeptorlösung praktisch unterbunden wurde. Bei diesen Membranen bestanden auch keine Albuminauflagerungen.

Tabelle 5: Selektive Abtrennung von Fettsäuren aus einer wässrigen Albuminlösung.

| **Versuchsserie/ Versuchsreihe** | **Membran** | **$P_{DK}$ (mmHg)** | **[b]Protein-Ablagerung** | **[a]Protein-$_{AK}$** | **[c]FS-$_{AK}$ (mmol/l)** |
|---|---|---|---|---|---|
| I./V1 | FAA-3-PK-130 | 2 | ++ | -- | < 0,1 |
| I./V1 | PC 400D-250-250 | 6 | +++ | -- | < 0,1 |
| I./V1 | PC 200D-250-250 | 3 | ++ | -- | < 0,1 |
| I./V1 | PC1.1 | 174 | +++ | ++ | 4,3 |
| I./V1 | M5.1 | 134 | +++ | +++ | 5,9 |
| I./V1 | M5.3 | 126 | +++ | +++ | 6,2 |
| I./V1 | M5.12 | 2 | -- | -- | 15,2 |
| I./V1 | M5.8 | 3 | -- | -- | 17,4 |
| II./V1 | FAA-3-PK-130 | 6 | +++ | -- | < 0,1 |
| II./V1 | PC 400D-250-250 | 11 | +++ | -- | < 0,1 |

(fortgesetzt)

| Versuchsserie/ Versuchsreihe | Membran | $P_{DK}$ (mmHg) | [b]Protein-Ablagerung | [a]Protein-AK | [c]$FS-_{AK}$ (mmol/l) |
|---|---|---|---|---|---|
| II./V1 | PC 200D-250-250 | 9 | +++ | -- | < 0,1 |
| II./V1 | PC1.1 | 366 | +++ | ++ | 8,4 |
| II./V1 | M5.1 | 310 | +++ | +++ | 12,3 |
| II./V1 | M5.3 | 321 | +++ | +++ | 14,1 |
| II./V1 | M5.12 | 7 | -- | -- | 37,8 |
| II./V1 | M5.8 | 10 | -- | -- | 42,2 |
| I./V2 | FAA-3-PK-130 | 2 | ++ | -- | < 0,1 |
| I./V2 | PC 400D-250-250 | 5 | ++ | -- | < 0,1 |
| I./V2 | PC 200D-250-250 | 4 | ++ | -- | < 0,1 |
| I./V2 | PC1.1 | 156 | ++ | ++ | 5,3 |
| I./V2 | M5.1 | 101 | +++ | + | 6,4 |
| I./V2 | M5.3 | 106 | +++ | + | 7,1 |
| I./V2 | M5.12 | 2 | -- | -- | 16,2 |
| I./V2 | M5.8 | 3 | -- | -- | 19,8 |
| II./V2 | FAA-3-PK-130 | 7 | ++ | -- | < 0,1 |
| II./V2 | PC 400D-250-250 | 11 | +++ | -- | < 0,1 |
| II./V2 | PC 200D-250-250 | 10 | ++ | -- | < 0,1 |
| II./V2 | PC1.1 | 125 | +++ | +++ | 9,2 |
| II./V2 | M5.1 | 111 | +++ | ++ | 10,1 |
| II./V2 | M5.3 | 103 | +++ | +++ | 11,2 |
| II./V2 | M5.12 | 2 | -- | -- | 22,6 |
| II./V2 | M5.8 | 2 | -- | -- | 28,3 |

[a]$P_{DK}$ = Maximal in der Donorkammer gemessener Druck während der ED;
[b]Protein-Ablagerung: Donorkammerseitiger Belag mit positivem Proteinnachweis;
[c]$FS_{AK}$ = Fettsäurekonzentration in der Akzeptorlösung nach Versuchsende

**Patentansprüche**

1. Verfahren zur Herstellung von offenporigen Membranen zur diffusiven und/oder elektrophoretischen Abtrennung von Carbonsäuren aus flüssigen Stoffgemischen, umfassend die Schritte:

   a) Bereitstellen einer offenporigen Trägermembran,
   b) Einbringen einer Mono- und/oder Oligomerlösung in die offenporige Trägermembran aus Schritt a),
   c) Reaktionsaktivierung zur Ausbildung eines offenporigen raumfüllenden nanocavitären polymeren Strukturnetzwerkes.

2. Verfahren gemäß Anspruch 1, bei dem in Schritt c) die Ausbildung eines offenporigen raumfüllenden nanocavitären polymeren Strukturnetzwerkes durch eine ringöffnende Polymerisation erfolgt, wobei die Mono- und/oder Oligomerlösung erwärmt wird und/oder Mono- und/oder Oligomerlösung Lösungsmittel enthält, die eine ringöffnende oder radikalische Polymerisation initiieren können.

**3.** Verfahren gemäß Anspruch 1 oder 2, bei dem in Schritt c) die Polymerisationsreaktion durch die Hinzugabe/Applikation eines chemischen und/oder physikalischen Starters einer radikalischen oder nukleophilen Polymerisation erfolgt.

**4.** Verfahren gemäß einem der Ansprüche 1 - 3, bei dem in Schritt c) eine Polykondensation einer thermischen Schmelze der Mono- und/oder Oligomerlösung erfolgt.

**5.** Verfahren gemäß einem der Ansprüche 1 - 4, bei dem die Mono- und/oder Oligomerlösung Biomonomere und/oder Biooligomere enthält.

**6.** Verfahren gemäß einem der Ansprüche 1 - 5, bei dem die Mono- und/oder Oligomerlösung Phenylalanin und/oder Oligophenylalanin enthält.

**7.** Verfahren gemäß einem der Ansprüche 1 - 6, das zusätzlich Schritt d2) umfasst: d2) Funktionalisieren der Oberfläche der raumfüllenden Polymerstrukturen aus Schritt c), mit amphiphilen Verbindungen.

**8.** Verfahren gemäß Anspruch 7, wobei im Verfahrensschritt d2) die Oberflächen der nanocavitären raumfüllenden Polymerstrukturen mit ionischen und/oder ionisierbaren Verbindungen funktionalisiert werden.

**9.** Verfahren gemäß der Ansprüche 1 - 8, bei dem die offenporige Membran einen Selektivitätsindex für Carbonsäuren gegenüber den korrespondierenden Alkoholen von > 4 aufweist.

**10.** Verfahren gemäß einem der Ansprüche 1 - 9, bei dem die offenporige Trägermembran der Stufe a) aus einer porösen frei-tragenden Membran, einem Gewebe oder Texturen sowie porösen Materialien besteht, die in einer Verbundstruktur vorliegen.

**11.** Verfahren gemäß einem der Ansprüche 1 - 10, bei dem eine reaktionsfähige Monomerlösung in eine mikro- und/oder makroporöse Trägermembran vollständig eingebracht wird und die Polymerisation durch die Lösungsmittelphase initiiert wird.

**12.** Verfahren gemäß Anspruch 7 oder 8, bei dem in dem Schritt d2) die Oberflächen der nanocavitären Polymerstrukturen mit polykationischen Verbindungen funktionalisiert werden.

**13.** Offenporige Membran hergestellt nach einem Verfahren gemäß einem der Ansprüche 1 - 12.

**14.** Verwendung einer offenporigen Membran hergestellt nach einem Verfahren gemäß einem der Ansprüche 1 - 12 zur diffusiven und/oder elektrophoretischen Abtrennung von Carbonsäuren aus flüssigen Stoffgemischen.

**15.** Verwendung einer offenporigen Membran hergestellt nach einem Verfahren gemäß einem der Ansprüche 1 - 12 zur Separation von Carbonsäuren aus wässrigen Medien, wobei es sich bei dem wässrigen Medium um Blut und Blutprodukte handelt.

Europäisches Patentamt
European Patent Office
Office européen des brevets

# EUROPÄISCHER RECHERCHENBERICHT

Nummer der Anmeldung
EP 16 18 7197

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 3 000 523 A1 (KOREA MACH & MATERIALS INST [KR]) 30. März 2016 (2016-03-30)<br>* Seite 8, Zeile 24 - Seite 11, Zeile 7; Beispiel 1 *<br>----- | 1-4,8-13 | INV.<br>B01D67/00<br>B01D57/02<br>B01D69/10<br>B01D69/12<br>C07C51/42<br>A61M1/16 |
| X | WO 98/17377 A1 (UNIV MCMASTER [CA]; MIKA ALICJA M [CA]; CHILDS RONALD F [CA]; DICKSON) 30. April 1998 (1998-04-30)<br>* Ansprüche 1,10,12,13; Abbildung 4a *<br>----- | 1,2,4-6, 8,10,12, 13 | |
| X | EP 1 987 892 A1 (TOAGOSEI CO LTD [JP]) 5. November 2008 (2008-11-05)<br>* Absatz [0037]; Ansprüche *<br>----- | 1,7,8, 10-13 | |
| X | WO 2013/083229 A1 (THYSSENKRUPP UHDE GMBH [DE]) 13. Juni 2013 (2013-06-13)<br>* Absatz [0020] *<br>----- | 14 | |
| X | US 5 635 071 A (AL-SAMADI RIAD A [CA]) 3. Juni 1997 (1997-06-03) | 14 | |
| A | * das ganze Dokument *<br>----- | 15 | RECHERCHIERTE SACHGEBIETE (IPC) |
| | | | B01D<br>C07C<br>A61M |

Der vorliegende Recherchenbericht wurde für alle Patentansprüche erstellt

1

| Recherchenort | Abschlußdatum der Recherche | Prüfer |
|---|---|---|
| München | 20. März 2017 | Goers, Bernd |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 16 18 7197

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-03-2017

| Im Recherchenbericht angeführtes Patentdokument | Datum der Veröffentlichung | Mitglied(er) der Patentfamilie | Datum der Veröffentlichung |
|---|---|---|---|
| EP 3000523 A1 | 30-03-2016 | EP 3000523 A1 | 30-03-2016 |
| | | KR 101595185 B1 | 19-02-2016 |
| | | SG 10201500791R A | 28-04-2016 |
| | | US 2016059190 A1 | 03-03-2016 |
| WO 9817377 A1 | 30-04-1998 | AT 479492 T | 15-09-2010 |
| | | AU 731825 B2 | 05-04-2001 |
| | | CA 2268955 A1 | 30-04-1998 |
| | | EP 0948392 A1 | 13-10-1999 |
| | | JP 4297516 B2 | 15-07-2009 |
| | | JP 2000503898 A | 04-04-2000 |
| | | NZ 335446 A | 29-06-2001 |
| | | US 6258276 B1 | 10-07-2001 |
| | | US 2003168404 A1 | 11-09-2003 |
| | | WO 9817377 A1 | 30-04-1998 |
| EP 1987892 A1 | 05-11-2008 | CN 101384376 A | 11-03-2009 |
| | | EP 1987892 A1 | 05-11-2008 |
| | | JP 4640503 B2 | 02-03-2011 |
| | | KR 20080097187 A | 04-11-2008 |
| | | TW 200733460 A | 01-09-2007 |
| | | US 2009313813 A1 | 24-12-2009 |
| | | WO 2007094279 A1 | 23-08-2007 |
| WO 2013083229 A1 | 13-06-2013 | DE 102011120632 A1 | 13-06-2013 |
| | | EP 2788310 A1 | 15-10-2014 |
| | | US 2014371486 A1 | 18-12-2014 |
| | | WO 2013083229 A1 | 13-06-2013 |
| US 5635071 A | 03-06-1997 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **N. HILAL ; H. AL-ZOUBI ; N.A. DARWISH ; A.W. MOHAMMAD ; M. ABU ARABI.** *Desalination,* 2004, vol. 170, 281-308
- **JONGYOON HAN ; JIANPING FU ; RETO B.** Molecular Sieving Using Nanofilters: Past, Present and Future. *Schoch. Lab Chip.,* 2008, vol. 8 (1), 23-33
- **DAMIAN J. ODOM ; LANE A. BAKER ; CHARLES R. MARTIN.** Solvent-Extraction and Langmuir-Adsorption-Based Transport in Chemically Functionalized Nanopore Membranes. *J. Phys. Chem. B,* 2005, vol. 109, 20887-20894
- **LINDSTRAND, V ; SUNDSTROM, G ; JÖNSSON, ANN-SOFI LU.** Fouling of electrodialysis membranes by organic substances. *Desalination,* 2000, vol. 128 (1), 102-91